# EUROPEAN PATENT SPECIFICATION

(11) **EP 4 210 596 B1**
(45) Date of publication and mention of the grant of the patent: **12.11.2025**
(21) Application number: 22747421.0
(22) Date of filing: 08.07.2022
(51) Int. Cl.: A61B 17/072, A61B 17/00, A61B 90/00

(54) **CARTRIDGE RETENTION FEATURES FOR CURVED SURGICAL STAPLER**
PATRONENRÜCKHALTESYSTEM FÜR GEBOGENE CHIRURGISCHE KLAMMERGERÄTE
CARACTÉRISTIQUES DE RÉTENTION DE CARTOUCHE POUR AGRAFEUSE CHIRURGICALE COURBE

(30) Priority: 09.07.2021 US 202163220145 P; 31.05.2022 US 202217828693
(43) Date of publication of application: 19.07.2023
(73) Proprietor: Cilag GmbH International, 6300 Zug (CH)
(72) Inventor: ADAMS, Thomas E., Cincinnati, Ohio 45242 (US); COURTWRIGHT, Nicholas D., Cincinnati, Ohio 45242 (US); PALMER, Daniel A., Cincinnati, Ohio 45242 (US)
(74) Representative: Carpmaels & Ransford LLP
(86) International application number: PCT/IB2022/056333
(87) International publication number: WO 2023/281459

(56) References cited:
- WO-A1-2014/043971
- US-A1- 2017 189 022

## Description

### BACKGROUND

Some surgical staplers are operable to clamp down on one or more layers of patient tissue, form staples through the layers of tissue to substantially seal the layers of tissue together near the formed staples, and cut through the layers of tissue for forming severed ends of operatively sealed tissue. An exemplary stapling instrument may include a pair of cooperating elongate jaw members, where each jaw member may be adapted to be inserted into a patient and positioned relative to tissue that is to be stapled and/or incised. One of the jaw members may support a staple cartridge with at least two laterally spaced rows of staples contained therein, and the other jaw member may support an anvil with staple-forming pockets aligned with the rows of staples in the staple cartridge. Generally, the stapling instrument may further include a pusher bar and a knife blade that are slidable relative to the jaw members to sequentially or simultaneously eject the staples from the staple cartridge via camming surfaces on the pusher bar and/or camming surfaces on a wedge sled that is pushed by the pusher bar. The camming surfaces may be configured to activate one or more staple drivers carried by the cartridge and associated with the staples in order to push the staples against the anvil and form laterally spaced rows of deformed staples in the tissue gripped between the jaw members. Such rows may be arranged as linear rows and/or arcuate rows for sequentially or simultaneously stapling and cutting the tissue of the patient in the form of a predetermined pattern. The knife blade may trail the camming surfaces and cut the tissue along a linear or arcuate line between the rows of staples formed in the tissue.

Merely exemplary surgical staplers are disclosed in U.S. Pat. No. 6,988,650, entitled "Retaining Pin Lever Advancement Mechanism for a Curved Cutter Stapler," issued January 24, 2006; U.S. Pat. No. 7,134,587, entitled "Knife Retraction Arm for a Curved Cutter Stapler," issued November 14, 2006; U.S. Pat. No. 7,147,139, entitled "Closure Plate Lockout for a Curved Cutter Stapler," issued December 12, 2006, U.S. Pat. No. 7,147,140, entitled "Cartridge Retainer for a Curved Cutter Stapler," issued December 12, 2006; U.S. Pat. No. 7,204,404, entitled "Slotted Pins Guiding Knife in a Curved Cutter Stapler," issued April 17, 2007; and U.S. Pat. No. 7,207,472, entitled "Cartridge with Locking Knife for a Curved Cutter Stapler," issued April 24, 2007. Additional merely exemplary surgical staplers are disclosed in U.S. Pat. Pub. No. 2005/0139636, entitled "Replaceable Cartridge Module for a Surgical Stapling and Cutting Instrument," published on June 30, 2005; U.S. Pat. Pub. No. 2005/0143759, entitled "Curved Cutter Stapler Shaped for Male Pelvis," published on June 30, 2005, now abandoned; and U.S. Pat. Pub. No. 2005/0145672, entitled "Curved Cutter Stapler with Aligned Tissue Retention Feature," published on July 7, 2005, now abandoned.

A surgical stapler may be inserted into a patient to perform colorectal surgery. Such procedures may include the use of the stapler to operatively seal, sever, and remove the colon of the patient, in whole or in part. For instance, a proctocolectomy may be performed during a lower anterior resection ("LAR") for treating and inhibiting the spread of colorectal cancer cells. Of course, surgical staplers may be used in various other settings and procedures.

U.S. Pat. Pub. US 2017/0189022 describes the surgical stapling instrument that is depicted in FIGS. 1 - 5 of the drawings herein, and below in section I., subsection B. below. In this device, a one side of a staple cartridge housing includes a longitudinally extending detent slot defined between a confronting pair of resilient members. A first side of the detent slot includes a first proximal detent protrusion, and an opposed second side of the detent slot includes a second proximal detent protrusion and a distal detent protrusion. The detent slot slidably receives a detent post of a knife holder and a detent post of a staple driver assembly. As the staple driver assembly and knife holder translate distally within the cartridge housing, the detent posts resiliently engage respective detent protrusions. An anvil is coupled to a cartridge housing.

International Pat. Pub. WO2014/043971 also describes a way of latching a staple driver assembly into a cartridge housing of a curved surgical stapler.

### SUMMARY

The present invention provides a surgical instrument according to claim 1. The instrument comprises a body, a shaft extending distally from the body, and an end effector at a distal end of the shaft. The end effector includes a staple cartridge unit and a cartridge retaining member. The staple cartridge unit includes a housing that includes a plurality of staples, and an anvil opposed from the housing. The anvil and the housing are configured to cooperate to clamp tissue. The anvil is configured to form staples ejected from the housing into the clamped tissue. The cartridge retaining member includes an opposed pair of jaws configured to cooperate to grip the housing of the staple cartridge unit. At least one deflectable retention member is configured to removably secure the housing of the staple cartridge unit to the cartridge retaining member. The at least one deflectable retention member is presented by one of the staple cartridge unit or the cartridge retaining member. The at least one deflectable retention member is configured to be deflected relative to a portion of the one of the staple cartridge unit or the cartridge retaining member from an undeflected state to a deflected state by the other of the staple cartridge unit or the cartridge retaining member.

The present invention also provides a method of preparing a surgical instrument according to claim 12. The instrument includes a body, a shaft extending distally from the body, and an end effector at a distal end of the shaft. The method comprises, in response to a staple cartridge unit of the end effector being directed along an arcuate path relative to a cartridge retaining member of the end effector from a removed state toward an installed state, deflecting at least one deflectable retention member presented by one of the staple cartridge unit or the cartridge retaining member relative to a portion of the one of the staple cartridge unit or the cartridge retaining member from an undeflected state to a deflected state by the other of the staple cartridge unit or the cartridge retaining member, and after the at least one deflectable retention member has assumed the deflected state, retaining the staple cartridge unit with the cartridge retaining member. The staple cartridge unit comprises a housing that includes a plurality of staples, and an anvil opposed from the housing, wherein the anvil and the housing are configured to cooperate to clamp tissue, wherein the anvil is configured to form staples ejected from the housing into the clamped tissue.

### BRIEF DESCRIPTION OF THE DRAWINGS

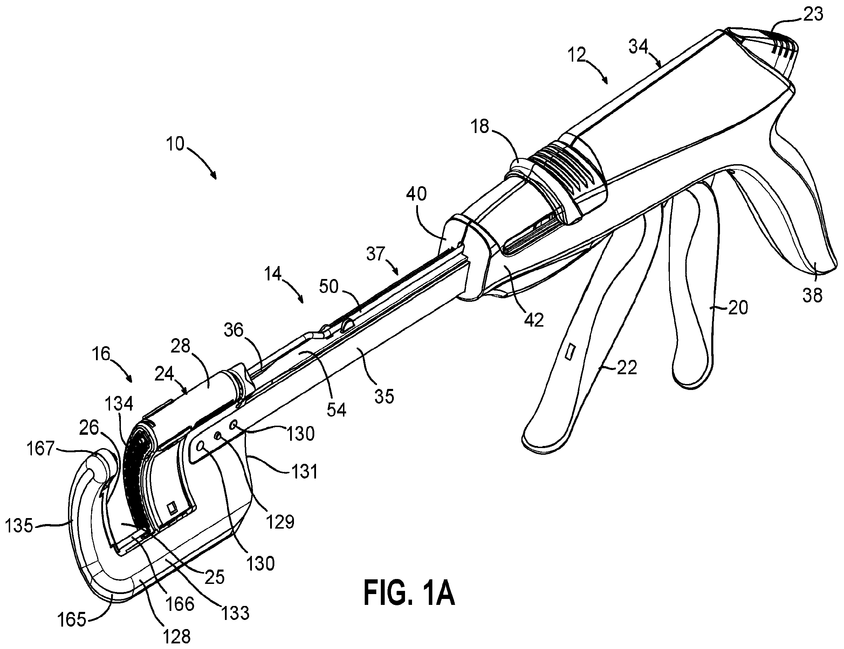

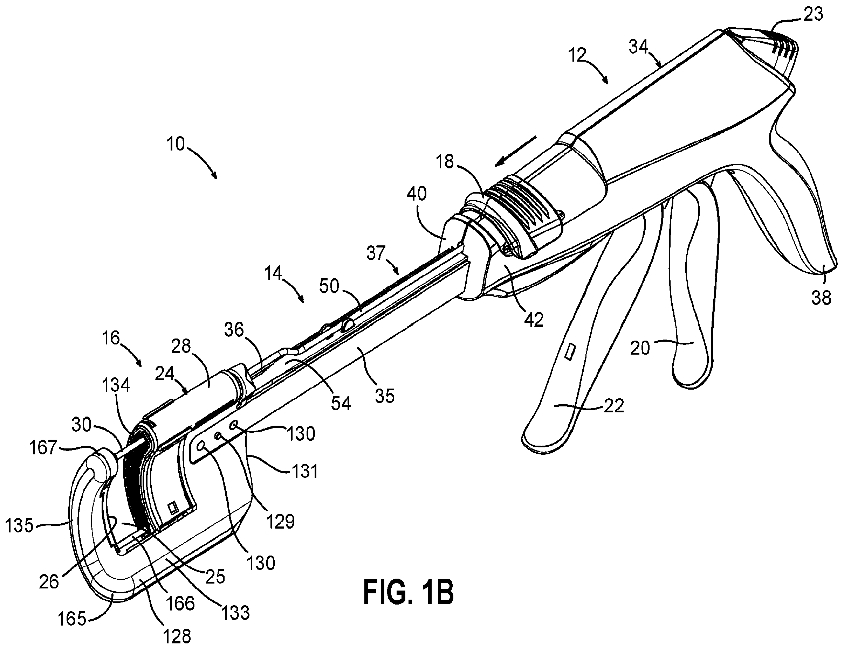

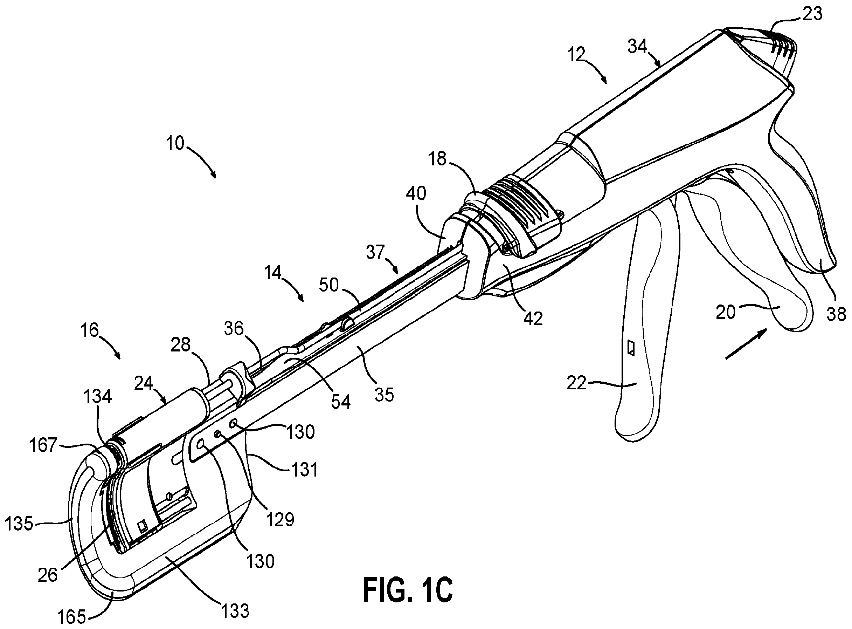

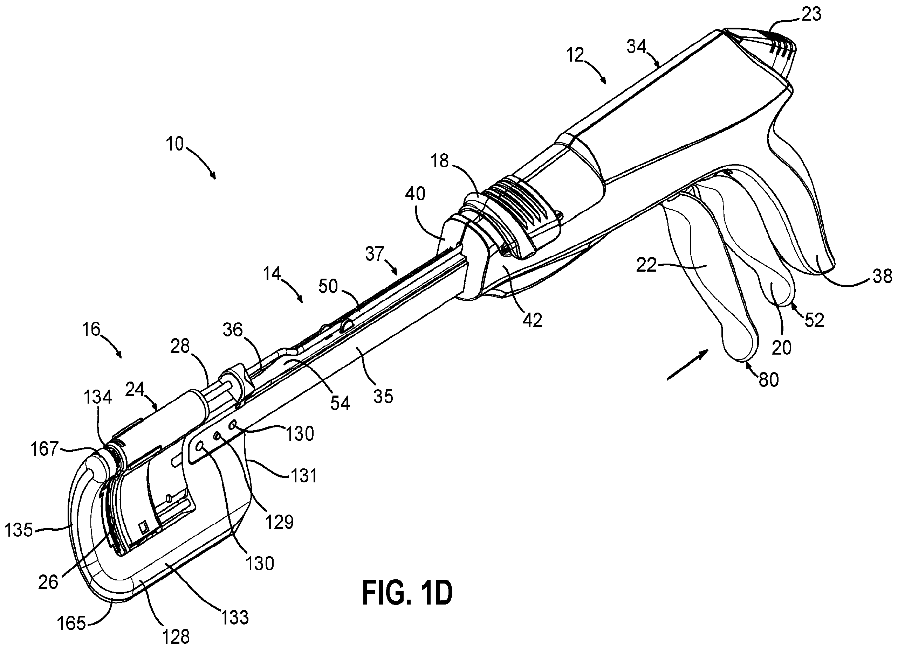

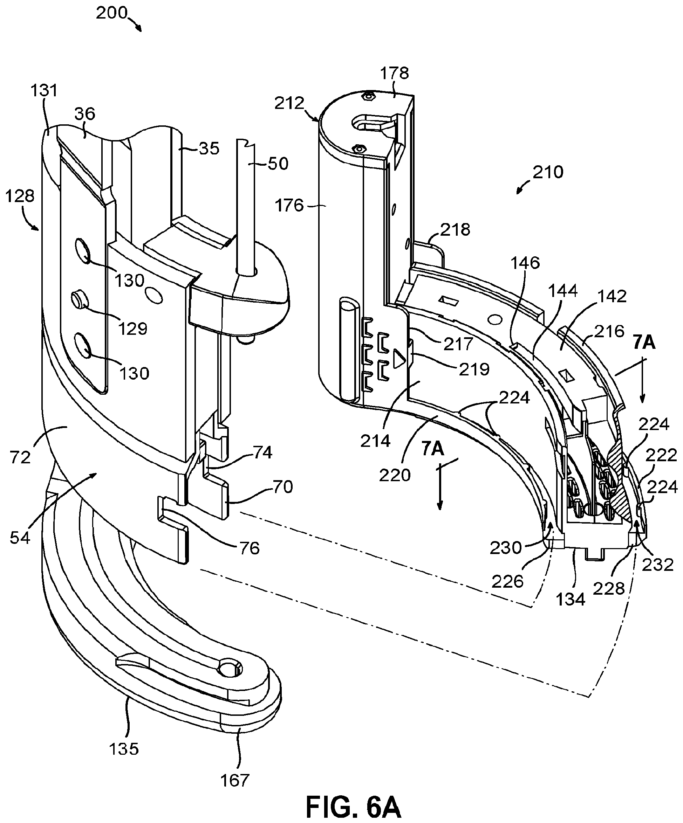

The accompanying drawings, which are incorporated in and constitute a part of this specification, illustrate embodiments of the invention in FIGS. 6A - 40, and background and contextual material in FIGS. 1 - 5. These, together with the general description of the invention given above, and the detailed description given below, serve to explain the principles of the present invention.
FIG. 1A depicts a perspective view of an exemplary surgical stapling instrument with a pin actuation mechanism in an open position and a staple cartridge unit of an end effector of the instrument in an open position;
FIG. 1B depicts a perspective view of the surgical stapling instrument of FIG. 1A with the pin actuation mechanism in a closed position and the staple cartridge unit in the open position;
FIG. 1C depicts a perspective view of the surgical stapling instrument of FIG. 1A with the pin actuation mechanism in the closed position and the staple cartridge unit in a closed position via actuation of a closure mechanism;
FIG. 1D depicts a perspective view of the surgical stapling instrument of FIG. 1A with the pin actuation mechanism and the staple cartridge unit in the closed positions and a firing trigger in a fired position for stapling and cutting tissue of a patient;
FIG. 2 depicts a partially exploded front perspective view of the surgical stapling instrument of FIG. 1A, showing the staple cartridge unit removed from a remainder of an end effector;
FIG. 3 depicts a distal perspective view of the staple cartridge unit of FIG. 2;
FIG. 4 depicts a proximal perspective view of the staple cartridge unit of FIG. 2;
FIG. 5 depicts an exploded perspective view of the staple cartridge unit of FIG. 2;
FIG. 6A depicts an exploded perspective view of an exemplary end effector for use with the surgical stapling instrument of FIG. 1A, showing an exemplary staple cartridge unit with deflectable rounded ribs removed from the closure member of the end effector, with the anvil of the staple cartridge unit omitted;
FIG. 6B depicts a perspective view of the end effector of FIG. 6A, showing the staple cartridge unit installed within the closure member of the end effector, with the anvil of the staple cartridge unit omitted;
FIG. 7A depicts a cross-sectional view of the staple cartridge unit removed from the closure member of the end effector of FIG. 6A, taken along section line 7A-7A in FIG. 6A, showing a deflectable rounded rib of the staple cartridge unit in an undeflected state;
FIG. 7B depicts a cross-sectional view of the staple cartridge unit installed within the closure member of the end effector of FIG. 6A, taken along section line 7B-7B in FIG. 6B, showing the deflectable rounded rib urged to a deflected state by a jaw of the closure member;
FIG. 8 depicts a cross-sectional view of another exemplary staple cartridge unit for use with the end effector of FIG. 6A, showing a deflectable pointed rib of the staple cartridge unit;
FIG. 9A depicts an exploded perspective view of another exemplary end effector for use with the surgical stapling instrument of FIG. 1A, showing an exemplary staple cartridge unit with a deflectable detent removed from the closure member of the end effector, with the anvil of the staple cartridge unit omitted;
FIG. 9B depicts a perspective view of the end effector of FIG. 9A, showing the staple cartridge unit installed within the closure member of the end effector, with the anvil of the staple cartridge unit omitted;
FIG. 10 depicts a magnified perspective view of the end effector of FIG. 9B, showing the deflectable detent engaging a jaw of the closure member;
FIG. 11 depicts a magnified perspective view of another exemplary staple cartridge unit and closure member for use with the surgical stapling instrument of FIG. 1A, showing a deflectable detent of the staple cartridge unit engaging a jaw of the closure member;
FIG. 12A depicts an exploded perspective view of another exemplary end effector for use with the surgical stapling instrument of FIG. 1A, showing an exemplary staple cartridge unit with a ramped groove surface removed from an exemplary closure member of the end effector, with the anvil of the staple cartridge unit omitted;
FIG. 12B depicts a perspective view of the end effector of FIG. 12A, showing the staple cartridge unit installed within the closure member of the end effector, with the anvil of the staple cartridge unit omitted;
FIG. 13 depicts a magnified side elevational view of the staple cartridge unit removed from the closure member of the end effector of FIG. 12A, showing the ramped groove surface of the staple cartridge unit aligned with a detent of the closure member;
FIG. 14 depicts a cross-sectional view of the staple cartridge unit removed from the closure member of the end effector of FIG. 12A, taken along section line 14-14 in FIG. 13, showing the ramped groove surface of the staple cartridge unit aligned with the detent of the closure member;
FIG. 15 depicts a cross-sectional view of another exemplary staple cartridge unit with a ramped groove surface removed from the closure member of the end effector of FIG. 12A, showing the ramped groove surface of the staple cartridge unit aligned with the detent of the closure member;
FIG. 16 depicts a cross-sectional view of another exemplary staple cartridge unit with a ramped groove surface removed from the closure member of the end effector of FIG. 12A, showing the ramped groove surface of the staple cartridge unit aligned with the detent of the closure member;
FIG. 17A depicts an exploded perspective view of another exemplary end effector for use with the surgical stapling instrument of FIG. 1A, showing an exemplary staple cartridge unit with a deflectable detent removed from an exemplary closure member of the end effector, with the anvil of the staple cartridge unit omitted;
FIG. 17B depicts a perspective view of the end effector of FIG. 17A, showing the staple cartridge unit installed within the closure member of the end effector, with the anvil of the staple cartridge unit omitted;
FIG. 18A depicts a magnified perspective view of the end effector of FIG. 17A, showing the deflectable detent aligned with a notch of the closure member;
FIG. 18B depicts a magnified perspective view of the end effector of FIG. 17A, showing the deflectable detent received within the notch of the closure member;
FIG. 19 depicts a perspective view of another exemplary end effector for use with the surgical stapling instrument of FIG. 1A, showing an exemplary staple cartridge unit with a deflectable detent installed within an exemplary closure member of the end effector;
FIG. 20 depicts a magnified perspective view of the end effector of FIG. 19, taken within area 20 in FIG. 19, showing the deflectable detent received within an aperture of the closure member;
FIG. 21 depicts a cross-sectional view of the end effector of FIG. 19, taken along section line 21-21 in FIG. 20, showing the deflectable detent received within the aperture;
FIG. 22 depicts a partial perspective view of another exemplary end effector for use with the surgical stapling instrument of FIG. 1A, showing an exemplary staple cartridge unit installed within an exemplary closure member having a deflectable recess, and further showing the deflectable recess engaged with a static detent of the staple cartridge unit;
FIG. 23 depicts a partial perspective view of another exemplary end effector for use with the surgical stapling instrument of FIG. 1A, showing an exemplary staple cartridge unit installed within an exemplary closure member having a plurality of deflectable recesses;
FIG. 24 depicts a magnified perspective view of the end effector of FIG. 23, showing one of the deflectable recesses engaged with a static detent of the staple cartridge unit;
FIG. 25 depicts a perspective view of another exemplary end effector for use with the surgical stapling instrument of FIG. 1A, showing an exemplary staple cartridge unit installed within the closure member of the end effector, and further showing an exemplary support structure having an embedded magnet;
FIG. 26 depicts a perspective view of another exemplary end effector for use with the surgical stapling instrument of FIG. 1A, showing an exemplary staple cartridge unit with a plurality of deflectable recesses installed within an exemplary closure member of the end effector;
FIG. 27 depicts a magnified perspective view of the end effector of FIG. 26, taken within area 27 in FIG. 26, showing two of the deflectable recesses engaged with respective static detents of the closure member;
FIG. 28 depicts a cross-sectional view of the end effector of FIG. 26, taken along section line 28-28 in FIG. 27, showing the deflectable recesses engaged with the respective static detents;
FIG. 29 depicts a perspective view of another exemplary end effector for use with the surgical stapling instrument of FIG. 1A, showing an exemplary staple cartridge unit installed within an exemplary closure member having a plurality of deflectable recesses;
FIG. 30 depicts a magnified perspective view of the end effector of FIG. 29, showing one of the deflectable recesses engaged with a static detent of the staple cartridge unit;
FIG. 31 depicts a cross-sectional view of the end effector of FIG. 29, taken along section line 31-31 in FIG. 30, showing the deflectable recess engaged with the static detent;
FIG. 32 depicts a perspective view of another exemplary end effector for use with the surgical stapling instrument of FIG. 1A, showing an exemplary staple cartridge unit installed within an exemplary closure member having a pair of deflectable beams;
FIG. 33A depicts a magnified side elevational view of the staple cartridge unit removed from the closure member of the end effector of FIG. 32, showing the deflectable beams of the closure member aligned with corresponding detents of the staple cartridge unit and in respective undeflected states;
FIG. 33B depicts a magnified side elevational view of the end effector of FIG. 32, showing the deflectable beams of the closure member urged to respective deflected states by the corresponding detents of the staple cartridge unit;
FIG. 34 depicts a partial perspective view of another exemplary end effector for use with the surgical stapling instrument of FIG. 1A, showing an exemplary staple cartridge unit with a deflectable detent and corresponding release button installed within an exemplary closure member of the end effector;
FIG. 35 depicts a cross-sectional view of the end effector of FIG. 34, taken along section line 35-35 in FIG. 34, showing the deflectable detent received within an aperture of the closure member;
FIG. 36 depicts an exploded perspective view of another exemplary end effector for use with the surgical stapling instrument of FIG. 1A, showing an exemplary staple cartridge unit removed from an exemplary closure member of the end effector, and further showing a slidable catch removed from the staple cartridge unit, with the anvil of the staple cartridge unit omitted;
FIG. 37 depicts a magnified perspective view of the end effector of FIG. 36, showing the staple cartridge unit installed within the closure member of the end effector;
FIG. 38 depicts a magnified and cutaway perspective view of the end effector of FIG. 36, showing the slidable catch engaged with a static detent of the closure member;
FIG. 39 depicts an exploded perspective view of another exemplary end effector for use with the surgical stapling instrument of FIG. 1A, showing an exemplary staple cartridge unit removed from an exemplary closure member of the end effector, and further showing a pinchable catch removed from the staple cartridge unit, with the anvil of the staple cartridge unit omitted; and
FIG. 40 depicts a perspective view of the end effector of FIG. 39, showing the staple cartridge unit installed within the closure member of the end effector, with the anvil of the staple cartridge unit omitted.

The drawings are not intended to be limiting in any way. The accompanying drawings incorporated in and forming a part of the specification illustrate several aspects of the present invention, and together with the description serve to explain the principles of the invention; it being understood, however, that this invention is not limited to the precise arrangements shown.

### DETAILED DESCRIPTION

For clarity of disclosure, the terms "proximal" and "distal" are defined herein relative to a human or robotic operator of the surgical instrument. The term "proximal" refers the position of an element closer to the human or robotic operator of the surgical instrument and further away from the surgical end effector of the surgical instrument. The term "distal" refers to the position of an element closer to the surgical end effector of the surgical instrument and further away from the human or robotic operator of the surgical instrument. It will be further appreciated that for convenience and clarity, spatial terms such as "vertical," "horizontal," "lower," "upper," "front," and "rear" are used herein with respect to the drawings. However, surgical instruments are used in many orientations and positions, and these terms are not intended to be limiting and/or absolute.

As used herein, the terms "about," "approximately," and the like in connection with any numerical values or ranges of values are intended to encompass the exact value(s) referenced, as well as a suitable dimensional tolerance that enables the referenced feature or combination of features to function for the intended purpose described herein.

### I. Overview of Exemplary Surgical Stapler

FIGS. 1A-1D depict an exemplary surgical stapler (10) that includes a handle assembly (12), a shaft assembly (14) extending distally from handle assembly (12), and an end effector (16) at a distal end of shaft assembly (14). It should be understood that terms such as "proximal," "distal," "right," and "left" are used herein with reference to a clinician gripping handle assembly (12) of surgical stapler (10). Thus, end effector (16) is distal with respect to the relatively proximal handle assembly (12). Except as otherwise described herein, surgical stapler (10) may be configured and operable in accordance with at least some of the teachings of U.S. Pat. Pub. No. 2005/0143759, entitled "Curved Cutter Stapler Shaped for Male Pelvis," published on June 30, 2005, now abandoned; and/or U.S. Pat. No. 10,194,913, entitled "Surgical Instrument Comprising Systems for Assuring the Proper Sequential Operation of the Surgical Instrument," issued on February 5, 2019.

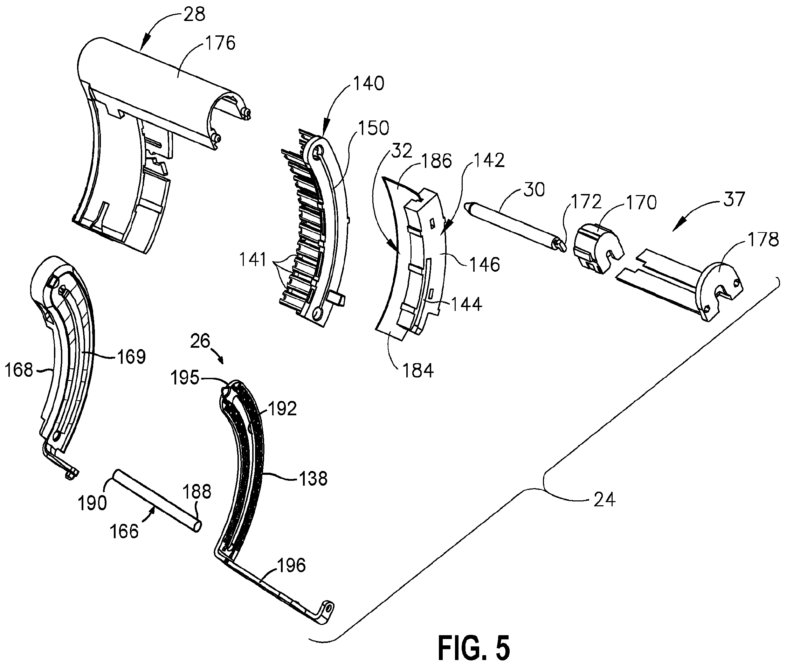

Handle assembly (12) includes several actuation mechanisms for operating end effector (16) during the surgical procedure. To this end, exemplary handle assembly (12) includes a saddle shaped slide (18), a closure trigger (20), and a firing trigger (22) in communication with end effector (16) via shaft assembly (14). FIG. 1A shows slide (18) and closure trigger (20) in open configurations such that end effector (16) is configured to receive tissue laterally within a gap (25) of a replaceable cartridge unit (24) mounted within end effector (16), between an anvil (26) and a cartridge housing (28) of cartridge unit (24). As described in greater detail below, translating slide (18) distally toward end effector (16) slides a retaining pin (30) of end effector (16) distally, as shown in FIG. 1B, for capturing the tissue between anvil (26) and cartridge housing (28). As shown in FIGS. 1C and 1D, sequentially actuating closure trigger (20) and firing trigger (22) respectively compresses the tissue between anvil (26) and cartridge housing (28) in a closed configuration, and then forms a plurality of staples (not shown) within the tissue and severs the tissue with a curved knife (32) (see FIG. 5).

### A. Handle Assembly and Shaft Assembly of Surgical Stapler

As shown in FIG. 1A, handle assembly (12) of surgical stapler (10) includes a handle housing (34) and a pair of handle frame plates (35, 36) having proximal portions (not shown) housed within handle housing (34) and elongate distal portions that extend distally along shaft assembly (14). As briefly described above, handle assembly (12) further includes saddle shaped slide (18), closure trigger (20), and firing trigger (22). Handle housing (34) defines a hand grip (38), which the operator may grasp with the palm of at least one hand. Handle housing (34) of the present example is formed by a right shroud handle portion (40) and a left shroud handle portion (42). Closure trigger (20) is proximally positioned relative to firing trigger (22), and each trigger (20, 22) is pivotally mounted to frame plates (35, 36) and are exposed through an underside of handle housing (34) to be manipulated by the fingers of the operator. FIG. 1A shows closure and firing triggers (20, 22) in unactuated positions prior to the closing of end effector (16) and firing of staples (not shown) and curved knife (32). Accordingly, cartridge housing (28) is spaced proximally from anvil (26) for receiving tissue within gap (25) therebetween.

Surgical stapler (10) is operable to capture tissue via a tissue retaining pin actuation mechanism (37) prior to actuation of the closure and firing triggers (20, 22). Tissue retaining pin actuation mechanism (37) includes slide (18) of handle assembly (12), a tissue retaining pin (30) of end effector (16), and an elongate pushrod (50) of shaft assembly (14). Slide (18) is mounted on an upper surface of handle housing (34) and is configured to linearly translate between proximal and distal positions. Pushrod (50) operatively couples slide (18) with tissue retaining pin (30), such that longitudinal translation of slide (18) drives longitudinal actuation of tissue retaining pin (30) between a proximal open position (see FIG. 1A) and a distal closed position (see FIG. 1B), via pushrod (50).

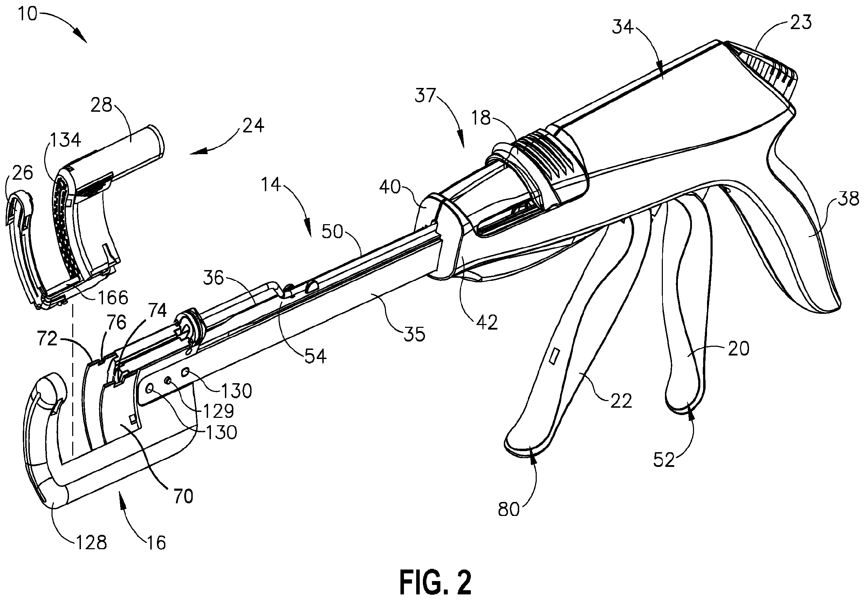

A closure mechanism (52) of surgical stapler (10) is configured to selectively actuate cartridge housing (28) of cartridge unit (24) between a proximal open position (FIG. 1A) and a distal closed position (FIG. 1C) for clamping tissue between cartridge housing (28) and anvil (26). Closure mechanism (52) includes closure trigger (20) of handle assembly (12) and an elongate closure member (54) coupled at its proximal end with closure trigger (20). Closure member (54) has a generally U-shaped cross-section and extends distally from handle assembly (12), through shaft assembly (14), and into end effector (16), such that a distal end of closure member (54) is configured to receive cartridge unit (24) within end effector (16), as shown in FIG. 2. In this regard, the distal end of closure member (54) includes opposed first and second C-shaped jaws (70, 72) extending along respective lateral sides thereof for gripping replaceable cartridge unit (24). More particularly, first jaw (70) extends along a concave first lateral side of the distal end of closure member (54), and second jaw (72) extends along a convex second lateral side of the distal end of closure member (54). First and second jaws (70, 72) are spaced apart from each other by a suitable distance for securely receiving cartridge unit (24) therebetween. More particularly, first jaw (70) is configured to frictionally engage a concave first lateral side of cartridge unit (24) and second jaw (72) is configured to frictionally engage a convex second lateral side of cartridge unit (24) when cartridge unit (24) is removably installed in closure member (54). As shown in FIG. 2, first and second jaws (70, 72) include first and second upper notches (74, 76) extending downwardly from upper edges thereof, respectively, the purposes of which are described below.

A proximal end of closure member (54) is operatively connected with closure trigger (20) by a plurality of linkages (not shown) configured to convert pivoting motion of closure trigger (20) into translation of closure member (54). Accordingly, pivoting of closure trigger (20) toward pistol grip (38) to a closed position (FIG. 1C) drives closure member (54) distally, which in turn drives cartridge housing (28) distally toward anvil (26) for clamping tissue therebetween. Subsequently, pivoting of closure trigger (20) away from pistol grip (38) to an open position (FIG. 1A) drives closure member (54) proximally, which in turn drives cartridge housing (28) proximally away from anvil (26) for releasing stapled tissue.

In some versions, closure member (54) may be further configured to cooperate with tissue retaining pin actuation mechanism (37) to automatically actuate retaining pin (30) distally to its closed position when the operator squeezes closure trigger (20). Such automation may be useful in the event that the operator did not manually actuate retaining pin (30) distally via slide (18) prior to squeezing closure trigger (20). Closure trigger (20) may be biased toward the open position by a resilient member (not shown) housed within handle housing (34).

A firing mechanism (80) of surgical stapler (10) is configured to actuate end effector (16) to staple and sever tissue clamped between anvil (26) and cartridge housing (28) in response to manipulation of firing trigger (22) of handle assembly (12). In that regard, firing mechanism (80) includes firing trigger (22), cartridge unit (24), and an elongate firing bar (not shown) that extends longitudinally through shaft assembly (14) and operatively couples firing trigger (22) with cartridge unit (24). Firing trigger (22) is positioned distally of closure trigger (20) such that firing trigger (22) may be pivoted closed only once closure trigger (20) has first been pivoted closed. Pivoting of firing trigger (22) from an open position (FIG. 1C) toward a closed (or "fired") position (FIG. 1D) drives the firing bar distally, which in turn drives internal components of cartridge housing (28) distally to thereby staple and sever the tissue clamped by end effector (16), as described in greater detail below.

One or both of closure trigger (20) and firing trigger (22) may be configured to releasably lock in one or more pivot positions, such as a fully closed position and/or one or more intermediate positions between fully open (i.e., unactuated) and fully closed (i.e., fully actuated), for example. Accordingly, and advantageously, the operator may release one or more hands from the trigger (20, 22) and hand grip (38) to perform another task during the surgical procedure, while the trigger (20, 22) maintains its position. The operator may then release the trigger (20, 22) from its locked state by depressing a release button (23) arranged on a proximal end of handle assembly (12).

Though not shown, shaft assembly (14) of surgical stapler (10) may include various additional components, such as an articulating joint, or may include a rearrangement of various components such that shaft assembly (14) may be modular relative to handle assembly (12).

### B. End Effector of Surgical Stapler

As shown best in FIGS. 1A-5, end effector (16) of the present example includes a C-shaped support structure (128) and replaceable cartridge unit (24) removably received by C-shaped support structure (128). The support structure (128) includes a proximal base portion (131), a medial arm (133) that extends distally from proximal base portion (131), and a distal arm (135) that projects laterally (e.g., upwardly) from a distal end of medial arm (133). Proximal base portion (131) is secured to the distal end of handle frame plates (35, 36) at the distal end of shaft assembly (14) by a shoulder rivet (129) and a pair of posts (130). Distal arm (135) includes a fixed end (165) secured to medial arm (133), and a free end (167). Proximal base portion (131) is configured to releasably receive and support cartridge housing (28), and distal arm (135) is configured to releasably receive and support anvil (26) and washer (168).

The term "C-shaped" is used herein as reference to the curvature of support structure (128) and cartridge unit (24), each of which has a concave first lateral side and a convex second lateral side opposed from one another. In other words, support structure (128) and each component of cartridge unit (24) extends along a respective arcuate path in a respective plane that is orthogonal to and intersected by a longitudinal axis defined by shaft assembly (14). Such a configuration provides enhanced functionality and access to tissue within the patient. By way of example only, the C-shaped construction of support structure (128) and cartridge unit (24) may enable end effector (16) to easily access the lower colon within the pelvic bowl of a patient, for example for performing a lower anterior resection ("LAR") in a proctocolectomy procedure. Accordingly, the term "C-shaped" as used herein should be construed to include a variety of concave shapes that would similarly enhance the functionality of surgical stapling and cutting instruments. In other versions, cartridge unit (24) and support structure (128) may be shaped in various other curved and non-curved manners. For instance, cartridge unit (24) and support structure (128) may be formed or shaped with a linear configuration, for example as described in U.S. Pub. No. 2020/0337698, entitled "Tissue Cutting Washer for Right Angle Surgical Stapler," published October 29, 2020.

Replaceable cartridge unit (24) includes anvil (26) and cartridge housing (28), movably coupled to one another by a guide pin (166) and an anvil arm (196), as described in greater detail below. A distal end of cartridge housing (28) defines a distally facing staple deck (134) configured to contact tissue. Staple deck (134) includes a plurality of staple openings (136) arranged in staggered formation in a pair of rows on each side of an arcuate knife slot (152). Various other quantities of rows of staple openings (136) may be provided in other versions. Cartridge housing (28) houses a plurality of staples (not shown) configured to be driven distally through staple openings (136) and against anvil (26) to thereby form the staples in patient tissues. Though not shown, cartridge unit (24) may further include a retainer configured to removably couple to staple deck (134) to cover staple openings (136) and knife slot (152) before use of cartridge unit (24), for instance when cartridge unit (24) is stored, and optionally also after use of cartridge unit (24).

As shown in FIG. 5, cartridge housing (28) additionally houses retaining pin (30), a staple driver assembly (140), and a knife holder (142). Staple driver assembly (140) is positioned just proximally of the staples (not shown) housed within cartridge housing (28) and distally of knife holder (142). Staple driver assembly (140) of the present example is formed as a unitary structure defining a plurality of staple drivers (141). Thus, the term "assembly," as used in connection with staple driver assembly (140), is not intended to be limited to an assembly of individual components, but may also include integrally formed components with unitary structures. Driver assembly (140) is configured to translate distally within cartridge housing (28) so that staple drivers (141) drive staples distally from respective staple openings (136) and toward anvil (26) for formation within tissue clamped between anvil (26) and cartridge housing (28).

Knife holder (142) is movably disposed within cartridge housing (28) just proximally of staple driver assembly (140). Knife holder (142) supports curved knife (32) along a distal side thereof, and knife holder (142) is configured to translate within cartridge housing (28) such that curved knife (32) extends distally through an arcuate slot (150) of driver assembly (140) and arcuate slot (152) of staple deck (134). A proximal side of knife holder (142) includes a slot (144) and a ledge (146) configured to couple with a knife retractor hook (not shown) for retraction of curved knife (32) after firing of cartridge unit (24), for example as disclosed in U.S. Pat. No. 10,045,780, entitled "Method of Applying Staples in Lower Anterior Bowel Resection," issued August 14, 2018.

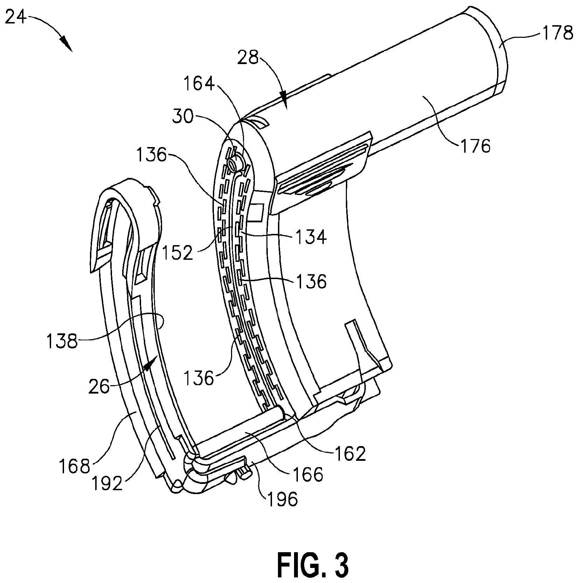

As shown in FIG. 3, cartridge housing (28) includes two longitudinally extending, generally circular holes (162, 164) at respective upper and lower ends of arcuate knife slot (152) on staple deck (134). Holes (162, 164) of the present example are positioned such that staple openings (136), and the staples ejected therefrom, extend beyond holes (162, 164) at the upper and lower ends of staple deck (134). Lower hole (162) is shaped and dimensioned to slidably receive a guide pin (166) that extends longitudinally between cartridge housing (28) and anvil (26). Upper hole (164) is shaped and dimensioned to slidably receive retaining pin (30) therethrough, such that retaining pin (30) may actuate longitudinally relative to cartridge housing (28) and anvil (26) between the proximal retracted position (FIG. 1A) and the distal extended position (FIG. 1B).

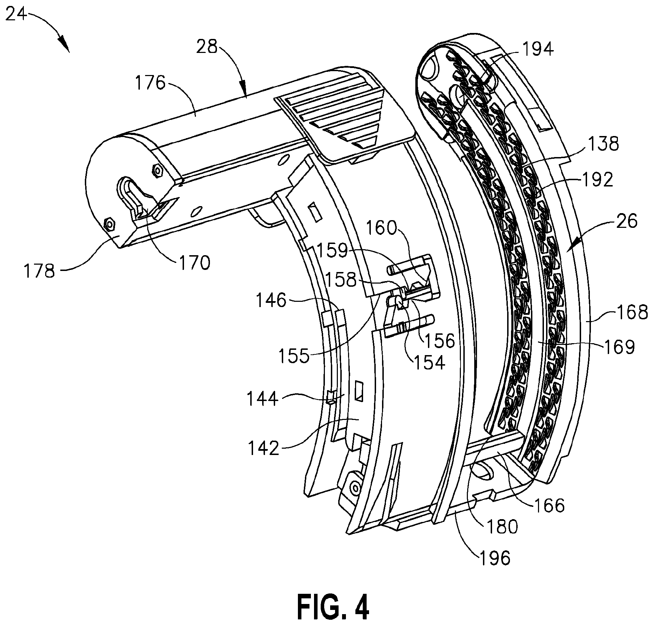

As shown in FIG. 4, a lateral side of cartridge housing (28) includes a longitudinally extending detent slot (155) defined between a confronting pair of resilient members. A first side of detent slot (155) includes a first proximal detent protrusion (156), and an opposed second side of detent slot (155) includes a second proximal detent protrusion (159) and a distal detent protrusion (160). Detent slot (155) is configured to slidably receive a detent post (154) of knife holder (142) and a detent post (158) of staple driver assembly (140). As staple driver assembly (140) and knife holder (142) translate distally within cartridge housing (28), detent post (154) resiliently engages detent protrusion (156), and detent post (158) resiliently engages detent protrusions (159, 160).

As shown in FIG. 5, a proximal end of retaining pin (30) includes a first coupling feature (172) (e.g., a projection) configured to couple with a corresponding coupling feature (not shown) (e.g., a groove) of a couplet (170), so that retaining pin (30) is secured to couplet (170). Couplet (170) and retaining pin (30) are slidably disposed within an upper arm (176) of cartridge housing (28), and are captured proximally therein by an end cap (178) secured to upper arm (176) proximally of couplet (170). A distal end of pushrod (50) of tissue retaining pin actuation mechanism (37), described above, is operatively coupled with couplet (170). Accordingly, longitudinal actuation of pushrod (50) via slide (18) of handle assembly (12) drives couplet (170) and thus retaining pin (30) longitudinally relative to cartridge housing (28) for capturing tissue to be stapled by end effector (16).

Anvil (26) of the present example includes a plate portion (138), which is secured to a proximal side of an anvil plate backing member in the form of a plastic cutting washer (168). Anvil plate portion (138) includes an elongate arcuate slot (192) configured to receive a cutting feature of cutting washer (168) in the form of an arcuate projection (169) therethrough to secure anvil plate portion (138) relative to cutting washer (168). As shown best in FIG. 4, anvil plate portion (138) further includes a plurality of pockets arranged in rows along either side of arcuate slot (192). These pockets are configured to receive and form the legs of staples (not shown) driven distally from staple openings (136) of staple deck (134). Accordingly, anvil (26) is spaced distally from and is aligned with staple deck (134) such that each pocket of anvil plate portion (138) aligns with a respective staple opening (136).

Anvil plate portion (138) further includes a first circular opening (194) disposed at an upper end of arcuate slot (192), and a second circular opening (see FIG. 3) disposed at a lower end of arcuate slot (192). First opening (194) is configured to slidably receive a pointed distal tip of tissue retaining pin (30) when tissue retaining pin (30) is actuated distally to capture tissue positioned between anvil (26) and cartridge housing (28). The lower second opening of anvil plate portion (138) receives a distal end (190) of guide pin (166) therethrough, which extends into and fixedly couples to a lower end of cutting washer (168), such that guide pin (166) is longitudinally fixed relative to anvil (26).

A proximal end (188) of guide pin (166) is slidably received through lower hole (162) formed in staple deck (134) of cartridge housing (28), as described above. An anvil arm (196) projecting proximally from a lower end of anvil plate portion (138) is movably received through an open lower end of cartridge housing (28) to thereby trap proximal end (188) of guide pin (166) within cartridge housing (28), while still permitting cartridge housing (28) to actuate toward anvil (26). Accordingly, cartridge housing (28) is configured to slide longitudinally along guide pin (166) (and tissue retaining pin (30)) relative to anvil (26) in response to actuation of closure trigger (20), described above. As shown in FIG. 5, an interior side of guide pin (166) includes a longitudinal slot (180) configured to slidably receive a corresponding lower end (184) of curved knife (32) as cartridge housing (28) actuates longitudinally relative to anvil (26). An interior side of tissue retaining pin (30) may include a similar longitudinal slot (not shown) configured to slidably receive a corresponding upper end (186) of curved knife (32) as cartridge housing (28) actuates longitudinally relative to anvil (26).

### C. Exemplary Actuation of Surgical Stapler

Having described various structural features of surgical stapler (10) above, including cartridge unit (24), exemplary actuation of surgical stapler (10) during a surgical procedure will now be described below. Surgical stapler (10) is first suitably manipulated within a body cavity of a patient to position patient tissue within gap (25) (see FIG. 1A) between anvil (26) and cartridge housing (28). As shown in FIG. 1B, slide (18) is then actuated distally to drive pushrod (50) distally, thereby driving tissue retaining pin (30) distally from cartridge housing (28) toward anvil (26). The pointed distal tip of tissue retaining pin (30) securely engages (e.g., pierces) the tissue and thereby captures the tissue within gap (25).

As shown in FIG. 1C, closure trigger (20) is then squeezed toward pistol grip (38) to drive closure member (54) distally, thereby driving cartridge housing (28) distally toward anvil (26) along tissue retaining pin (30) and guide pin (166) to clamp the tissue between cartridge deck (134) and anvil (26). Cartridge housing (28) may be maintained in this closed position relative to anvil (26) by an internal locking mechanism (not shown) of handle assembly (12) that holds closure trigger (20) in the squeezed position, as described above. In the present version, firing trigger (22) pivots proximally to an intermediate position simultaneously with the squeezing of closure trigger (20). As shown in FIG. 1D, while cartridge unit (24) remains in this closed position, firing trigger (22) is then squeezed toward closure trigger (20) and pistol grip (38) to drive the elongate firing bar (not shown) distally, thereby driving staple driver assembly (140) and knife holder (142) distally within cartridge housing (28). Stapler drivers (141) of driver assembly (140) drive staples (not shown) distally through the captured tissue and against anvil plate portion (138) of anvil (26) to form the staples within the tissue and thereby fluidly seal the tissue. As the staples are being formed, curved knife (32) is driven distally by knife holder (142) through arcuate slots (150, 152), through the clamped tissue, and against arcuate projection (169) (see FIG. 5) of cutting washer (168), thereby severing the clamped tissue along an arcuate path extending between the two innermost rows of the formed staples. Upon cutting fully through the clamped tissue, curved knife (32) may penetrate distally into the arcuate projection (169) of cutting washer (168). Optionally, in response to such penetration, a body of cutting washer (168) may fracture along the distal cutting edge of curved knife (32), thereby providing an audible indication (e.g., via a "snapping" sound) to the surgeon that the firing stroke is complete and that the clamped tissue has been fully stapled and severed.

Similar to closure trigger (20), firing trigger (22) may be held in its squeezed position by the internal locking mechanism (not shown) of handle assembly (12). It will be appreciated that surgical stapler (10) may be configured in some versions such that the tissue clamped by end effector (16) within gap (25) is stapled and cut simultaneously; and be alternatively configured in other versions such that the tissue is fully stapled and subsequently cut in sequential steps.

Once surgical stapler (10) has been fully fired into the patient tissue as described above, the operator may depress release button (23) of handle assembly (12) to release firing trigger (22) and closure trigger (20) from their squeezed positions. In this manner, curved knife (32) may be retracted proximally back into cartridge housing (28), and cartridge housing (28) may be retracted proximally along pins (30, 166) to thereby release the newly stapled and severed tissue from between anvil (26) and cartridge deck (134). The fired cartridge unit (24) may then be removed from support structure (128) of end effector (16), discarded, and replaced for further treatment if so desired.

Surgical stapler (10) may be further configured and operable in accordance with any of the teachings of the references cited herein.

### II. Exemplary Cartridge Retention Features

In some instances, it may be desirable to provide cartridge retention features configured to provide improved mechanical engagement between cartridge unit (24) and closure member (54). Such improved mechanical engagement may increase the threshold insertion force that must be applied to cartridge unit (24) to fully install cartridge unit (24) in closure member (54) of end effector (16) and/or increase the threshold separation force that must be applied to cartridge unit (24) to remove cartridge unit (24) from closure member (54) of end effector (16). Increasing the threshold separation force may inhibit inadvertent dislodgement of cartridge unit (24) from closure member (54) that might otherwise result from shock, vibrations, and/or impacts experienced by surgical stapler (10) during transit and general handling, for example. Increasing the threshold separation force may also inhibit slight drifting of a fully loaded (i.e., unspent) cartridge unit (24) relative to closure member (54) that might otherwise be sufficient to prematurely activate a lockout feature (not shown) of surgical stapler (10) that is intended to prevent surgical stapler (10) from operating when cartridge unit (24) is either spent or not present within closure member (54), which may interfere with the operation of surgical stapler (10) despite the presence of fully loaded cartridge unit (24) within closure member (54). It may also be desirable to increase such threshold insertion and separation forces while maintaining such threshold insertion and separation forces within ergonomic ranges.

In addition, or alternatively, it may be desirable to provide cartridge retention features that provide audible and/or tactile feedback to the operator indicating that cartridge unit (24) is fully installed within or uninstalled from closure member (54). In some instances, it may be desirable to provide cartridge retention features that are resistant to wear to inhibit attrition of the threshold insertion and separation forces that might otherwise result from the cartridge retention features degrading (e.g., forming increased clearances between a pair of static interlocking retention features) during cyclic unloading and reloading of one or more cartridge unit(s) (24) from and into closure member (54). Thus, providing wear-resistant cartridge retention features may improve consistency of the retention of cartridge unit(s) (24) within closure member (54). Each of the end effectors (200, 300, 400, 500, 600, 700, 800, 900, 1000, 1100, 1200, 1300, 1400, 1500), closure members (402, 502, 602, 702, 802, 1002, 1102, 1202, 1302, 1402, 1502), and/or cartridge units (210, 310, 410, 510, 610, 710, 810, 910, 1010, 1110, 1210, 1310, 1410, 1510) described below provides one or more of these functionalities.

### A. Exemplary Cartridge Unit with Ribs on Outer Rails

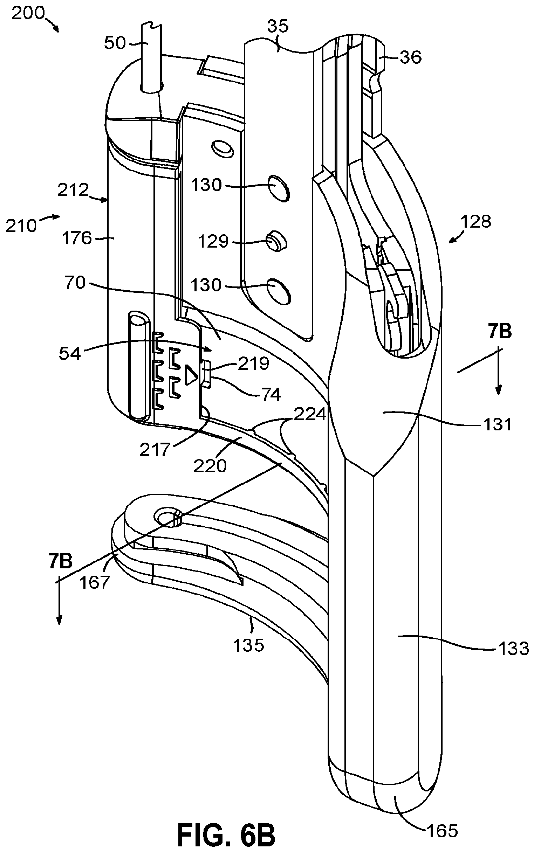

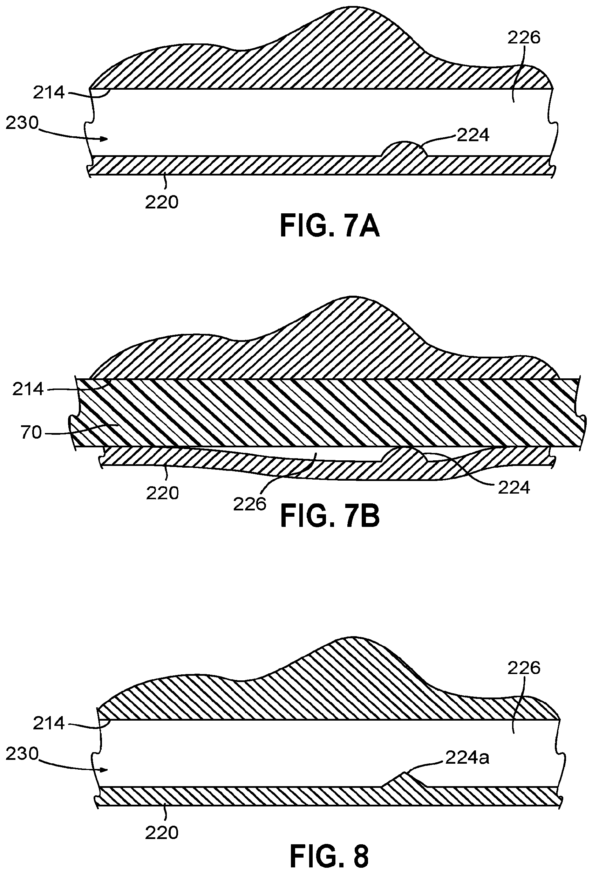

FIGS. 6A-7B show an exemplary end effector (200) including closure member (54), C-shaped support structure (128), and a replaceable cartridge unit (210) removably received by closure member (54). As described in greater detail below, end effector (200) has one or more cartridge retention features configured to provide improved mechanical engagement between cartridge unit (210) and closure member (54), at least relative to the mechanical engagement between cartridge unit (24) and closure member (54) of end effector (16). End effector (200) and cartridge unit (210) are similar to end effector (16) and cartridge unit (24) described above, respectively, except as otherwise described below. FIGS. 6A and 7A depict cartridge unit (210) removed from closure member (54), while FIGS. 6B and 7B depict cartridge unit (210) installed in closure member (54).

Cartridge unit (210) of the present example includes an anvil (not shown), such as anvil (26), movably coupled to a cartridge housing (212) similar to cartridge housing (28) described above except as otherwise described below. In this regard, cartridge housing (212) defines staple deck (134), houses various internal components including a plurality of staples (not shown), a retaining pin (not shown), such as retaining pin (30), a staple driver assembly (not shown), such as staple driver assembly (140), and knife holder (142), and includes upper arm (176) and end cap (178). As shown, cartridge housing (212) includes opposed first and second rigid, C-shaped sidewalls (214, 216) extending along respective lateral sides of cartridge unit (210). More particularly, first sidewall (214) extends along the concave first lateral side of cartridge unit (210), and second sidewall (216) extends along the convex second lateral side of cartridge unit (210). Cartridge housing (212) also includes opposed first and second upper flanges (217, 218) positioned laterally outwardly of upper portions of first and second sidewalls (214, 216), respectively. In the present version, cartridge housing (212) also includes at least one tab (219) (one shown) extending laterally inwardly from a respective flange (217, 218) toward a respective sidewall (214, 216), for insertion into a respective notch (74, 76) when cartridge unit (210) is installed in closure member (54), as best shown in FIG. 6B. In some versions, tab (219) may be coupled to the respective sidewall (214, 216).

As best shown in FIG. 6A, cartridge housing (212) further includes opposed first and second flexible, C-shaped guide rails (220, 222) extending downwardly from respective flanges (217, 218) along respective lateral sides of cartridge unit (210). More particularly, first rail (220) extends along a distal portion of the concave first lateral side of cartridge unit (210) laterally outwardly of first sidewall (214), and second rail (222) extends along a distal portion of the convex second lateral side of cartridge unit (210) laterally outwardly of second sidewall (216). A plurality of deflectable retention members in the form of rounded ribs (224) extend laterally inwardly from the laterally inner surfaces of first and second rails (220, 222), toward the respective sidewall (214, 216). In the example shown, each rib (224) has a generally semicircular cross-sectional shape and extends between proximal and distal ends of the respective rail (220, 222). In some versions, each rib (224) may extend laterally inwardly from the laterally inner surface of the respective rail (220, 222) by between approximately 0.003 inch and approximately 0.010 inch. In any event, ribs (224) of the present example are spaced apart from each other at substantially equal intervals along the lengths of the respective rails (220, 222), such that each rib (224) on first rail (220) may be generally laterally opposed from a corresponding rib (224) on second rail (222). It will be appreciated that ribs (224) may be configured and/or arranged in any other suitable manner(s).

In the present version, rails (220, 222) are each configured to flex laterally outwardly away from the respective sidewall (214, 216), and are resiliently biased laterally inwardly to their respective unflexed states, as shown in FIGS. 6A and 7A. As shown, first rail (220) is coupled to first sidewall (214) by a first support (226) extending between distal portions thereof, and second rail (222) is coupled to second sidewall (216) by a second support (228) extending between distal portions thereof, to assist in maintaining rails (220, 222) in their respective unflexed states in the absence of external forces acting upon rails (220, 222). In this manner, the laterally inner surface of first rail (220), a laterally outer surface of first sidewall (214), and a proximal surface of first support (226) collectively define a first groove (230). Likewise, the laterally inner surface of second rail (222), a laterally outer surface of second sidewall (216), and a proximal surface of second support (228) collectively define a second groove (232).

First and second grooves (230, 232) may be sized to slidably receive respective jaws (70, 72) of closure member (54) for guiding relative movement between cartridge unit (210) and closure member (54) during installation and/or removal of cartridge unit (210), such as for guiding notches (74, 76) along respective arcuate paths toward or away from the respective tabs (219), and for permitting jaws (70, 72) to frictionally engage the corresponding ribs (224). For example, first and second grooves (230, 232) may each have a width substantially equal to or greater than a thickness of the respective jaw (70, 72), and each rib (224) may be spaced apart from the corresponding sidewall (214, 216) by a lateral clearance that is substantially equal to or less than the thickness of the respective jaw (70, 72). As best shown in FIGS. 6B and 7B, when jaws (70, 72) are received within the respective grooves (230, 232), a laterally inner surface of each jaw (70, 72) may frictionally engage the laterally outer surface of the respective sidewall (214, 216), and a laterally outer surface of each jaw (70, 72) may urge the corresponding ribs (224) on the respective rail (220, 222), together with the adjacent portions of the respective rail (220, 222), laterally outwardly away from the respective sidewall (214, 216). In this manner, rails (220, 222) may each be urged laterally outwardly by the corresponding jaw (70, 72) from their respective unflexed states, as best shown with respect to first rail (220) in FIG. 7A, to their respective flexed states, as best shown with respect to first rail (220) in FIG. 7B.

As best shown in FIGS. 6B and 7B, when jaws (70, 72) are fully docked in the respective grooves (230, 232) (e.g., with tabs (219) of cartridge housing (212) received by the respective notches (74, 76)), ribs (224) may each be positioned laterally outwardly of the corresponding jaw (70, 72). Due to the biasing of rails (220, 222) toward their respective unflexed states, ribs (224) may each frictionally engage the respective jaw (70, 72) while rails (220, 222) remain in their respective flexed states, thereby sandwiching jaws (70, 72) against sidewalls (214, 216) for improved retention of cartridge unit (210) within closure member (54). In some versions, rails (220, 222) may be substantially rigid and ribs (224) may be deformable and biased toward their respective illustrated undeformed states, such that ribs (224) may be deformed laterally outwardly by the corresponding jaw (70, 72) from their respective undeformed states to their respective deformed states (not shown) for sandwiching jaws (70, 72) against sidewalls (214, 216). In such cases, ribs (224) may be referred to as "crush bumps."

In any event, ribs (224) may provide interference with the respective jaws (70, 72) at discrete points therealong to thereby increase the threshold insertion and separation forces for causing relative movement between cartridge unit (210) and closure member (54). Since ribs (224) are deflectable laterally outwardly by the respective jaws (70, 72) (e.g., via flexibility of rails (220, 222) and/or deformability of ribs (224)), ribs (224) and the laterally outer surfaces of jaws (70, 72) may each be resistant to wear, even as one or more cartridge units (210) are cyclically unloaded from and reloaded into closure member (54), at least by comparison to a pair of interlocking static cartridge retention features.

Thus, jaws (70, 72) may be advanced along the respective grooves (230, 232) (e.g., for guiding notches (74, 76) toward the respective tabs (219)) by applying a threshold insertion force to at least one of cartridge unit (210) or closure member (54) that is sufficient to overcome the interference between ribs (224) and the respective jaws (70, 72). Likewise, jaws (70, 72) may be retracted along the respective grooves (230, 232) (e.g., for guiding notches (74, 76) away from the respective tabs (219)) by applying a threshold separation force to at least one of cartridge unit (210) or closure member (54) that is sufficient to overcome the interference between ribs (224) and the respective jaws (70, 72).

In some instances, and as briefly described above, it may be desirable to configure ribs (224) in other suitable manners. For example, FIG. 8 depicts an exemplary alternative, pointed rib (224a) having a generally triangular cross-sectional shape extending laterally inwardly from the laterally inner surface of first rail (220) toward the respective sidewall (214, 216), between the proximal and distal ends of first rail (220). Rib (224a) may be spaced apart from first sidewall (214) by a lateral clearance that is substantially equal to or less than the thickness of first jaw (70), and may function in substantially the same manner as ribs (224).

While ribs (224, 224a) have been shown and described as extending laterally inwardly from respective rails (220, 222) for frictionally engaging respective jaws (70, 72), it will be appreciated that ribs (224, 224a) may alternatively extend laterally outwardly from respective jaws (70, 72) for frictionally engaging respective rails (220, 222) in an inverse arrangement of end effector (200).

### B. Exemplary Cartridge Unit with Lower, Laterally-Outwardly Facing Deflectable Cantilevered Beam

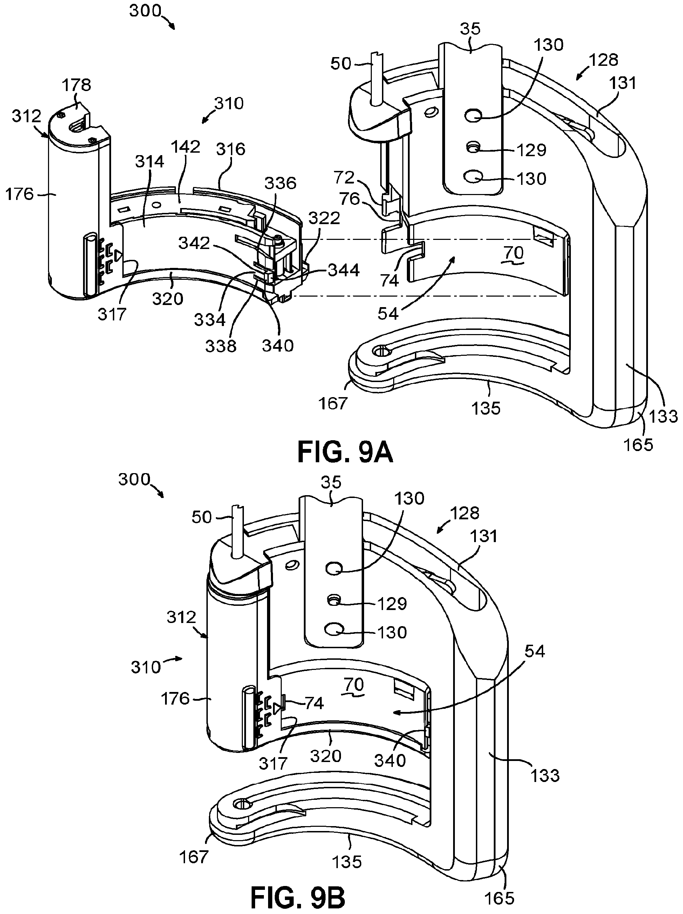

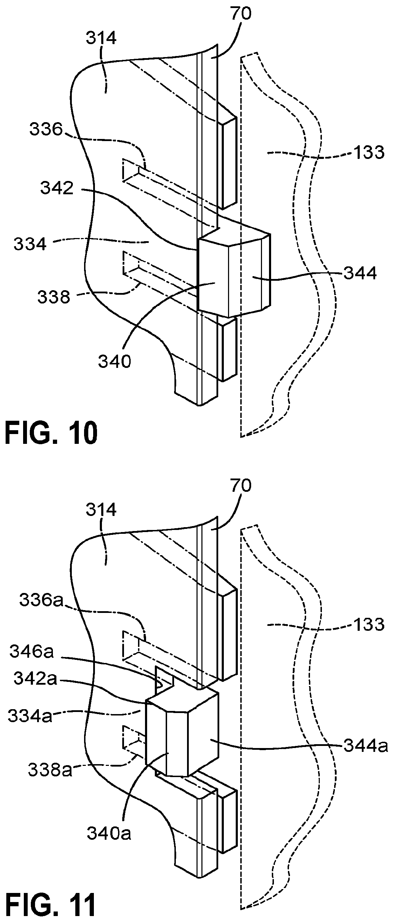

FIGS. 9A-10 show an exemplary end effector (300) including closure member (54), - C-shaped support structure (128), and a replaceable cartridge unit (310) removably received by closure member (54). As described in greater detail below, end effector (300) has one or more cartridge retention features configured to provide improved mechanical engagement between cartridge unit (310) and closure member (54), at least relative to the mechanical engagement between cartridge unit (24) and closure member (54) of end effector (16). End effector (300) and cartridge unit (310) are similar to end effector (16) and cartridge unit (24) described above, respectively, except as otherwise described below. FIG. 9A depicts cartridge unit (310) removed from closure member (54), while FIGS. 9B and 10 depict cartridge unit (310) installed in closure member (54).

Cartridge unit (310) of the present example includes an anvil (not shown), such as anvil (26), movably coupled to a cartridge housing (312) similar to cartridge housing (28) described above except as otherwise described below. In this regard, cartridge housing (312) defines a staple deck (not shown), such as staple deck (134), houses various internal components including a plurality of staples (not shown), a retaining pin (not shown), such as retaining pin (30), a staple driver assembly (not shown), such as staple driver assembly (140), and knife holder (142), and includes upper arm (176) and end cap (178). As shown, cartridge housing (312) includes opposed first and second rigid, C-shaped sidewalls (314, 316) extending along respective lateral sides of cartridge unit (310). More particularly, first sidewall (314) extends along the concave first lateral side of cartridge unit (310), and second sidewall (316) extends along the convex second lateral side of cartridge unit (310). Cartridge housing (312) also includes a first upper flange (317) positioned laterally outwardly of an upper portion of first sidewall (314) and opposed from a second upper flange (not shown) positioned laterally outwardly of an upper portion of second sidewall (316), and may include at least one tab (not shown) extending laterally inwardly from a respective flange (317) toward a respective sidewall (314, 316), for insertion into a respective notch (74, 76) when cartridge unit (310) is installed in closure member (54). Cartridge housing (312) also includes opposed first and second C-shaped guide rails (320, 322) extending downwardly from respective flanges (317) along respective lateral sides of cartridge unit (310) and spaced apart from the respective sidewalls (314, 316) by grooves (not shown), similar to grooves (230, 232) described above, for slidably receiving respective jaws (70, 72) of closure member (54), in a manner similar to that described above with respect to cartridge unit (210).

As best shown in FIG. 9A, cartridge housing (312) further includes a flexible lower beam (334) defined between generally parallel proximal and distal slots (336, 338) which extend upwardly from a lower edge of first sidewall (314). Thus, beam (334) may be integrally formed with a remainder of first sidewall (314) and cantilevered relative thereto. In the present version, beam (334) extends below the lower edge(s) of the remainder of first sidewall (314). It will be appreciated that beam (334) may be configured and/or arranged in any other suitable manner(s). In any event, a deflectable retention member in the form of a detent (340) extends laterally outwardly from a lower end of beam (334) and includes an upper flat abutment surface (342) and a lower tapered ramp surface (344), the purposes of which are described below.

In the present version, beam (334) is configured to flex laterally inwardly toward second sidewall (316), and is resiliently biased laterally outwardly to its unflexed state, as shown in FIGS. 9A and 10. The integrally formed, cantilevered configuration of beam (334) relative to the remainder of first sidewall (314) may assist in maintaining beam (334) in its unflexed state in the absence of external forces acting upon beam (334). When jaws (70, 72) are initially received within the respective grooves of cartridge housing (312), such as for guiding notches (74, 76) toward the respective tabs of cartridge housing (312), the laterally inner surface of first jaw (70) may cammingly engage ramp surface (344) of detent (340) to thereby urge detent (340), together with beam (334), laterally inwardly toward second sidewall (316) to permit advancement of jaws (70, 72) along the respective grooves. In this regard, ramp surface (344) may be oriented relative to the laterally inner surface of beam (334) at a predetermined angle selected to provide a desired camming engagement with the laterally inner surface of first jaw (70).

As best shown in FIGS. 9B and 10, when jaws (70, 72) are fully docked in the respective grooves (e.g., with the tabs of cartridge housing (312) received by the respective notches (74, 76)), detent (340) may be positioned below a lower edge of first jaw (70), such that first jaw (70) disengages ramp surface (344). Due to the biasing of beam (334) toward its unflexed state, beam (334) may return to its unflexed state to allow abutment surface (342) to latch against the lower edge of first jaw (70) for improved retention of cartridge unit (310) within closure member (54). Such latching of abutment surface (342) against the lower edge of first jaw (70) may coincide with a "hard stop" between anvil arm (196) of cartridge unit (310) and medial arm (133) of support structure (128). In some versions, beam (334) may be substantially rigid and detent (340) may be deformable and biased toward its illustrated undeformed state, such that detent (340) may be deformed laterally inwardly by first jaw (70) from its undeformed state to a deformed state (not shown) for permitting advancement of jaws (70, 72) along the respective grooves, and may return to its undeformed state when jaws (70, 72) are fully docked in the respective grooves.

In any event, abutment surface (342) may securely latch against the lower edge of first jaw (70) to thereby increase the threshold separation force for removing cartridge unit (310) from closure member (54). Moreover, the biasing of detent (340) toward its unflexed state may increase the threshold insertion force for inserting cartridge unit (310) into closure member (54). Since detent (340) is deflectable laterally inwardly by first jaw (70) (e.g., via flexibility of beam (334) and/or deformability of detent (340)), detent (340) and the lower edge of first jaw (70) may each be resistant to wear, even as one or more cartridge units (310) are cyclically unloaded from and reloaded into closure member (54), at least by comparison to a pair of interlocking static cartridge retention features. In some versions, the movement of detent (340) into latching engagement with the lower edge of first jaw (70) may generate an audible and/or tactile response (e.g., via a "clicking" sound) indicating that cartridge unit (310) is fully installed within closure member (54).

Thus, jaws (70, 72) may be advanced along the respective grooves of cartridge housing (312) (e.g., for guiding notches (74, 76) toward the respective tabs) by applying a threshold insertion force to at least one of cartridge unit (310) or closure member (54) that is sufficient to overcome the biasing of detent (340) and thereby urge detent (340) laterally inwardly. Jaws (70, 72) may be retracted along the respective grooves of cartridge housing (312) (e.g., for guiding notches (74, 76) away from the respective tabs) by applying a threshold unlatching force to detent (340) that is sufficient to overcome the biasing of detent (340) and thereby urge detent (340) laterally inwardly while also applying a separation force to at least one of cartridge unit (310) or closure member (54). In some versions, the lower edge of first jaw (70) may cammingly engage abutment surface (342) of detent (340) to thereby urge detent (340), together with beam (334), laterally inwardly toward second sidewall (316) to permit retraction of jaws (70, 72) along the respective grooves, such that the threshold unlatching force may be achieved via application of a threshold separation force to at least one of cartridge unit (310) or closure member (54). In this regard, abutment surface (342) may be oriented relative to the laterally outer surface of beam (334) at a predetermined angle selected to provide a desired camming engagement with the lower edge of first jaw (70).

In some instances, it may be desirable to reduce or eliminate the distance by which detent (340) extends below the lower edge of first jaw (70) when jaws (70, 72) are fully docked in the respective grooves of cartridge housing (312). For example, FIG. 11 depicts an exemplary lower notch (346a) extending upwardly from the lower edge of first jaw (70). FIG. 11 further depicts an exemplary alternative beam (334a) defined between proximal and distal slots (336a, 338a) extending upwardly from the lower edge first sidewall (314), with a detent (340a) having an abutment surface (342a) and a ramp surface (344a). As shown, beam (334a) is relatively short, at least by comparison to beam (334), and is substantially coextensive with the remainder of first sidewall (314) in the downward direction. Thus, abutment surface (342a) may securely latch against a recessed lower edge of first jaw (70) defined by an upper edge of notch (346a) when first jaw (70) is fully docked in a respective groove of cartridge housing (312) (e.g., upon reaching the "hard stop" between anvil arm (196) of cartridge unit (310) and medial arm (133) of support structure (128)), such that detent (340a) may remain substantially above the lower edge of first jaw (70), thereby reducing or eliminating the distance by which detent (340a) extends below the lower edge of first jaw (70), at least by comparison to detent (340) and first jaw (70).

While beams (334, 334a) have been shown and described as being provided on first sidewall (314) for engaging first jaw (70), beams (334, 334a) may alternatively be provided on second sidewall (316) for engaging second jaw (72). In some versions, first and second beams (334, 334a) may be provided on first and second sidewalls (314, 316), respectively, for engaging first and second jaws (70, 72), respectively. It will also be appreciated that beams (334, 334a) may alternatively be provided on one or more jaws (70, 72) (e.g., at or near an upper edge thereof) for engaging one or more sidewalls (314, 316) (e.g., at or near an upper edge thereof) in an inverse arrangement of end effector (300).

### C. Exemplary Cartridge Unit with Ramped Groove Surface

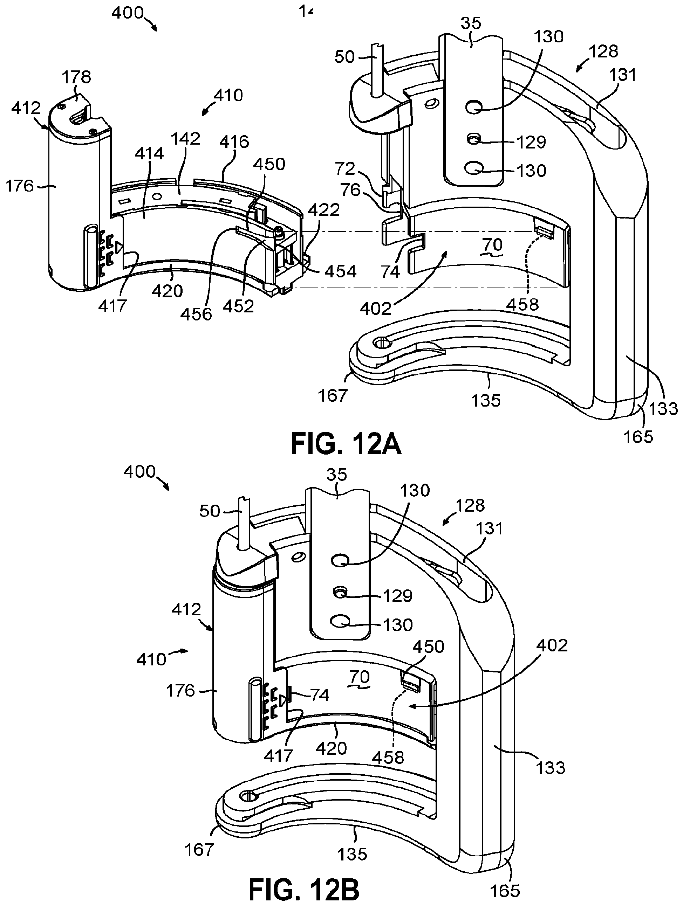

FIGS. 12A-14 show an exemplary end effector (400) including C-shaped support structure (128), a closure member (402), and a replaceable cartridge unit (410) removably received by closure member (402). As described in greater detail below, end effector (400) has one or more cartridge retention features configured to provide improved mechanical engagement between cartridge unit (410) and closure member (402), at least relative to the mechanical engagement between cartridge unit (24) and closure member (54) of end effector (16). End effector (400), closure member (402), and cartridge unit (410) are similar to end effector (16), closure member (54), and cartridge unit (24) described above, respectively, except as otherwise described below. FIGS. 12A, 13, and 14 depict cartridge unit (410) removed from closure member (402), while FIG. 12B depicts cartridge unit (410) installed in closure member (402).

Cartridge unit (410) of the present example includes an anvil (not shown), such as anvil (26), movably coupled to a cartridge housing (412) similar to cartridge housing (28) described above except as otherwise described below. In this regard, cartridge housing (412) defines a staple deck (not shown), such as staple deck (134), houses various internal components including a plurality of staples (not shown), a retaining pin (not shown), such as retaining pin (30), a staple driver assembly (not shown), such as staple driver assembly (140), and knife holder (142), and includes upper arm (176) and end cap (178). As shown, cartridge housing (412) includes opposed first and second rigid, C-shaped sidewalls (414, 416) extending along respective lateral sides of cartridge unit (410). More particularly, first sidewall (414) extends along the concave first lateral side of cartridge unit (410), and second sidewall (416) extends along the convex second lateral side of cartridge unit (410). Cartridge housing (412) also includes a first upper flange (417) positioned laterally outwardly of an upper portion of first sidewall (414) and opposed from a second upper flange (not shown) positioned laterally outwardly of an upper portion of second sidewall (416), and may include at least one tab (not shown) extending laterally inwardly from a respective flange (417) toward a respective sidewall (414, 416), for insertion into a respective notch (74, 76) when cartridge unit (410) is installed in closure member (402). Cartridge housing (412) also includes opposed first and second C-shaped guide rails (420, 422) extending downwardly from respective flanges (417) along respective lateral sides of cartridge unit (410) and spaced apart from the respective sidewalls (414, 416) by grooves (not shown), similar to grooves (230, 232) described above, for slidably receiving respective jaws (70, 72) of closure member (402), in a manner similar to that described above with respect to cartridge unit (210).

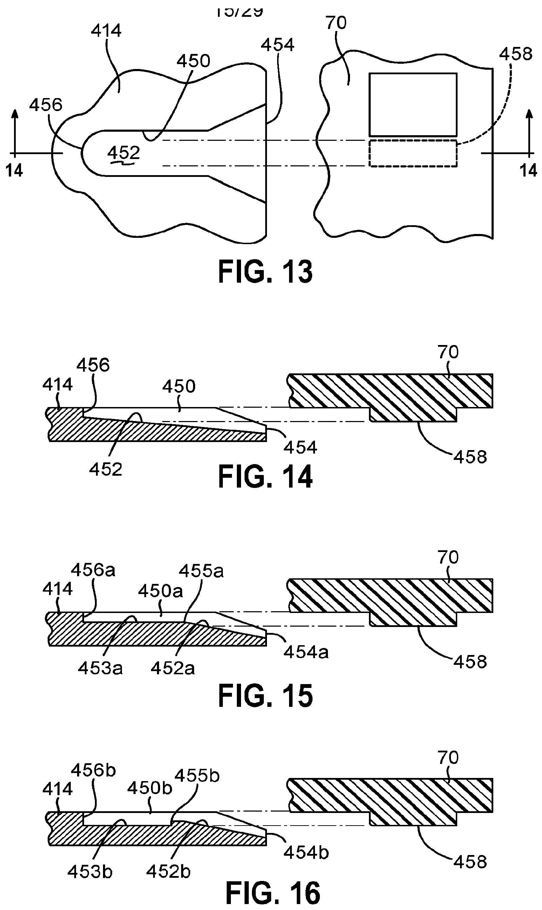

As best shown in FIG. 12A, cartridge housing (412) further includes a retention member in the form of a Y-shaped groove (450) extending upwardly from a lower edge of first sidewall (414) and laterally inwardly from a laterally outer surface thereof. In the present version, groove (450) is at least partially defined by a Y-shaped, tapered ramp surface (452) and has an open lower end (454) at the lower edge of first sidewall (414) and a closed upper end (456). The term "Y-shaped" is used herein as reference to the relatively wide, angled portion of groove (450) near lower end (454) and the relatively narrow, straight portion of groove (450) near upper end (456). As best shown in FIG. 14, ramp surface (452) tapers laterally outwardly in an upward direction from lower end (454) to upper end (456), such that groove (450) has a first depth at lower end (454) and a second depth at upper end (456) that is less than the first depth. It will be appreciated that groove (450), including ramp surface (452), may be configured and/or arranged in any other suitable manner(s).

Closure member (402) of the present example includes first and second jaws (70, 72) including first and second upper notches (74, 76). A retention member in the form of a detent (458) extends laterally inwardly from the laterally inner surface of first jaw (70) of closure member (402) toward second jaw (72) for insertion into groove (450) when cartridge unit (410) is installed in closure member (402), as best shown in FIG. 12B. In this regard, and as best shown in FIG. 14, detent (458) has a thickness less than or equal to the first depth of groove (450) at or near lower end (454) and greater than the second depth of groove (450) at or near upper end (456). It will be appreciated that detent (458) may be configured and/or arranged in any other suitable manner(s).

When jaws (70, 72) are initially received within the respective grooves of cartridge housing (412), such as for guiding notches (74, 76) toward the respective tabs of cartridge housing (412), the laterally inner surface of first jaw (70) may frictionally engage the laterally outer surface of first sidewall (414) while groove (450) loosely receives detent (458) at lower end (454). Due to the tapering of ramp surface (452) laterally outwardly in the upward direction, detent (458) may begin to frictionally engage ramp surface (452) as jaws (70, 72) are advanced along the respective grooves of cartridge housing (412), and such frictional engagement may gradually increase as such advancement continues. In the present version, the frictional engagement between detent (458) and ramp surface (452) is maximized when jaws (70, 72) are fully docked in the respective grooves with detent (458) at or near upper end (456) of groove (450), for improved retention of cartridge unit (410) within closure member (402). In some versions, detent (458) and ramp surface (452) may be deflectable laterally away from each other.

In any event, detent (458) may frictionally engage ramp surface (452) of groove (450) to thereby increase the threshold insertion and separation forces for causing relative movement between cartridge unit (410) and closure member (402). Since detent (458) and ramp surface (452) may be deflectable laterally away from each other (e.g., via flexibility of first jaw (70), deformability of detent (458), and/or flexibility of first sidewall (414)), detent (458) and ramp surface (452) may each be resistant to wear, even as one or more cartridge units (410) are cyclically unloaded from and reloaded into closure member (402), at least by comparison to a pair of interlocking static cartridge retention features.

Thus, jaws (70, 72) may be advanced along the respective grooves of cartridge housing (412) (e.g., for guiding notches (74, 76) toward the respective tabs) by applying a gradually increasing threshold insertion force to at least one of cartridge unit (410) or closure member (402) that is sufficient to overcome the gradually increasing frictional engagement between detent (458) and groove (450) as detent (458) approaches upper end (456) of groove (450). Jaws (70, 72) may be retracted along the respective grooves of cartridge housing (412) (e.g., for guiding notches (74, 76) away from the respective tabs) by applying a gradually decreasing threshold separation force to at least one of cartridge unit (410) or closure member (402) that is sufficient to overcome the gradually decreasing frictional engagement between detent (458) and groove (450) as detent (458) approaches lower end (454) of groove (450).

In some instances, it may be desirable to vary the frictional engagement between detent (458) and the surface(s) of groove (450) within a first range of motion, and to maintain the frictional engagement between detent (458) and the surface(s) of groove (450) substantially constant within a second range of motion during advancement and/or retraction of jaws (70, 72) along the respective grooves of cartridge housing (412).

For example, FIG. 15 depicts an exemplary alternative groove (450a) at least partially defined by a lower tapered ramp surface (452a) and an upper non-tapered dwell surface (453a). Ramp surface (452a) extends between an open lower end (454a) at the lower edge of first sidewall (414) and an intermediate edge (455a), and non-tapered dwell surface (453a) extends between intermediate edge (455a) and a closed upper end (456a). In the present version, ramp surface (452a) tapers laterally outwardly in an upward direction from lower end (454a) to intermediate edge (455a), and dwell surface (453a) extends substantially parallel to the laterally outer surface of first sidewall (414) between intermediate edge (455a) and upper end (456a), such that groove (450a) has a first depth at lower end (454a) and a second depth between intermediate edge (455a) and upper end (456a) that is less than the first depth. Detent (458) has a thickness less than or equal to the first depth of groove (450a) at or near lower end (454a) and greater than the second depth of groove (450a) between intermediate edge (455a) and upper end (456a).

Thus, the frictional engagement between detent (458) and ramp surface (452a) may gradually increase toward a maximum value during initial advancement of jaws (70, 72) along the respective grooves of cartridge housing (412) within a first range of motion defined by ramp surface (452a), and the frictional engagement between detent (458) and dwell surface (453a) may subsequently be maintained substantially constant at the maximum value during continued advancement of jaws (70, 72) along the respective grooves of cartridge housing (412) within a second range of motion defined by dwell surface (453a). Similarly, the frictional engagement between detent (458) and dwell surface (453a) may be maintained substantially constant at the maximum value during initial retraction of jaws (70, 72) along the respective grooves of cartridge housing (412) within the second range of motion, and the frictional engagement between detent (458) and ramp surface (452a) may subsequently gradually decrease below the maximum value during continued retraction of jaws (70, 72) along the respective grooves of cartridge housing (412) within the first range of motion.

In some instances, it may be desirable to vary the frictional engagement between detent (458) and the surface(s) of groove (450) within a first range of motion, and to release the frictional engagement between detent (458) and the surface(s) of groove (450) within a second range of motion during advancement and/or retraction of jaws (70, 72) along the respective grooves of cartridge housing (412). It may also be desirable to latch detent (458) against an edge of groove (450) when the frictional engagement between detent (458) and the surface(s) of groove (450) is released within the second range of motion.

For example, FIG. 16 depicts an exemplary alternative groove (450b) at least partially defined by a lower tapered ramp surface (452b) and an upper non-tapered release surface (453b). Ramp surface (452b) extends between an open lower end (454b) at the lower edge of first sidewall (414) and an intermediate ledge (455b), and release surface (453b) extends between intermediate ledge (455b) and a closed upper end (456b). In the present version, ramp surface (452a) tapers laterally outwardly in an upward direction from lower end (454a) to intermediate ledge (455b), and dwell surface (453b) extends substantially parallel to the laterally inner surface of first sidewall (414) between intermediate ledge (455b) and upper end (456b) laterally inwardly of intermediate ledge (455b), such that groove (450b) has a first depth at lower end (454b), a second depth at intermediate ledge (455b) that is less than the first depth, and a third depth between intermediate ledge (455b) and upper end (456b) that is greater than the second depth. Detent (458) has a thickness less than or equal to the first depth of groove (450b) at or near lower end (454b), greater than the second depth of groove (450b) between intermediate ledge (455b), and less than or equal to the third depth of groove (450b) between intermediate ledge (455b) and upper end (456b).

Thus, the frictional engagement between detent (458) and ramp surface (452b) may gradually increase toward a maximum value during initial advancement of jaws (70, 72) along the respective grooves of cartridge housing (412) within a first range of motion defined by ramp surface (452b), and the frictional engagement between detent (458) and release surface (453b) may subsequently be substantially reduced (or eliminated) relative to the maximum value during continued advancement of jaws (70, 72) along the respective grooves of cartridge housing (412) within a second range of motion defined by release surface (453b). In any event, detent (458) may securely latch against intermediate ledge (455b) for improved retention of cartridge unit (410) within closure member (402) (e.g., upon reaching the "hard stop" between anvil arm (196) of cartridge unit (410) and medial arm (133) of support structure (128)). In some versions, the movement of detent (458) into latching engagement with intermediate ledge (455b) may generate an audible and/or tactile response (e.g., via a "clicking" sound) indicating that cartridge unit (410) is fully installed within closure member (402).

While grooves (450, 450a, 450b) have been shown and described as being provided on first sidewall (414) and detent (458) has been shown and described as being provided on first jaw (70), grooves (450, 450a, 450b) may alternatively be provided on second sidewall (416) and detent (458) may be provided on second jaw (72). In some versions, first and second grooves ((450, 450a, 450b) may be provided on first and second sidewalls (414, 416), respectively, and first and second detents (458) may be provided on first and second jaws (70, 72), respectively, for engagement therewith. It will also be appreciated that grooves (450, 450a, 450b) may alternatively be provided on one or more jaws (70, 72) (e.g., on a laterally inner side thereof) and detent (458) may be provided on one or more sidewalls (414, 416) (e.g., on a laterally outer side thereof) in an inverse arrangement of end effector (400).

### D. Exemplary Cartridge Unit with Upper, Proximally-Extending Deflectable Cantilevered Beam

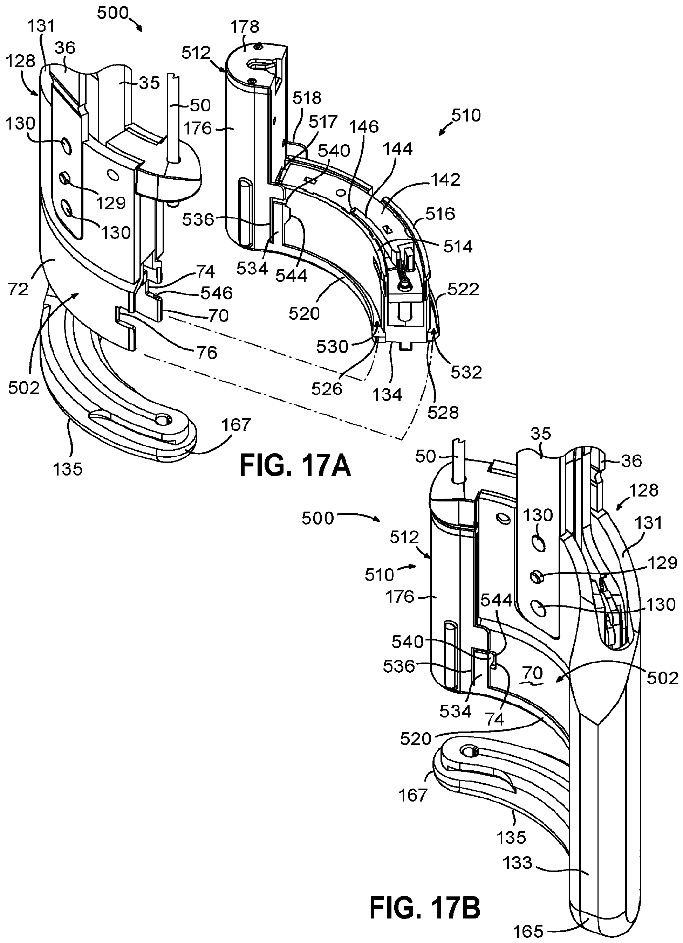

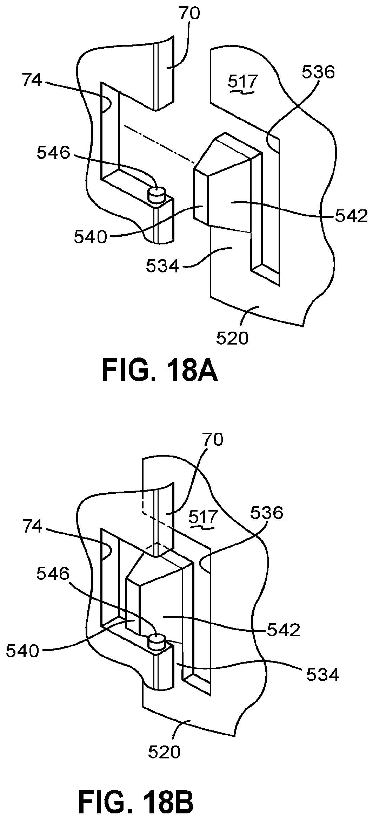

FIGS. 17A-18B show an exemplary end effector (500) including C-shaped support structure (128), a closure member (502), and a replaceable cartridge unit (510) removably received by closure member (502). As described in greater detail below, end effector (500) has one or more cartridge retention features configured to provide improved mechanical engagement between cartridge unit (510) and closure member (502), at least relative to the mechanical engagement between cartridge unit (24) and closure member (54) of end effector (16). End effector (500), closure member (502), and cartridge unit (510) are similar to end effector (16), closure member (54), and cartridge unit (24) described above, respectively, except as otherwise described below. FIGS. 17A and 18A depict cartridge unit (510) removed from closure member (502), while FIGS. 17B and 18B depict cartridge unit (510) installed in closure member (502).

Cartridge unit (510) of the present example includes an anvil (not shown), such as anvil (26), movably coupled to a cartridge housing (512) similar to cartridge housing (28) described above except as otherwise described below. In this regard, cartridge housing (512) defines staple deck (134), houses various internal components including a plurality of staples (not shown), a retaining pin (not shown), such as retaining pin (30), a staple driver assembly (not shown), such as staple driver assembly (140), and knife holder (142), and includes upper arm (176) and end cap (178). As shown, cartridge housing (512) includes opposed first and second rigid, C-shaped sidewalls (514, 516) extending along respective lateral sides of cartridge unit (510). More particularly, first sidewall (514) extends along the concave first lateral side of cartridge unit (510), and second sidewall (516) extends along the convex second lateral side of cartridge unit (510). Cartridge housing (512) also includes opposed first and second upper flanges (517, 518) positioned laterally outwardly of upper portions of first and second sidewalls (514, 516), respectively. Cartridge housing (512) also includes opposed first and second C-shaped guide rails (520, 522) extending downwardly from respective flanges (517, 518) along respective lateral sides of cartridge unit (510), coupled to the respective sidewalls (514, 516) by respective supports (526, 528), and spaced apart from the respective sidewalls (514, 516) by respective grooves (530, 532) for slidably receiving respective jaws (70, 72) of closure member (502), in a manner similar to that described above with respect to cartridge unit (210).

As best shown in FIG. 17A, cartridge housing (512) further includes a flexible upper beam (534) extending proximally from first rail (520) and separated from first flange (517) by an L-shaped slot (536) such that beam (534) may be integrally formed with first rail (520) and cantilevered relative thereto. It will be appreciated that beam (534) may be configured and/or arranged in any other suitable manner(s). In any event, a deflectable retention member in the form of a detent (540) extends laterally inwardly from a proximal end of beam (534) toward first sidewall (514) for insertion into first notch (74) when cartridge unit (510) is installed in closure member (502), as best shown in FIG. 17B. Detent (540) includes an upper tapered abutment surface (542) and a lower tapered ramp surface (544), the purposes of which are described below. In the present version, beam (534) is configured to flex laterally outwardly away from first sidewall (514), and is resiliently biased laterally inwardly to its unflexed state, as shown in FIGS. 17A-18B. The integrally formed, cantilevered configuration of beam (534) relative to first rail (520) may assist in maintaining beam (534) in its unflexed state in the absence of external forces acting upon beam (534).

Closure member (502) of the present example includes first and second jaws (70, 72) including first and second upper notches (74, 76). A static (e.g., non-deflectable) retention member in the form of a bump (546) extends proximally from an upper portion of a distal surface of first notch (74) of closure member (502) for engaging detent (540) when cartridge unit (510) is installed in closure member (502), as best shown in FIG. 18B. It will be appreciated that bump (546) may be configured and/or arranged in any other suitable manner(s). For example, in some versions bump (546) may be deflectable.

When jaws (70, 72) are initially received within the respective grooves (530, 532), such as for guiding first notch (74) toward detent (540), bump (546) of first jaw (70) may cammingly engage ramp surface (544) of detent (540) to thereby urge detent (540), together with beam (534), laterally outwardly away from first sidewall (514) to permit advancement of jaws (70, 72) along the respective grooves (530, 532). In this regard, ramp surface (544) may be oriented relative to the laterally outer surface of beam (534) at a predetermined angle selected to provide a desired camming engagement with bump (546).

As best shown in FIGS. 17B and 18B, when jaws (70, 72) are fully docked in the respective grooves (530, 532), detent (540) may be positioned below bump (546), such that bump (546) disengages ramp surface (544). Due to the biasing of beam (534) toward its unflexed state, beam (534) may return to its unflexed state to allow abutment surface (542) to latch against bump (546) for improved retention of cartridge unit (510) within closure member (502). Such latching of abutment surface (542) against bump (546) may coincide with a "hard stop" between anvil arm (196) of cartridge unit (510) and medial arm (133) of support structure (128). In some versions, beam (534) may be substantially rigid and detent (540) may be deformable and biased toward its illustrated undeformed state, such that detent (540) may be deformed laterally outwardly by bump (546) from its undeformed state to a deformed state (not shown) for permitting advancement of jaws (70, 72) along the respective grooves (530, 532), and may return to its undeformed state when jaws (70, 72) are fully docked in the respective grooves (530, 532).

In any event, abutment surface (542) may securely latch against bump (546) to thereby increase the threshold separation force for removing cartridge unit (510) from closure member (502). Moreover, the biasing of detent (540) toward its unflexed state may increase the threshold insertion force for inserting cartridge unit (510) into closure member (502). Since detent (540) is deflectable laterally outwardly by bump (546) (e.g., via flexibility of beam (534) and/or deformability of detent (540)), detent (540) and bump (546) may each be resistant to wear, even as one or more cartridge units (510) are cyclically unloaded from and reloaded into closure member (502), at least by comparison to a pair of interlocking static cartridge retention features. In some versions, the movement of detent (540) into latching engagement with bump (546) may generate an audible and/or tactile response (e.g., via a "clicking" sound) indicating that cartridge unit (510) is fully installed within closure member (502).

Thus, jaws (70, 72) may be advanced along the respective grooves (530, 532) (e.g., for guiding first notch (74) toward detent (540)) by applying a threshold insertion force to at least one of cartridge unit (510) or closure member (502) that is sufficient to overcome the biasing of detent (540) and thereby urge detent (540) laterally outwardly. Jaws (70, 72) may be retracted along the respective grooves (530, 532) (e.g., for guiding first notch (74) away from detent (540)) by applying a threshold unlatching force to detent (540) that is sufficient to overcome the biasing of detent (540) and thereby urge detent (540) laterally outwardly while also applying a separation force to at least one of cartridge unit (510) or closure member (502). In some versions, bump (546) of first jaw (70) may cammingly engage abutment surface (542) of detent (540) to thereby urge detent (540), together with beam (534), laterally outwardly away from first sidewall (514) to permit retraction of jaws (70, 72) along the respective grooves (530, 532), such that the threshold unlatching force may be achieved via application of a threshold separation force to at least one of cartridge unit (510) or closure member (502). In this regard, abutment surface (542) may be oriented relative to the laterally inner surface of beam (534) at a predetermined angle selected to provide a desired camming engagement with bump (546).

While beam (534) of the present version is provided on first rail (520) and bump (546) is provided on first jaw (70), beam (534) may alternatively be provided on second rail (522) and bump (546) may be provided on second jaw (72). In some versions, first and second beams (534) may be provided on first and second rails (520, 522), respectively, and first and second bumps (546) may be provided on first and second jaws (70, 72), respectively, for engagement therewith. It will also be appreciated that beam (534) may alternatively be provided on one or more jaws (70, 72) (e.g., at or near an upper edge thereof) and bump (546) may be provided on one or more flanges (517, 518) or sidewalls (514, 516) in an inverse arrangement of end effector (500).

### E. Exemplary Cartridge Unit with Upper, Downwardly-Extending Deflectable Cantilevered Beam

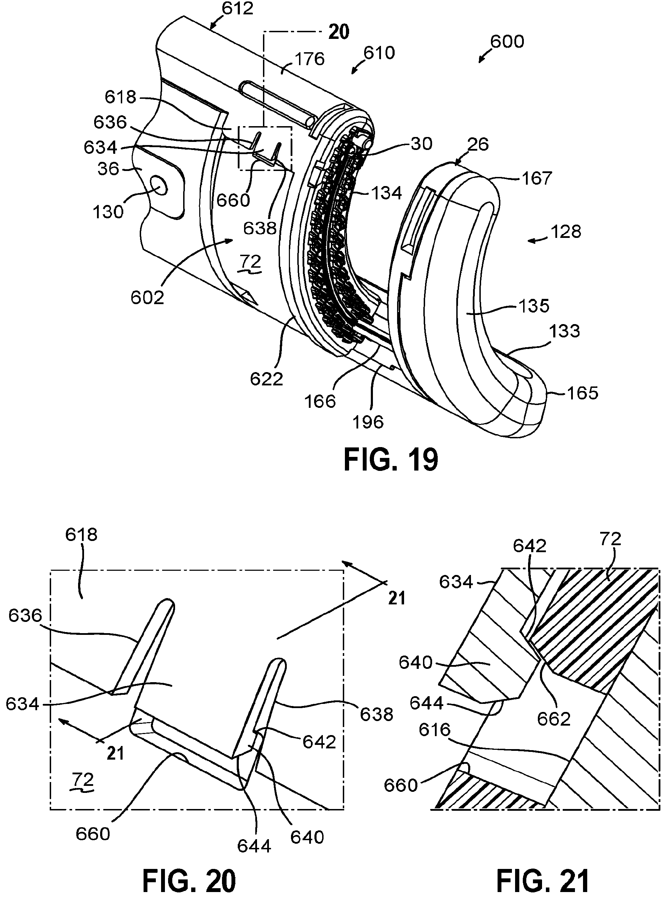

FIGS. 19-21 show an exemplary end effector (600) including C-shaped support structure (128), a closure member (602), and a replaceable cartridge unit (610) removably received by closure member (602). As described in greater detail below, end effector (600) has one or more cartridge retention features configured to provide improved mechanical engagement between cartridge unit (610) and closure member (602), at least relative to the mechanical engagement between cartridge unit (24) and closure member (54) of end effector (16). End effector (600), closure member (602), and cartridge unit (610) are similar to end effector (16), closure member (54), and cartridge unit (24) described above, respectively, except as otherwise described below.

Cartridge unit (610) of the present example includes anvil (26) movably coupled by guide pin (166) and anvil arm (196) to a cartridge housing (612) similar to cartridge housing (28) described above except as otherwise described below. In this regard, cartridge housing (612) defines staple deck (134) and houses various internal components including a plurality of staples (not shown), retaining pin (30), a staple driver assembly (not shown), such as staple driver assembly (140), and a knife holder (not shown), such as knife holder (142), and includes upper arm (176). Cartridge housing (612) includes a first rigid, C-shaped sidewall (not shown) opposed from a second rigid, C-shaped sidewall (616), each extending along respective lateral sides of cartridge unit (610). More particularly, the first sidewall extends along the concave first lateral side of cartridge unit (610), and second sidewall (616) extends along the convex second lateral side of cartridge unit (610). Cartridge housing (612) also includes a first upper flange (not shown) positioned laterally outwardly of an upper portion of the first sidewall and opposed from a second upper flange (618) positioned laterally outwardly of an upper portion of second sidewall (616). Cartridge housing (612) also includes a first C-shaped rail (not shown) opposed from a second C-shaped rail (622), each extending downwardly from respective flanges (618) along respective lateral sides of cartridge unit (610) and spaced apart from the respective sidewalls (616) by grooves (not shown), similar to grooves (230, 232) described above, for slidably receiving respective jaws (70, 72) of closure member (602), in a manner similar to that described above with respect to cartridge unit (210).

As best shown in FIGS. 19 and 20, cartridge housing (612) further includes a flexible upper beam (634) defined between generally parallel proximal and distal slots (636, 638) which extend upwardly from a lower edge of second flange (618). Thus, beam (634) may be integrally formed with a remainder of second flange (618) and cantilevered relative thereto. It will be appreciated that beam (634) may be configured and/or arranged in any other suitable manner(s). In any event, a deflectable retention member in the form of a detent (640) extends laterally inwardly from a lower end of beam (634) and includes an upper tapered abutment surface (642) and a lower tapered ramp surface (644), the purposes of which are described below. In the present version, beam (634) is configured to flex laterally outwardly away from second sidewall (616), and is resiliently biased laterally inwardly to its unflexed state, as shown in FIGS. 19-21. The integrally formed, cantilevered configuration of beam (634) relative to the remainder of second flange (618) may assist in maintaining beam (634) in its unflexed state in the absence of external forces acting upon beam (634).

Closure member (602) of the present example includes first and second jaws (70, 72). In the example shown, closure member (602) also includes an upper aperture (660) extending through second jaw (72) and at least partially defined by a beveled upper edge (662). As best shown in FIG. 21, upper edge (662) may be oriented relative to the laterally outer surface of second jaw (72) at a same or similar angle as that at which abutment surface (642) is oriented relative to the laterally inner surface of beam (634). It will be appreciated that aperture (660) may be configured and/or arranged in any other suitable manner(s).

When jaws (70, 72) are initially received within the respective grooves of cartridge housing (612), the laterally outer surface of second jaw (72) may cammingly engage ramp surface (644) of detent (640) to thereby urge detent (640), together with beam (634), laterally outwardly away from second sidewall (616) to permit advancement of jaws (70, 72) along the respective grooves. In this regard, ramp surface (644) may be oriented relative to the laterally outer surface of beam (634) at a predetermined angle selected to provide a desired camming engagement with the laterally outer surface of second jaw (72).

As best shown in FIGS. 20 and 21, when jaws (70, 72) are fully docked in the respective grooves, detent (640) may be positioned below upper edge (662) of second jaw (72), such that second jaw (72) disengages ramp surface (644). Due to the biasing of beam (634) toward its unflexed state, beam (634) may return to its unflexed state to allow abutment surface (642) to mate with or otherwise latch against upper edge (662) of second jaw (72) for improved retention of cartridge unit (610) within closure member (602). Such latching of abutment surface (642) against upper edge (662) may coincide with a "hard stop" between anvil arm (196) of cartridge unit (610) and medial arm (133) of support structure (128). In some versions, beam (634) may be substantially rigid and detent (640) may be deformable and biased toward its illustrated undeformed state, such that detent (640) may be deformed laterally outwardly by second jaw (72) from its undeformed state to a deformed state (not shown) for permitting advancement of jaws (70, 72) along the respective grooves, and may return to its undeformed state when jaws (70, 72) are fully docked in the respective grooves.

In any event, abutment surface (642) may securely latch against upper edge (662) of second jaw (72) to thereby increase the threshold separation force for removing cartridge unit (610) from closure member (602). Moreover, the biasing of detent (640) toward its unflexed state may increase the threshold insertion force for inserting cartridge unit (610) into closure member (602). Since detent (640) is deflectable laterally outwardly by second jaw (72) (e.g., via flexibility of beam (634) and/or deformability of detent (640)), detent (640) and upper edge (662) may each be resistant to wear, even as one or more cartridge units (610) are cyclically unloaded from and reloaded into closure member (602), at least by comparison to a pair of interlocking static cartridge retention features. In some versions, the movement of detent (640) into latching engagement with upper edge (662) of second jaw (72) may generate an audible and/or tactile response (e.g., via a "clicking" sound) indicating that that cartridge unit (610) is fully installed within closure member (602).

Thus, jaws (70, 72) may be advanced along the respective grooves of cartridge housing (612) (e.g., for guiding aperture (660) toward detent (640)) by applying a threshold insertion force to at least one of cartridge unit (610) or closure member (602) that is sufficient to overcome the biasing of detent (640) and thereby urge detent (640) laterally outwardly. Jaws (70, 72) may be retracted along the respective grooves of cartridge housing (612) (e.g., for guiding aperture (660) away from detent (640)) by applying a threshold unlatching force to detent (640) that is sufficient to overcome the biasing of detent (640) and thereby urge detent (640) laterally outwardly while also applying a separation force to at least one of cartridge unit (610) or closure member (602). In some versions, upper edge (662) may cammingly engage abutment surface (642) of detent (640) to thereby urge detent (640), together with beam (634), laterally outwardly away from second sidewall (616) to permit retraction of jaws (70, 72) along the respective grooves, such that the threshold unlatching force may be achieved via application of a threshold separation force to at least one of cartridge unit (610) or closure member (602). In this regard, upper edge (662) may be oriented relative to the laterally outer surface of second jaw (72) at a predetermined angle selected to provide a desired camming engagement with abutment surface (642). Likewise, abutment surface (642) may also be oriented relative to the laterally inner surface of beam (634) at such a predetermined angle.

While beam (634) of the present version is provided on second flange (618) and aperture (660) is provided on second jaw (72), beam (634) may alternatively be provided on the first flange and aperture (660) may be provided on first jaw (70). In some versions, first and second beams (634) may be provided on first and second flanges (618), respectively, and first and second apertures (660) may be provided on first and second jaws (70, 72), respectively, for engagement therewith. It will also be appreciated that beam (634) may alternatively be provided on one or more jaws (70, 72) and aperture (660) may be provided on one or more flanges (618) or sidewalls (616) in an inverse arrangement of end effector (600).

### F. Exemplary Closure Member with Upper Deflectable Cantilevered Beam Having Snap Feature

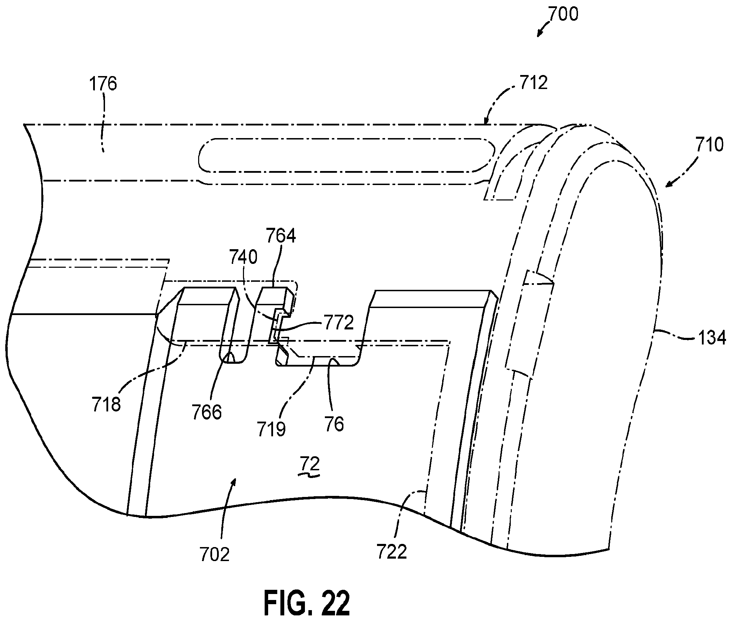

FIG. 22 shows an exemplary end effector (700) including a closure member (702) and a replaceable cartridge unit (710) removably received by closure member (702). As described in greater detail below, end effector (700) has one or more cartridge retention features configured to provide improved mechanical engagement between cartridge unit (710) and closure member (702), at least relative to the mechanical engagement between cartridge unit (24) and closure member (54) of end effector (16). End effector (700), closure member (702), and cartridge unit (710) are similar to end effector (16), closure member (702), and cartridge unit (24) described above, respectively, except as otherwise described below.

Cartridge unit (710) of the present example includes an anvil (not shown), such as anvil (26), movably coupled to a cartridge housing (712) similar to cartridge housing (28) described above except as otherwise described below. In this regard, cartridge housing (712) defines staple deck (134) and houses various internal components including a plurality of staples (not shown), a retaining pin (not shown), such as retaining pin (30), a staple driver assembly (not shown), such as staple driver assembly (140), and a knife holder (not shown), such as knife holder (142), and includes upper arm (176). Cartridge housing (712) includes opposed first and second rigid, C-shaped sidewalls (not shown) extending along respective lateral sides of cartridge unit (710). More particularly, the first sidewall extends along the concave first lateral side of cartridge unit (710), and the second sidewall extends along the convex second lateral side of cartridge unit (710). Cartridge housing (712) also includes a first upper flange (not shown) positioned laterally outwardly of an upper portion of the first sidewall and opposed from a second upper flange (718) positioned laterally outwardly of an upper portion of the second sidewall. In the present version, cartridge housing (712) also includes at least one tab (719) (one shown) extending laterally inwardly from a respective flange (718) toward a respective sidewall, for insertion into a respective notch (76) when cartridge unit (710) is installed in closure member (702), as shown in FIG. 22. Cartridge housing (712) also includes a first C-shaped rail (not shown) opposed from a second C-shaped rail (722), each extending downwardly from respective flanges (718) along respective lateral sides of cartridge unit (710) and spaced apart from the respective sidewalls by grooves (not shown), similar to grooves (230, 232) described above, for slidably receiving respective jaws (70, 72) of closure member (702), in a manner similar to that described above with respect to cartridge unit (210).

As shown in FIG. 22, cartridge housing (712) further includes a static (e.g., non-deflectable) retention member in the form of a rectangular detent (740) extending proximally from a proximal edge of tab (719), the purpose of which is described below. It will be appreciated that detent (740) may be configured and/or arranged in any other suitable manner(s). For example, in some versions detent (740) may be deflectable.

Closure member (702) of the present example includes first and second jaws (70, 72) including first and second upper notches (74, 76). In the example shown, closure member (702) further includes a flexible upper beam (764) defined between second notch (76) and a generally parallel proximal slot (766) which extends downwardly from an upper edge of second jaw (72). Thus, beam (764) may be integrally formed with a remainder of second jaw (72) and cantilevered relative thereto. It will be appreciated that beam (764) may be configured and/or arranged in any other suitable manner(s). In any event, a deflectable retention member in the form of a recess (772) extends proximally from a distal surface of beam (764), the purpose of which is described below. In the present version, beam (764) is configured to flex proximally into slot (766), and is resiliently biased distally to its unflexed state, as shown in FIG. 22. The integrally formed, cantilevered configuration of beam (764) relative to the remainder of second jaw (72) may assist in maintaining beam (764) in its unflexed state in the absence of external forces acting upon beam (764).

When jaws (70, 72) are initially received within the respective grooves of cartridge housing (712), detent (740) may cammingly engage the upper and/or distal surface(s) of beam (764) to thereby urge with beam (764) proximally into slot (766) to permit advancement of jaws (70, 72) along the respective grooves. In this regard, beam (764) may include a ramp surface (not shown) oriented at a predetermined angle selected to provide a desired camming engagement with detent (740).

As shown in FIG. 22, when jaws (70, 72) are fully docked in the respective grooves, detent (740) may be aligned with recess (772). Due to the biasing of beam (764) toward its unflexed state, beam (764) may return to its unflexed state to allow recess (772) to snap over or otherwise receive detent (740) such that an upper surface of detent (740) latches against an upper surface of recess (772) for improved retention of cartridge unit (710) within closure member (702). Such latching of the upper surface of detent (740) against the upper surface of recess (772) may coincide with a "hard stop" between anvil arm (196) of cartridge unit (710) and medial arm (133) of support structure (128).

In any event, the upper surface of detent (740) may securely latch against the upper surface of recess (772) to thereby increase the threshold separation force for removing cartridge unit (710) from closure member (702). Moreover, the biasing of beam (764) toward its unflexed state may increase the threshold insertion force for inserting cartridge unit (710) into closure member (702). Since recess (772) is deflectable proximally by detent (740) (e.g., via flexibility of beam (764)), recess (772) and detent (740) may each be resistant to wear, even as one or more cartridge units (710) are cyclically unloaded from and reloaded into closure member (702), at least by comparison to a pair of interlocking static cartridge retention features. In some versions, the movement of recess (772) into latching engagement with detent (740) may generate an audible and/or tactile response (e.g., via a "clicking" sound) indicating that cartridge unit (710) is fully installed within closure member (702).

Thus, jaws (70, 72) may be advanced along the respective grooves of cartridge housing (712) (e.g., for guiding second notch (76) toward tab (719)) by applying a threshold insertion force to at least one of cartridge unit (710) or closure member (702) that is sufficient to overcome the biasing of recess (772) and thereby urge recess (772) proximally. Jaws (70, 72) may be retracted along the respective grooves of cartridge housing (712) (e.g., for guiding second notch (76) away from tab (719)) by applying a threshold unlatching force to recess (772) that is sufficient to overcome the biasing of recess (772) and thereby urge recess (772) proximally while also applying a separation force to at least one of cartridge unit (710) or closure member (702). In some versions, the upper surface of detent (740) may cammingly engage the upper surface of recess (772) to thereby urge recess (772), together with beam (764), proximally toward slot (766) to permit retraction of jaws (70, 72) along the respective grooves, such that the threshold unlatching force may be achieved via application of a threshold separation force to at least one of cartridge unit (710) or closure member (702).

While beam (764) of the present version is provided on second jaw (72) and detent (740) is provided on second flange (718), beam (764) may alternatively be provided on first jaw (70) and detent (740) may be provided on the first flange. In some versions, first and second beams (764) may be provided on first and second jaws (70, 72), respectively, and first and second detents (740) may be provided on first and second flanges (718), respectively, for engagement therewith. It will also be appreciated that beam (764) may alternatively be provided on one or more flanges (718) or sidewalls and detent (740) may be provided on one or more jaws (70, 72) in an inverse arrangement of end effector (700).

### G. Exemplary Closure Member with Upper Deflectable Cantilevered Beam Having Zip Lock Feature

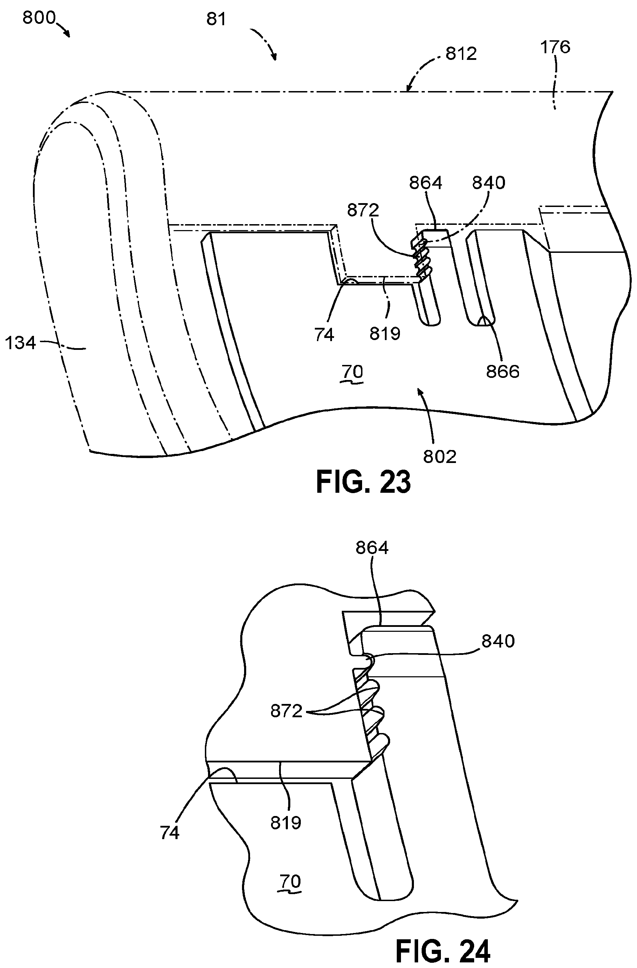

FIGS. 23-24 show an exemplary end effector (800) including a closure member (802) and a replaceable cartridge unit (810) removably received by closure member (802). As described in greater detail below, end effector (800) has one or more cartridge retention features configured to provide improved mechanical engagement between cartridge unit (810) and closure member (802), at least relative to the mechanical engagement between cartridge unit (24) and closure member (54) of end effector (16). End effector (800), closure member (802), and cartridge unit (810) are similar to end effector (16), closure member (54), and cartridge unit (24) described above, respectively, except as otherwise described below.

Cartridge unit (810) of the present example includes an anvil (not shown), such as anvil (26), movably coupled to a cartridge housing (812) similar to cartridge housing (28) described above except as otherwise described below. In this regard, cartridge housing (812) defines staple deck (134) and houses various internal components including a plurality of staples (not shown), a retaining pin (not shown), such as retaining pin (30), a staple driver assembly (not shown), such as staple driver assembly (140), and a knife holder (not shown), such as knife holder (142), and includes upper arm (176). Cartridge housing (812) includes opposed first and second rigid, C-shaped sidewalls (not shown) extending along respective lateral sides of cartridge unit (810). More particularly, the first sidewall extends along the concave first lateral side of cartridge unit (810), and the second sidewall extends along the convex second lateral side of cartridge unit (810). Cartridge housing (812) also includes opposed first and second upper flanges (not shown) positioned laterally outwardly of upper portions of the first and second sidewalls, respectively. In the present version, cartridge housing (812) also includes at least one tab (819) (one shown) extending laterally inwardly from a respective flange toward a respective sidewall, for insertion into a respective notch (74) when cartridge unit (810) is installed in closure member (802), as shown in FIGS. 23-24. Cartridge housing (812) may also include opposed first and second C-shaped rails (not shown) extending downwardly from respective flanges along respective lateral sides of cartridge unit (810) and spaced apart from the respective sidewalls by grooves (not shown), similar to grooves (230, 232) described above, for slidably receiving respective jaws (70, 72) of closure member (802), in a manner similar to that described above with respect to cartridge unit (210).

As shown in FIG. 22, cartridge housing (812) further includes a static (e.g., non-deflectable) retention member in the form of a rounded detent (840) extending proximally from a proximal edge of tab (819), the purpose of which is described below. It will be appreciated that detent (840) may be configured and/or arranged in any other suitable manner(s). For example, in some versions detent (840) may be deflectable.

Closure member (802) of the present example includes first and second jaws (70, 72) including first and second upper notches (74, 76). In the example shown, closure member (802) further includes a flexible upper beam (864) defined between first notch (74) and a generally parallel proximal slot (866) which extends downwardly from an upper edge of first jaw (70). Thus, beam (864) may be integrally formed with a remainder of first jaw (70) and cantilevered relative thereto. It will be appreciated that beam (864) may be configured and/or arranged in any other suitable manner(s). In any event, a plurality of deflectable retention members in the form of recesses (872) extend proximally from a distal surface of beam (864) and are spaced apart from each other along a length of beam (864), the purposes of which are described below. In the present version, beam (864) is configured to flex proximally into slot (866), and is resiliently biased distally to its unflexed state, as shown in FIGS. 23-24. The integrally formed, cantilevered configuration of beam (864) relative to the remainder of second jaw (72) may assist in maintaining beam (864) in its unflexed state in the absence of external forces acting upon beam (864).

When jaws (70, 72) are initially received within the respective grooves of cartridge housing (812), detent (840) may cammingly engage the upper and/or distal surface(s) of beam (864) to thereby urge beam (864) proximally into slot (866) to permit advancement of jaws (70, 72) along the respective grooves. In this regard, beam (864) may include a ramp surface (not shown) oriented at a predetermined angle selected to provide a desired camming engagement with detent (840).

As shown in FIGS. 23 and 24, when jaws (70, 72) are fully docked in the respective grooves, detent (840) may be aligned with one of recesses (872). Due to the biasing of beam (864) toward its unflexed state, beam (864) may return to its unflexed state to allow the aligned recess (872) to snap over or otherwise receive detent (840) such that an upper surface of detent (840) latches against an upper surface of the aligned recess (872) for improved retention of cartridge unit (810) within closure member (802). Such latching of the upper surface of detent (840) against the upper surface of the aligned recess (872) may coincide with a "hard stop" between anvil arm (196) of cartridge unit (810) and medial arm (133) of support structure (128). In some versions, detent (840) may be sequentially received within more than one of recesses (872) as jaws (70, 72) are advanced along the respective grooves to provide a ratcheting engagement between detent (840), which in such cases may be referred to as a "pawl," and a "rack" collectively defined by recesses (872). In other versions, a plurality of detents (840) may be spaced apart from each other along a length of tab (819) for receipt within corresponding recesses (872). In still other versions, a single recess (872) may be provided for receiving a single detent (840), such that the remaining recesses (872) may be omitted.

In any event, the upper surface of detent (840) may securely latch against the upper surface of the aligned recess (872) to thereby increase the threshold separation force for removing cartridge unit (810) from closure member (802). Moreover, the biasing of recesses (872) toward their unflexed state may increase the threshold insertion force for inserting cartridge unit (810) into closure member (802). Since recesses (872) are deflectable proximally by detent (840) (e.g., via flexibility of beam (864)), recesses (872) and detent (840) may each be resistant to wear, even as one or more cartridge units (810) are cyclically unloaded from and reloaded into closure member (802), at least by comparison to a pair of interlocking static cartridge retention features. In some versions, the movement of a recess (872) into latching engagement with detent (840) may generate an audible and/or tactile response (e.g., via a "clicking" sound) indicating that cartridge unit (810) is fully installed within closure member (802).

Thus, jaws (70, 72) may be advanced along the respective grooves of cartridge housing (812) (e.g., for guiding second notch (76) toward tab (819)) by applying a threshold insertion force to at least one of cartridge unit (810) or closure member (802) that is sufficient to overcome the biasing of recesses (872) and thereby urge recesses (872) proximally. Jaws (70, 72) may be retracted along the respective grooves of cartridge housing (812) (e.g., for guiding second notch (76) away from tab (819)) by applying a threshold unlatching force to recesses (872) that is sufficient to overcome the biasing of recesses (872) and thereby urge recesses (872) proximally while also applying a separation force to at least one of cartridge unit (810) or closure member (802). In some versions, the upper surface of detent (840) may cammingly engage the upper surface of the aligned recess (872) to thereby urge recess (872), together with beam (864), proximally into slot (866) to permit retraction of jaws (70, 72) along the respective grooves, such that the threshold unlatching force may be achieved via application of a threshold separation force to at least one of cartridge unit (810) or closure member (802).

While beam (864) of the present version is provided on first jaw (70) and detent (840) is provided on the first side of cartridge unit (810), beam (864) may alternatively be provided on second jaw (72) and detent (840) may be provided on the second side of cartridge unit (810). In some versions, first and second beams (864) may be provided on first and second jaws (70, 72), respectively, and first and second detents (840) may be provided on the first and second sides of cartridge unit (810), respectively, for engagement therewith. It will also be appreciated that beam (864) may alternatively be provided on one or more sides of cartridge unit (810) and detent (840) may be provided on one or more jaws (70, 72) in an inverse arrangement of end effector (800).

In some other versions, beam (864) of FIGS. 23-24 may be provided on one of jaws (70, 72) and detent (840) of FIGS. 23-24 may be provided on the corresponding side of cartridge unit (810), while beam (764) of FIG. 22 may be provided on the other of jaws (70, 72) and detent (740) of FIG. 22 may be provided on the corresponding side of cartridge unit (810) for engagement therewith, in a hybrid arrangement of end effectors (700, 800).

### H. Exemplary Support Structure with Magnet

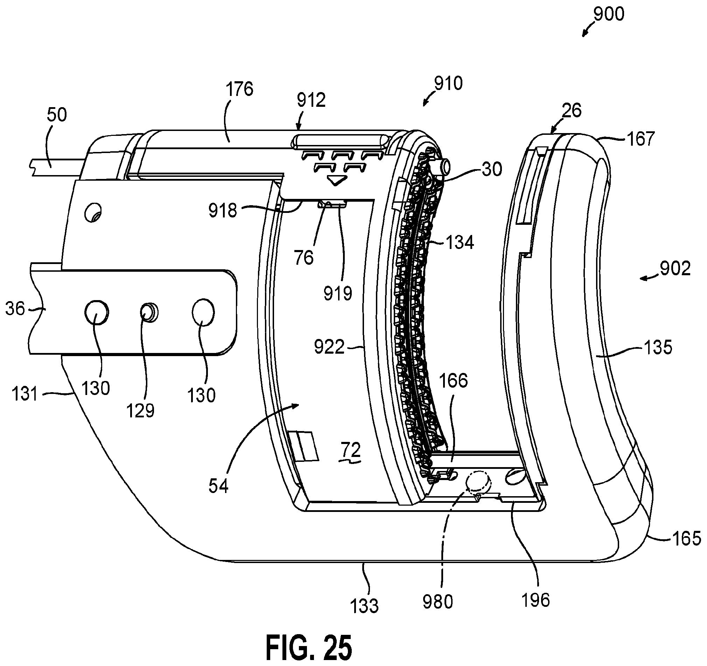

FIG. 25 shows an exemplary end effector (900) including closure member (54), a C-shaped support structure (902), and a replaceable cartridge unit (910) removably received by closure member (54). As described in greater detail below, end effector (900) has one or more cartridge retention features configured to provide improved mechanical engagement between cartridge unit (910) and closure member (54), at least relative to the mechanical engagement between cartridge unit (24) and closure member (54) of end effector (16). End effector (900), support structure (902), and cartridge unit (910) are similar to end effector (16), support structure (128), and cartridge unit (24) described above, respectively, except as otherwise described below.

Cartridge unit (910) of the present example includes anvil (26) movably coupled by guide pin (166) and anvil arm (196) to a cartridge housing (912) similar to cartridge housing (28) described above except as otherwise described below. In this regard, cartridge housing (912) defines staple deck (134) and houses various internal components including a plurality of staples (not shown), retaining pin (30), a staple driver assembly (not shown), such as staple driver assembly (140), and a knife holder (not shown), such as knife holder (142), and includes upper arm (176). Cartridge housing (912) includes opposed first and second rigid, C-shaped sidewalls (not shown) extending along respective lateral sides of cartridge unit (910). More particularly, the first sidewall extends along the concave first lateral side of cartridge unit (910), and the second sidewall extends along the convex second lateral side of cartridge unit (910). Cartridge housing (912) also includes a first upper flange (not shown) opposed from a second upper flange (918), each positioned laterally outwardly of upper portions of the first and second sidewalls, respectively. In the present version, cartridge housing (912) also includes at least one tab (919) (one shown) extending laterally inwardly from a respective flange (918) toward a respective sidewall, for insertion into a respective notch (76) when cartridge unit (910) is installed in closure member (54), as shown in FIG. 25. Cartridge housing (912) also includes a first C-shaped rail (not shown) opposed from a second C-shaped rail (922), each extending downwardly from respective flanges (918) along respective lateral sides of cartridge unit (910) and spaced apart from the respective sidewalls by grooves (not shown), similar to grooves (230, 232) described above, for slidably receiving respective jaws (70, 72) of closure member (54), in a manner similar to that described above with respect to cartridge unit (210).

In the present version, anvil arm (196) comprises a magnetic material. For example, anvil arm (196) may be formed of a ferrous and/or ferromagnetic material, such as ferritic stainless steel.

Support structure (902) of the present example includes proximal base portion (131), medial arm (133), and distal arm (135). In the example shown, support structure (902) further includes a contactless retention member in the form of a disc-shaped permanent magnet (980) embedded below an upper surface of medial arm (133). It will be appreciated that magnet (980) may be configured and/or arranged in any other suitable manner(s). For example, magnet (980) may be positioned at or above the upper surface of medial arm (133). In any event, magnet (980) may be configured to magnetically attract anvil arm (196) when anvil arm (196) is within a predetermined proximity of medial arm (133).

When jaws (70, 72) are initially received within the respective grooves of cartridge housing (912), anvil arm (196) may initially be sufficiently spaced apart from medial arm (133) to inhibit anvil arm (196) from being magnetically attracted to magnet (980), and may be gradually magnetically attracted to magnet (980) as the spacing between anvil arm (196) and medial arm (133) decreases. In this regard, magnet (980) may be positioned relative to the upper surface of medial arm (133) at a predetermined depth selected to provide a desired magnetic attraction with anvil arm (196). In addition, or alternatively, magnet (980) may be configured with a predetermined magnetic strength selected to provide a desired magnetic attraction with anvil arm (196).

As shown in FIG. 25, when jaws (70, 72) are fully docked in the respective grooves, anvil arm (196) may be sufficiently close to medial arm (133) to allow anvil arm (196) to be magnetically attracted to magnet (980). Due to the magnetic attraction of anvil arm (196) to magnet (980), a lower surface of anvil arm (196) may be magnetically pulled against the upper surface of medial arm (133) for improved retention of cartridge unit (910) within closure member (54). Such magnetic pulling of the lower surface of anvil arm (196) against the upper surface of medial arm (133) may define a "hard stop" between anvil arm (196) of cartridge unit (910) and medial arm (133) of support structure (902).

In any event, the lower surface of anvil arm (196) may be securely pulled against the upper surface of medial arm (133) by magnet (980) to thereby increase the threshold separation force for removing cartridge unit (910) from closure member (54). Since anvil arm (196) and magnet (980) interact with each other magnetically, anvil arm (196) and magnet (980) may each be resistant to wear, even as one or more cartridge units (910) are cyclically unloaded from and reloaded into closure member (54), at least by comparison to a pair of interlocking static cartridge retention features.

Thus, jaws (70, 72) may be advanced along the respective grooves of cartridge housing (912) (e.g., for guiding second notch (76) toward tab (919)), and such advancement may be assisted by the magnetic attraction of anvil arm (196) to magnet (980) when anvil arm (196) is within the predetermined proximity of medial arm (133). Jaws (70, 72) may be retracted along the respective grooves of cartridge housing (912) (e.g., for guiding second notch (76) away from tab (919)) by applying a threshold separation force to at least one of cartridge unit (910) or support structure (902) that is sufficient to overcome the magnetic attraction of anvil arm (196) to magnet (980).

### I. Exemplary Cartridge Unit with Upper, Downwardly-Extending Deflectable Cantilevered Beam Having Zip Lock Feature

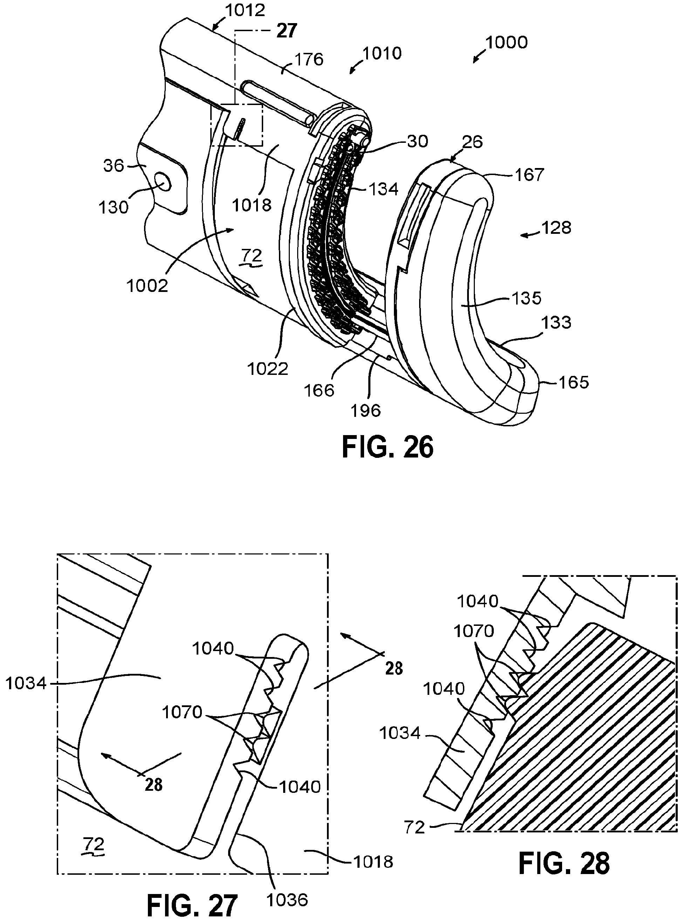

FIGS. 26-28 show an exemplary end effector (1000) including C-shaped support structure (128), a closure member (1002), and a replaceable cartridge unit (1010) removably received by closure member (1002). As described in greater detail below, end effector (1000) has one or more cartridge retention features configured to provide improved mechanical engagement between cartridge unit (1010) and closure member (1002), at least relative to the mechanical engagement between cartridge unit (24) and closure member (54) of end effector (16). End effector (1000), closure member (1002), and cartridge unit (1010) are similar to end effector (16), closure member (54), and cartridge unit (24) described above, respectively, except as otherwise described below.

Cartridge unit (1010) of the present example includes an anvil (not shown), such as anvil (26), movably coupled to a cartridge housing (1012) similar to cartridge housing (28) described above except as otherwise described below. In this regard, cartridge housing (1012) defines staple deck (134) and houses various internal components including a plurality of staples (not shown), a retaining pin (not shown), such as retaining pin (30), a staple driver assembly (not shown), such as staple driver assembly (140), and a knife holder (not shown), such as knife holder (142), and includes upper arm (176). Cartridge housing (1012) includes a first rigid, C-shaped sidewall (not shown) opposed from a second rigid, C-shaped sidewall (not shown), each extending along respective lateral sides of cartridge unit (1010). More particularly, the first sidewall extends along the concave first lateral side of cartridge unit (1010), and the second sidewall extends along the convex second lateral side of cartridge unit (1010). Cartridge housing (1012) also includes a first upper flange (not shown) positioned laterally outwardly of an upper portion of the first sidewall and opposed from a second upper flange (1018) positioned laterally outwardly of an upper portion of the second sidewall. Cartridge housing (1012) also includes a first C-shaped rail (not shown) opposed from a second C-shaped rail (1022), each extending downwardly from respective flanges (1018) along respective lateral sides of cartridge unit (1010) and spaced apart from the respective sidewalls by grooves (not shown), similar to grooves (230, 232) described above, for slidably receiving respective jaws (70, 72) of closure member (1002), in a manner similar to that described above with respect to cartridge unit (210).

As best shown in FIGS. 26 and 27, cartridge housing (1012) further includes a flexible upper beam (1034) defined by a slot (1036) which extends upwardly from a lower edge of second flange (1018). Thus, beam (1034) may be integrally formed with a remainder of second flange (1018) and cantilevered relative thereto. It will be appreciated that beam (1034) may be configured and/or arranged in any other suitable manner(s). In any event, a plurality of deflectable retention members in the form of recesses (1040) extend laterally outwardly from a laterally inner surface of beam (1034) and are spaced apart from each other along a length of beam (1034), the purposes of which are described below. In the present version, beam (1034) is configured to flex laterally outwardly away from the second sidewall, and is resiliently biased laterally inwardly to its unflexed state, as shown in FIGS. 26-28. The integrally formed, cantilevered configuration of beam (1034) relative to the remainder of second flange (1018) may assist in maintaining beam (1034) in its unflexed state in the absence of external forces acting upon beam (1034).

Closure member (1002) of the present example includes first and second jaws (70, 72) including first and second upper notches (74, 76). In the example shown, closure member (1002) further includes a plurality of static (e.g., non-deflectable) retention members in the form of a pair of rounded detents (1070) extending laterally outwardly from a laterally outer surface of second jaw (72), the purposes of which are described below. It will be appreciated that detents (1070) may be configured and/or arranged in any other suitable manner(s). For example, in some versions detents (1070) may be deflectable.

When jaws (70, 72) are initially received within the respective grooves of cartridge housing (1012), detents (1070) may cammingly engage the lower and/or laterally inner surface(s) of beam (1034) to thereby urge beam (1034) laterally outwardly to permit advancement of jaws (70, 72) along the respective grooves. In this regard, beam (1034) may include a ramp surface (not shown) oriented at a predetermined angle selected to provide a desired camming engagement with detents (1070).

As best shown in FIG. 28, when jaws (70, 72) are fully docked in the respective grooves, detents (1070) may be aligned with respective recesses (1040). Due to the biasing of beam (1034) toward its unflexed state, beam (1034) may return to its unflexed state to allow recesses (1040) to snap over or otherwise receive the respective aligned detents (1070) such that an upper surface of each detent (1070) latches against an upper surface of the respective aligned recess (1040) for improved retention of cartridge unit (1010) within closure member (1002). Such latching of the upper surface of detents (1070) against the upper surface of the respective aligned recesses (1040) may coincide with a "hard stop" between anvil arm (196) of cartridge unit (1010) and medial arm (133) of support structure (128). In some versions, detents (1070) may be sequentially received within more than one of recesses (1040) as jaws (70, 72) are advanced along the respective grooves to provide a ratcheting engagement between detents (1070), which in such cases may each be referred to as a "pawl," and a "rack" collectively defined by recesses (1040).

In any event, the upper surface of each detent (1070) may securely latch against the upper surface of the respective aligned recess (1040) to thereby increase the threshold separation force for removing cartridge unit (1010) from closure member (1002). Moreover, the biasing of recesses (1040) toward their unflexed state may increase the threshold insertion force for inserting cartridge unit (1010) into closure member (1002). Since recesses (1040) are deflectable proximally by detents (1070) (e.g., via flexibility of beam (1034)), recesses (1040) and detents (1070) may each be resistant to wear, even as one or more cartridge units (1010) are cyclically unloaded from and reloaded into closure member (1002), at least by comparison to a pair of interlocking static cartridge retention features. In some versions, the movement of detents (1070) into latching engagement with the respective aligned recess (1040) may generate an audible and/or tactile response (e.g., via a "clicking" sound) indicating that cartridge unit (1010) is fully installed within closure member (1002).

Thus, jaws (70, 72) may be advanced along the respective grooves of cartridge housing (1012) (e.g., for guiding detents (1070) toward recesses (1040)) by applying a threshold insertion force to at least one of cartridge unit (1010) or closure member (1002) that is sufficient to overcome the biasing of recesses (1040) and thereby urge recesses (1040) laterally outwardly. Jaws (70, 72) may be retracted along the respective grooves of cartridge housing (1012) (e.g., for guiding detents (1070) away from recesses (1040)) by applying a threshold unlatching force to recesses (1040) that is sufficient to overcome the biasing of recesses (1040) and thereby urge recesses (1040) laterally outwardly while also applying a separation force to at least one of cartridge unit (1010) or closure member (1002). In some versions, the upper surface of detents (1070) may cammingly engage the upper surface of the respective aligned recesses (1040) to thereby urge recesses (1040), together with beam (1034), laterally outwardly to permit retraction of jaws (70, 72) along the respective grooves, such that the threshold unlatching force may be achieved via application of a threshold separation force to at least one of cartridge unit (1010) or closure member (1002).

While beam (1034) of the present version is provided on the second side of cartridge unit (1010) and detents (1070) are provided on second jaw (72), beam (1034) may alternatively be provided on the first side of cartridge unit (1010) and detents (1070) may be provided on first jaw (70). In some versions, first and second beams (1034) may be provided on the first and second sides of cartridge unit (1010), respectively, and first and second detents (1070) may be provided on first and second jaws (70, 72), respectively, for engagement therewith. It will also be appreciated that beam (1034) may alternatively be provided on one or more jaws (70, 72) and detent (1070) may be provided on one or more sides of cartridge unit (1010) in an inverse arrangement of end effector (1000).

In some other versions, beam (1034) of FIGS. 26-28 and detent (740) of FIG. 22 may be provided on one of the sides of cartridge unit (1010), and detent (1070) of FIGS. 26-28 and beam (764) of FIG. 22 may be provided on the corresponding jaw (70, 72) for respective engagement therewith, in a hybrid arrangement of end effectors (700, 1000).

### J. Exemplary Closure Member with Lower, Upwardly-Extending Deflectable Cantilevered Beam Having Zip Lock Feature

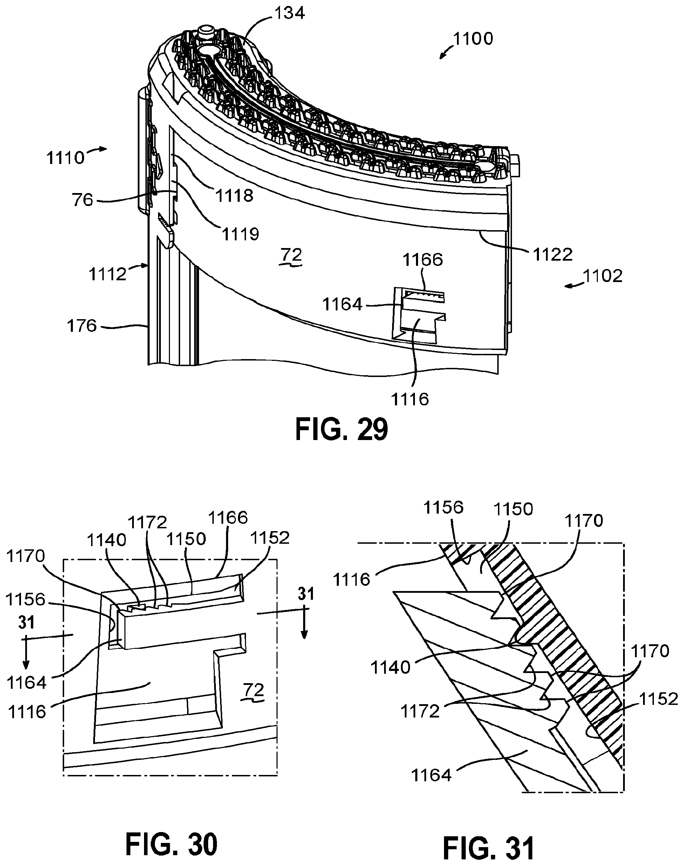

FIGS. 29-31 show an exemplary end effector (1100) including a closure member (1102) and a replaceable cartridge unit (1110) removably received by closure member (1102). As described in greater detail below, end effector (1100) has one or more cartridge retention features configured to provide improved mechanical engagement between cartridge unit (1110) and closure member (1102), at least relative to the mechanical engagement between cartridge unit (24) and closure member (54) of end effector (16). End effector (1100), closure member (1102), and cartridge unit (1110) are similar to end effector (16), closure member (54), and cartridge unit (24) described above, respectively, except as otherwise described below.

Cartridge unit (1110) of the present example includes an anvil (not shown), such as anvil (26), movably coupled to a cartridge housing (1112) similar to cartridge housing (28) described above except as otherwise described below. In this regard, cartridge housing (1112) defines staple deck (134) and houses various internal components including a plurality of staples (not shown), a retaining pin (not shown), such as retaining pin (30), a staple driver assembly (not shown), such as staple driver assembly (140), and a knife holder (not shown), such as knife holder (142), and includes upper arm (176). Cartridge housing (1112) includes a first rigid, C-shaped sidewall (not shown) opposed from a second rigid, C-shaped sidewall (1116), each extending along respective lateral sides of cartridge unit (1110). More particularly, the first sidewall extends along the concave first lateral side of cartridge unit (1110), and second sidewall (1116) extends along the convex second lateral side of cartridge unit (1110). Cartridge housing (1112) also includes a first upper flange (not shown) positioned laterally outwardly of an upper portion of the first sidewall and opposed from a second upper flange (1118) positioned laterally outwardly of an upper portion of second sidewall (1116). In the present version, cartridge housing (1112) also includes at least one tab (1119) (one shown) extending laterally inwardly from a respective flange (1118) toward a respective sidewall (1116), for insertion into a respective notch (76) when cartridge unit (1110) is installed in closure member (1102), as shown in FIG. 29. Cartridge housing (1112) also includes a first C-shaped rail (not shown) opposed from a second C-shaped rail (1122), each extending downwardly from respective flanges (1118) along respective lateral sides of cartridge unit (1110) and spaced apart from the respective sidewalls (1116) by grooves (not shown), similar to grooves (230, 232) described above, for slidably receiving respective jaws (70, 72) of closure member (1102), in a manner similar to that described above with respect to cartridge unit (210).

As shown in FIGS. 30 and 31, cartridge housing (1112) further includes a Y-shaped groove (1150) extending upwardly from a lower edge of second sidewall (1116) and laterally inwardly from a laterally outer surface thereof. In the present version, groove (1150) is at least partially defined by a Y-shaped recessed surface (1152) and has an open lower end (not shown) at the lower edge of second sidewall (1116) and a closed upper end (1156). The term "Y-shaped" is used herein as reference to the relatively wide, angled portion of groove (1150) near lower end and the relatively narrow, straight portion of groove (1150) near upper end (1156). Cartridge housing (1112) further includes a static (e.g., non-deflectable) retention member in the form of a rounded detent (1140) extending laterally outwardly from recessed surface (1152), the purpose of which is described below. It will be appreciated that detent (1140) may be configured and/or arranged in any other suitable manner(s). For example, in some versions detent (1140) may be deflectable.

Closure member (1102) of the present example includes first and second jaws (70, 72) including first and second upper notches (74, 76). In the example shown, closure member (1102) further includes a flexible lower beam (1164) defined by a generally C-shaped slot (1166) near a lower edge of second jaw (72). Thus, beam (1164) may be integrally formed with a remainder of second jaw (72) and cantilevered relative thereto. It will be appreciated that beam (1164) may be configured and/or arranged in any other suitable manner(s). In any event, a plurality of deflectable retention members in the form of detents (1170) extend laterally inwardly from a laterally inner surface of beam (1164) and are spaced apart from each other along a length of beam (1164) to define respective recesses (1172), the purposes of which are described below. In the present version, beam (1164) is configured to flex laterally outwardly, and is resiliently biased laterally inwardly to its unflexed state, as shown in FIGS. 29-31. The integrally formed, cantilevered configuration of beam (1164) relative to the remainder of second jaw (72) may assist in maintaining beam (1164) in its unflexed state in the absence of external forces acting upon beam (1164).

When jaws (70, 72) are initially received within the respective grooves of cartridge housing (1112), detent (1140) may cammingly engage the upper and/or distal surface(s) of detents (1170) to thereby urge beam (1164) laterally outwardly to permit advancement of jaws (70, 72) along the respective grooves.

As shown in FIGS. 30 and 31, when jaws (70, 72) are fully docked in the respective grooves, detent (1140) may be aligned with one of recesses (1172). Due to the biasing of beam (1164) toward its unflexed state, beam (1164) may return to its unflexed state to allow the aligned recess (1172) to snap over or otherwise receive detent (1140) such that a lower surface of detent (1140) latches against a lower surface of the aligned recess (1172) for improved retention of cartridge unit (1110) within closure member (1102). Such latching of the lower surface of detent (1140) against the lower surface of the aligned recess (1172) may coincide with a "hard stop" between anvil arm (196) of cartridge unit (1110) and medial arm (133) of support structure (128). In some versions, detent (1140) may be sequentially received within more than one of recesses (1172) as jaws (70, 72) are advanced along the respective grooves to provide a ratcheting engagement between detent (1140), which in such cases may be referred to as a "pawl," and a "rack" collectively defined by recesses (1172). In other versions, a plurality of detents (1140) may be spaced apart from each other along a length of recessed surface (1152) for receipt within corresponding recesses (1172). In still other versions, a single recess (1172) may be provided for receiving a single detent (1140), such that the remaining recesses (1172) may be omitted.

In any event, the lower surface of detent (1140) may securely latch against the lower surface of the aligned recess (1172) to thereby increase the threshold separation force for removing cartridge unit (1110) from closure member (1102). Moreover, the biasing of recesses (1172) toward their unflexed state may increase the threshold insertion force for inserting cartridge unit (1110) into closure member (1102). Since recesses (1172) are deflectable proximally by detent (1140) (e.g., via flexibility of beam (1164)), recesses (1172) and detent (1140) may each be resistant to wear, even as one or more cartridge units (1110) are cyclically unloaded from and reloaded into closure member (1102), at least by comparison to a pair of interlocking static cartridge retention features. In some versions, the movement of detent (1170) into latching engagement with the aligned recess (1172) may generate an audible and/or tactile response (e.g., via a "clicking" sound) indicating that cartridge unit (1110) is fully installed within closure member (1102).

Thus, jaws (70, 72) may be advanced along the respective grooves of cartridge housing (1112) (e.g., for guiding second notch (76) toward tab (1119)) by applying a threshold insertion force to at least one of cartridge unit (1110) or closure member (1102) that is sufficient to overcome the biasing of recesses (1172) and thereby urge recesses (1172) laterally outwardly. Jaws (70, 72) may be retracted along the respective grooves of cartridge housing (1112) (e.g., for guiding second notch (76) away from tab (1119)) by applying a threshold unlatching force to recesses (1172) that is sufficient to overcome the biasing of recesses (1172) and thereby urge recesses (1172) laterally outwardly while also applying a separation force to at least one of cartridge unit (1110) or closure member (1102). In some versions, the lower surface of detent (1140) may cammingly engage the lower surface of the aligned recess (1172) to thereby urge recess (1172), together with beam (1164), laterally outwardly to permit retraction of jaws (70, 72) along the respective grooves, such that the threshold unlatching force may be achieved via application of a threshold separation force to at least one of cartridge unit (1110) or closure member (1102).

While beam (1164) of the present version is provided on second jaw (72) and detent (1140) is provided on the second side of cartridge unit (1110), beam (1164) may alternatively be provided on first jaw (70) and detent (1140) may be provided on the first side of cartridge unit (1110). In some versions, first and second beams (1164) may be provided on first and second jaws (70, 72), respectively, and first and second detents (1140) may be provided on the first and second sides of cartridge unit (1110), respectively, for engagement therewith. It will also be appreciated that beam (1164) may alternatively be provided on one or more sides of cartridge unit (1110) and detent (1140) may be provided on one or more jaws (70, 72) in an inverse arrangement of end effector (1100).

In some other versions, beam (1164) of FIGS. 29-31, detent (1070) of FIGS. 26-28, and beam (764) of FIG. 22 may be provided on one of jaws (70, 72), and detent (1140) of FIGS. 29-31, beam (1034) of FIGS. 26-28, and detent (740) of FIG. 22 may be provided on the corresponding side of cartridge unit (1110) for respective engagement therewith, in a hybrid arrangement of end effectors (700, 1000, 1100).

### K. Exemplary Closure Member with Pair of Lower, Upwardly-Extending Deflectable Cantilevered Beams

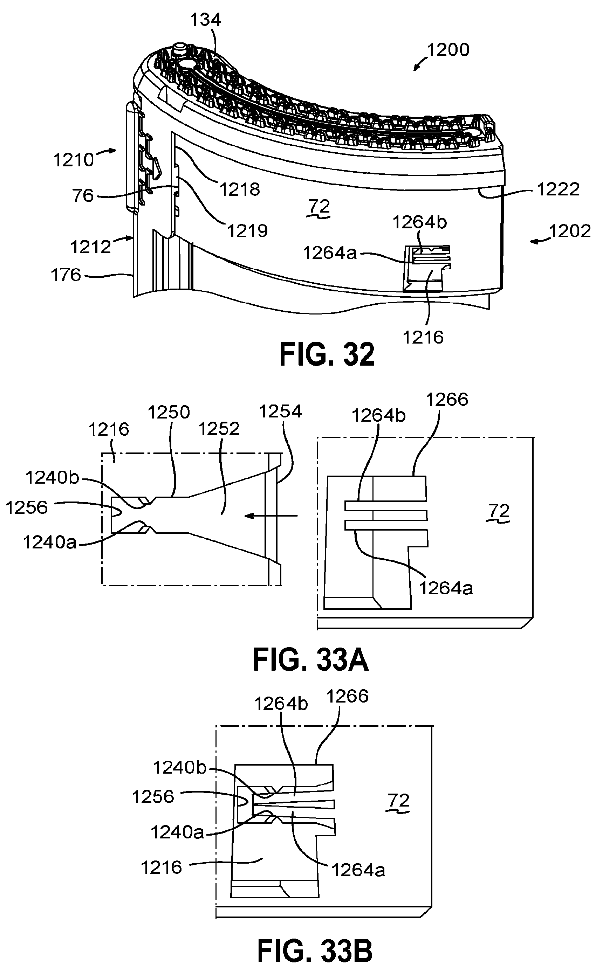

FIGS. 32-33B show an exemplary end effector (1200) including a closure member (1202) and a replaceable cartridge unit (1210) removably received by closure member (1202). As described in greater detail below, end effector (1200) has one or more cartridge retention features configured to provide improved mechanical engagement between cartridge unit (1210) and closure member (1202), at least relative to the mechanical engagement between cartridge unit (24) and closure member (54) of end effector (16). End effector (1200), closure member (1202), and cartridge unit (1210) are similar to end effector (16), closure member (54), and cartridge unit (24) described above, respectively, except as otherwise described below.

Cartridge unit (1210) of the present example includes an anvil (not shown), such as anvil (26), movably coupled to a cartridge housing (1212) similar to cartridge housing (28) described above except as otherwise described below. In this regard, cartridge housing (1212) defines staple deck (134) and houses various internal components including a plurality of staples (not shown), a retaining pin (not shown), such as retaining pin (30), a staple driver assembly (not shown), such as staple driver assembly (140), and a knife holder (not shown), such as knife holder (142), and includes upper arm (176). Cartridge housing (1212) includes a first rigid, C-shaped sidewall (not shown) opposed from a second rigid, C-shaped sidewall (1216), each extending along respective lateral sides of cartridge unit (1210). More particularly, the first sidewall extends along the concave first lateral side of cartridge unit (1210), and second sidewall (1216) extends along the convex second lateral side of cartridge unit (1210). Cartridge housing (1212) also includes a first upper flange (not shown) positioned laterally outwardly of an upper portion of the first sidewall and opposed from a second upper flange (1218) positioned laterally outwardly of an upper portion of second sidewall (1216). In the present version, cartridge housing (1212) also includes at least one tab (1219) (one shown) extending laterally inwardly from a respective flange (1218) toward a respective sidewall (1216), for insertion into a respective notch (76) when cartridge unit (1210) is installed in closure member (1202), as shown in FIG. 32. Cartridge housing (1212) also includes a first C-shaped rail (not shown) opposed from a second C-shaped rail (1222), each extending downwardly from respective flanges (1218) along respective lateral sides of cartridge unit (1210) and spaced apart from the respective sidewalls (1216) by grooves (not shown), similar to grooves (230, 232) described above, for slidably receiving respective jaws (70, 72) of support structure (1202), in a manner similar to that described above with respect to cartridge unit (210).

As best shown in FIGS. 33A-33B, cartridge housing (1212) further includes a Y-shaped groove (1250) extending upwardly from a lower edge of second sidewall (1216) and laterally inwardly from a laterally outer surface thereof. In the present version, groove (1250) is at least partially defined by a Y-shaped recessed surface (1252) having an open lower end (1254) at the lower edge of second sidewall (1216) and a closed upper end (1256). The term "Y-shaped" is used herein as reference to the relatively wide, angled portion of groove (1250) near lower end (1254) and the relatively narrow, straight portion of groove (1250) near upper end (1256). Cartridge housing (1212) further includes a plurality of static (e.g., non-deflectable) retention member in the form of proximal and distal pointed detents (1240a, 1240b) extending toward each other in the longitudinal direction from proximal and distal sides of groove (1250), respectively, the purposes of which are described below. It will be appreciated that detents (1240a, 1240b) may be configured and/or arranged in any other suitable manner(s). For example, in some versions detents (1240a, 1240b) may be deflectable.

Closure member (1202) of the present example includes first and second jaws (70, 72) including first and second upper notches (74, 76). In the example shown, closure member (1202) further includes a plurality of deflectable retention members in the form of generally parallel proximal and distal flexible lower beams (1264a, 1264b) defined by a generally E-shaped slot (1266) near a lower edge of second jaw (72). Thus, beams (1264a, 1264b) may each be integrally formed with a remainder of second jaw (72) and cantilevered relative thereto. It will be appreciated that beams (1264a, 1264b) may be configured and/or arranged in any other suitable manner(s). In the present version, proximal beam (1264a) is configured to flex distally, and is resiliently biased proximally to its unflexed state, as shown in FIGS. 32 and 33A. Likewise, distal beam (1264b) is configured to flex proximally, and is resiliently biased distally to its unflexed state, as shown in FIGS. 32 and 33A. The integrally formed, cantilevered configurations of beams (1264a, 1264b) relative to the remainder of second jaw (72) may assist in maintaining beams (1264a, 1264b) in their respective unflexed states in the absence of external forces acting upon beams (1264a, 1264b).

When jaws (70, 72) are initially received within the respective grooves of cartridge housing (1212), proximal detent (1240a) may cammingly engage the upper and/or proximal surface(s) of proximal beam (1264a) to thereby urge proximal beam (1264a) distally, and distal detent (1240b) may cammingly engage the upper and/or distal surface(s) of distal beam (1264b) to thereby urge distal beam (1264b) proximally to permit advancement of jaws (70, 72) along the respective grooves. In this regard, beams (1264a, 1164b) may include respective ramp surfaces (not shown) oriented at a predetermined angle selected to provide a desired camming engagement with the corresponding detents (1240a, 1240b).

In this manner, beams (1264a, 1264b) may each be urged toward each other in the longitudinal direction by the corresponding detent (1240a, 1240b) from their respective unflexed states, as best shown in FIG. 33A, to their respective flexed states, as best shown in FIG. 33B. Due to the biasing of beams (1264a, 1264b) toward their respective unflexed states, beams (1264a, 1264b) may each remain frictionally engaged with the respective detent (1240a, 1240b) while jaws (70, 72) are received within the respective grooves such that beams (1264a, 1264b) may be pinched toward and/or against each other by detents (1240a, 1240b) for improved retention of cartridge unit (1210) within closure member (1202). Since beams (1264a, 1264b) are deflectable toward each other in the longitudinal direction by the respective detents (1240a, 1240b), beams (1264a, 1264b) and detents (1240a, 1240b) may each be resistant to wear, even as one or more cartridge units (1210) are cyclically unloaded from and reloaded into closure member (1202), at least by comparison to a pair of interlocking static cartridge retention features.

Thus, jaws (70, 72) may be advanced along the respective grooves of cartridge housing (1212) (e.g., for guiding second notch (76) toward tab (1219)) by applying a threshold insertion force to at least one of cartridge unit (1210) or closure member (1202) that is sufficient to overcome the biasing of beams (1264a, 1264b) and thereby urge beams (1264a, 1264b) toward each other in the longitudinal direction. Jaws (70, 72) may be retracted along the respective grooves of cartridge housing (1212) (e.g., for guiding second notch (76) away from tab (1219)) by applying a threshold separation force to at least one of cartridge unit (1210) or closure member (1202) that is sufficient to overcome the frictional engagement between beams (1264a, 1264b) and the respective detents (1240a, 1240b). In some versions, the movement of beams (1264a, 1264b) out of engagement with the respective detents (1240a, 1240b) may generate an audible and/or tactile response (e.g., via a downward pushing of beams (1264a, 1264b) by detents (1240a, 1240b)) indicating that cartridge unit (1210) is uninstalled from closure member (1202).

While beams (1264a, 1264b) of the present version are provided on second jaw (72) and detents (1240a, 1240b) are provided on the second side of cartridge unit (1210), beams (1264a, 1264b) may alternatively be provided on first jaw (70) and detents (1240a, 1240b) may be provided on the first side of cartridge unit (1210). In some versions, first and second pairs of beams (1264a, 1264b) may be provided on first and second jaws (70, 72), respectively, and first and second pairs of detents (1240a, 1240b) may be provided on the first and second sides of cartridge unit (1210), respectively, for engagement therewith.

In some other versions, beams (1264a, 1264b) of FIGS. 32-33B and beam (764) of FIG. 22 may be provided on one of jaws (70, 72), and detents (1240a, 1240b) of FIGS. 32-33B and detent (740) of FIG. 22 may be provided on the corresponding side of cartridge unit (1210) for respective engagement therewith, in a hybrid arrangement of end effectors (700, 1200).

### L. Exemplary Cartridge Unit with Upper, Downwardly-Extending Deflectable Cantilevered Beam and Release Button

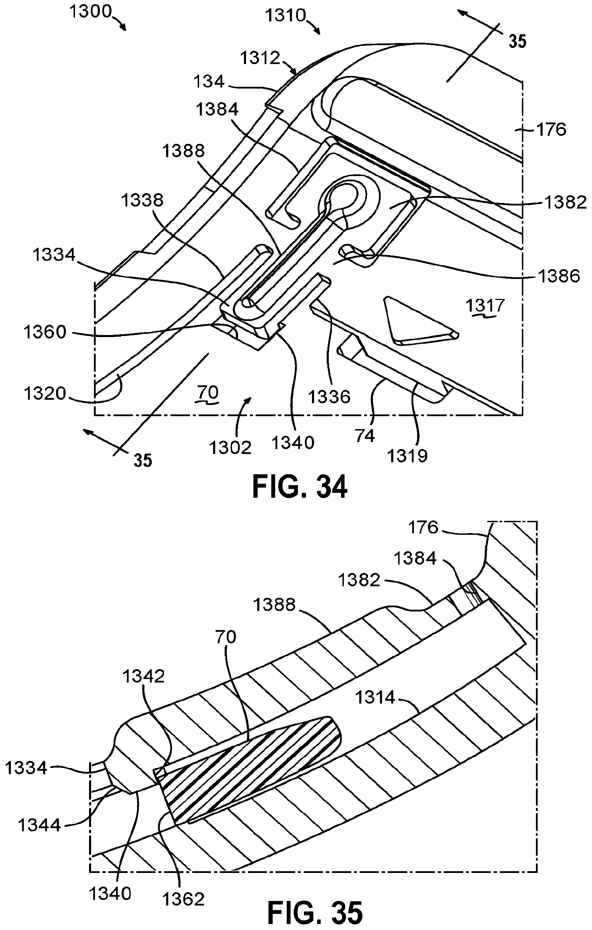

FIGS. 34-35 show an exemplary end effector (1300) including a closure member (1302) and a replaceable cartridge unit (1310) removably received by closure member (1302). As described in greater detail below, end effector (1300) has one or more cartridge retention features configured to provide improved mechanical engagement between cartridge unit (1310) and closure member (1302), at least relative to the mechanical engagement between cartridge unit (24) and closure member (54) of end effector (16). End effector (1300), closure member (1302), and cartridge unit (1310) are similar to end effector (16), closure member (54), and cartridge unit (24) described above, respectively, except as otherwise described below.

Cartridge unit (1310) of the present example includes an anvil (not shown), such as anvil (26), movably coupled to a cartridge housing (1312) similar to cartridge housing (28) described above except as otherwise described below. In this regard, cartridge housing (1312) defines staple deck (134) and houses various internal components including a plurality of staples (not shown), a retaining pin (not shown), such as retaining pin (30), a staple driver assembly (not shown), such as staple driver assembly (140), and a knife holder (not shown), such as knife holder (142), and includes upper arm (176). Cartridge housing (1312) includes a first rigid, C-shaped sidewall (1314) opposed from a second rigid, C-shaped sidewall (not shown), each extending along respective lateral sides of cartridge unit (1310). More particularly, first sidewall (1314) extends along the concave first lateral side of cartridge unit (1310), and the second sidewall extends along the convex second lateral side of cartridge unit (1310). Cartridge housing (1312) also includes a first upper flange (1317) positioned laterally outwardly of an upper portion of first sidewall (1314) and opposed from a second upper flange (not shown) positioned laterally outwardly of an upper portion of the second sidewall. In the present version, cartridge housing (1312) also includes at least one tab (1319) (one shown) extending laterally inwardly from a respective flange (1317) toward a respective sidewall (1314), for insertion into a respective notch (74) when cartridge unit (1310) is installed in closure member (1302), as shown in FIG. 34. Cartridge housing (1312) also includes a first C-shaped rail (1320) opposed from a second C-shaped rail (not shown), each extending downwardly from respective flanges (1317) along respective lateral sides of cartridge unit (1310) and spaced apart from the respective sidewalls (1314) by grooves (not shown), similar to grooves (230, 232) described above, for slidably receiving respective jaws (70, 72) of closure member (1302), in a manner similar to that described above with respect to cartridge unit (210).

As shown in FIGS. 34 and 35, cartridge housing (1312) further includes a flexible upper beam (1334) defined between generally parallel proximal and distal slots (1336, 1338) which extend upwardly from a lower edge of first flange (1317). Thus, beam (1334) may be integrally formed with a remainder of first flange (1317) and cantilevered relative thereto. It will be appreciated that beam (1334) may be configured and/or arranged in any other suitable manner(s). In any event, a deflectable retention member in the form of a detent (1340) extends laterally inwardly from a lower end of beam (1334) and includes an upper abutment surface (1342) and a lower tapered ramp surface (1344), the purposes of which are described below. In the present version, beam (1334) is configured to flex laterally outwardly away from first sidewall (1314), and is resiliently biased laterally inwardly to its unflexed state, as shown in FIGS. 34-35. The integrally formed, cantilevered configuration of beam (1334) relative to the remainder of first flange (1317) may assist in maintaining beam (1334) in its unflexed state in the absence of external forces acting upon beam (1334).

In the example shown, cartridge housing (1312) further includes a flexible upper release button (1382) aligned with beam (1334) in the vertical direction. In this regard, button (1382) is defined by a generally C-shaped slot (1384) having ends that are respectively positioned above proximal and distal slots (1336, 1338) and spaced apart therefrom by a bridge portion (1386). Thus, button (1382) may be integrally formed with a remainder of first flange (1317) and cantilevered relative thereto. It will be appreciated that button (1382) may be configured and/or arranged in any other suitable manner(s). In the present version, button (1382) is configured to flex laterally inwardly, and is resiliently biased laterally outwardly to its unflexed state, as shown in FIGS. 34-35. The integrally formed, cantilevered configuration of button (1382) relative to the remainder of first flange (1317) may assist in maintaining button (1382) in its unflexed state in the absence of external forces acting upon button (1382). In any event, button (1382) and beam (1334) may be configured to collectively pivot together about a fulcrum defined by bridge portion (1386), such that laterally inward flexing of button (1382) may cause laterally outward flexing of beam (1334). In this regard, a stiffening boss (1388) extends along both button (1382) and beam (1334) across bridge portion (1386) in the present example to assist with converting laterally inward flexing of button (1382) into laterally outward flexing of beam (1334).

Closure member (1302) of the present example includes first and second jaws (70, 72). In the example shown, closure member (1302) also includes an upper aperture (1360) extending through first jaw (70) and at least partially defined by an upper edge (1362). As best shown in FIG. 35, upper edge (662) may be oriented relative to the laterally outer surface of first jaw (70) at a same or similar angle as that at which abutment surface (1342) is oriented relative to the laterally inner surface of beam (1334). It will be appreciated that aperture (1360) may be configured and/or arranged in any other suitable manner(s).

When jaws (70, 72) are initially received within the respective grooves of cartridge housing (1312), the laterally outer surface of first jaw (70) may cammingly engage ramp surface (1344) of detent (1340) to thereby urge detent (1340), together with beam (1334), laterally outwardly away from first sidewall (1314) to permit advancement of jaws (70, 72) along the respective grooves. In this regard, ramp surface (1344) may be oriented relative to the laterally outer surface of beam (1334) at a predetermined angle selected to provide a desired camming engagement with the laterally outer surface of first jaw (70).

As shown in FIGS. 34 and 35, when jaws (70, 72) are fully docked in the respective grooves, detent (1340) may be positioned below upper edge (1362) of first jaw (70), such that first jaw (70) disengages ramp surface (1344). Due to the biasing of beam (1334) toward its unflexed state, beam (1334) may return to its unflexed state to allow abutment surface (1342) to mate with or otherwise latch against upper edge (1362) of first jaw (70) for improved retention of cartridge unit (1310) within closure member (1302). Such latching of abutment surface (1342) against upper edge (1362) may coincide with a "hard stop" between anvil arm (196) of cartridge unit (1310) and medial arm (133) of support structure (128).

In any event, abutment surface (1342) may securely latch against upper edge (1362) of first jaw (70) to thereby increase the threshold separation force for removing cartridge unit (1310) from closure member (1302). Moreover, the biasing of detent (1340) toward its unflexed state may increase the threshold insertion force for inserting cartridge unit (1310) into closure member (1302). Since detent (1340) is deflectable laterally outwardly by first jaw (70) (e.g., via flexibility of beam (1334)), detent (1340) and upper edge (1362) may each be resistant to wear, even as one or more cartridge units (1310) are cyclically unloaded from and reloaded into closure member (1302), at least by comparison to a pair of interlocking static cartridge retention features. In some versions, the movement of detent (1340) into latching engagement with upper edge (1362) of first jaw (70) may generate an audible and/or tactile response (e.g., via a "clicking" sound) indicating that cartridge unit (1310) is fully installed within closure member (1302).

Thus, jaws (70, 72) may be advanced along the respective grooves of cartridge housing (1312) (e.g., for guiding aperture (1360) toward detent (1340)) by applying a threshold insertion force to at least one of cartridge unit (1310) or closure member (1302) that is sufficient to overcome the biasing of detent (1340) and thereby urge detent (1340) laterally outwardly. Jaws (70, 72) may be retracted along the respective grooves of cartridge housing (1312) (e.g., for guiding aperture (1360) away from detent (1340)) by applying a threshold unlatching force to detent (1340) that is sufficient to overcome the biasing of detent (1340) and thereby urge detent (1340) laterally outwardly while also applying a separation force to at least one of cartridge unit (1310) or closure member (1302). For example, the threshold unlatching force may be achieved via application of a threshold pushing force against a laterally outer surface of button (1382). In some versions, upper edge (1362) may cammingly engage abutment surface (1342) of detent (1340) to thereby urge detent (1340), together with beam (1334), laterally outwardly away from first sidewall (1314) to permit retraction of jaws (70, 72) along the respective grooves, such that the threshold unlatching force may be achieved via application of a threshold separation force to at least one of cartridge unit (1310) or closure member (1302). In this regard, upper edge (1362) may be oriented relative to the laterally outer surface of first jaw (70) at a predetermined angle selected to provide a desired camming engagement with abutment surface (1342). Likewise, abutment surface (1342) may also be oriented relative to the laterally inner surface of beam (1334) at such a predetermined angle.

While beam (1334) and button (1382) of the present version are provided on first flange (1317) and aperture (1360) is provided on first jaw (70), beam (1334) and button (1382) may alternatively be provided on the second flange and aperture (1360) may be provided on second jaw (72). In some versions, first and second beams (1334) and corresponding buttons (1382) may be provided on first and second flanges (1317), respectively, and first and second apertures (1360) may be provided on first and second jaws (70, 72), respectively, for engagement therewith. It will also be appreciated that beam (1334) and button (1382) may alternatively be provided on one or more jaws (70, 72) and aperture (1360) may be provided on one or more flanges (1317) or sidewalls (1314) in an inverse arrangement of end effector (1300).

### M. Exemplary Cartridge Unit with Push-to-Release Catch

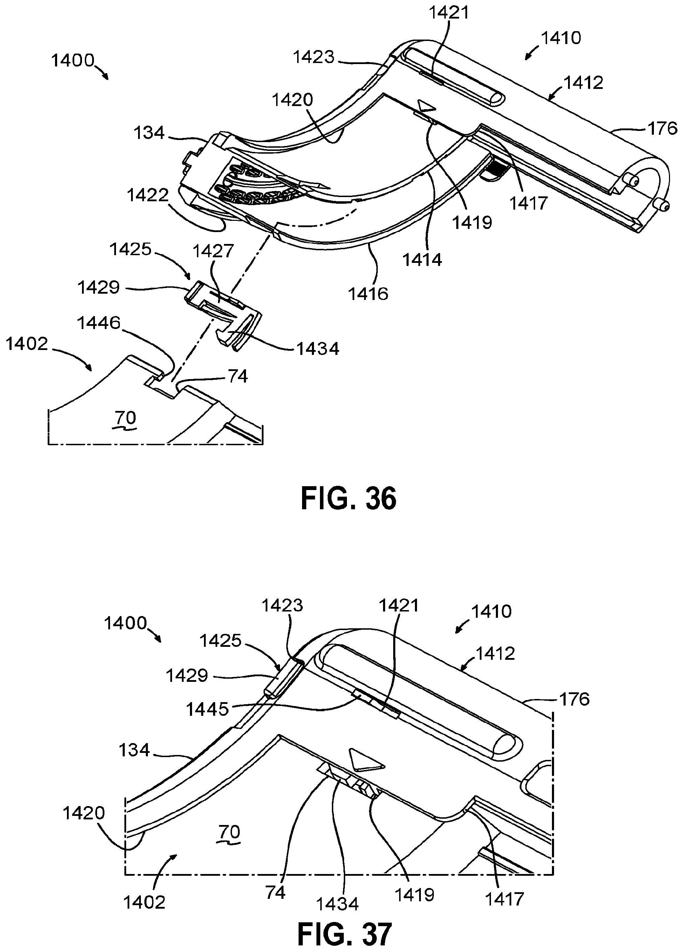

FIGS. 36-38 show an exemplary end effector (1400) including a closure member (1402) and a replaceable cartridge unit (1410) removably received by closure member (1402). As described in greater detail below, end effector (1400) has one or more cartridge retention features configured to provide improved mechanical engagement between cartridge unit (1410) and closure member (1402), at least relative to the mechanical engagement between cartridge unit (24) and closure member (54) of end effector (16). End effector (1400), closure member (1402), and cartridge unit (1410) are similar to end effector (16), closure member (54), and cartridge unit (24) described above, respectively, except as otherwise described below.

Cartridge unit (1410) of the present example includes an anvil (not shown), such as anvil (26), movably coupled to a cartridge housing (1412) similar to cartridge housing (28) described above except as otherwise described below. In this regard, cartridge housing (1412) defines staple deck (134) and houses various internal components including a plurality of staples (not shown), a retaining pin (not shown), such as retaining pin (30), a staple driver assembly (not shown), such as staple driver assembly (140), and a knife holder (not shown), such as knife holder (142), and includes upper arm (176). Cartridge housing (1412) includes a first rigid, C-shaped sidewall (1414) opposed from a second rigid, C-shaped sidewall (1416), each extending along respective lateral sides of cartridge unit (1410). More particularly, first sidewall (1414) extends along the concave first lateral side of cartridge unit (1410), and second sidewall (1416) extends along the convex second lateral side of cartridge unit (1410). Cartridge housing (1412) also includes a first upper flange (1417) positioned laterally outwardly of an upper portion of first sidewall (1414) and opposed from a second upper flange (not shown) positioned laterally outwardly of an upper portion of second sidewall (1416). In the present version, cartridge housing (1412) also includes a tab (1419) extending laterally inwardly from first upper flange (1417) toward a first sidewall (1414), for insertion into a respective notch (74) when cartridge unit (1410) is installed in closure member (1402), as shown in FIG. 37. Cartridge housing (1412) also includes a first C-shaped rail (1420) opposed from a second C-shaped rail (1422), each extending downwardly from respective flanges (1417) along respective lateral sides of cartridge unit (1410) and spaced apart from the respective sidewalls (1414, 1416) by grooves (not shown), similar to grooves (230, 232) described above, for slidably receiving respective jaws (70, 72) of closure member (1402), in a manner similar to that described above with respect to cartridge unit (210).

Cartridge housing (1412) of the present example also includes a longitudinally extending slot (1421) positioned along first flange (1417) (and/or upper arm (176)), and a longitudinally extending, generally rectangular bore (1423) near the upper end of staple deck (134) on the first side of cartridge unit (1410), the purposes of which are described below.

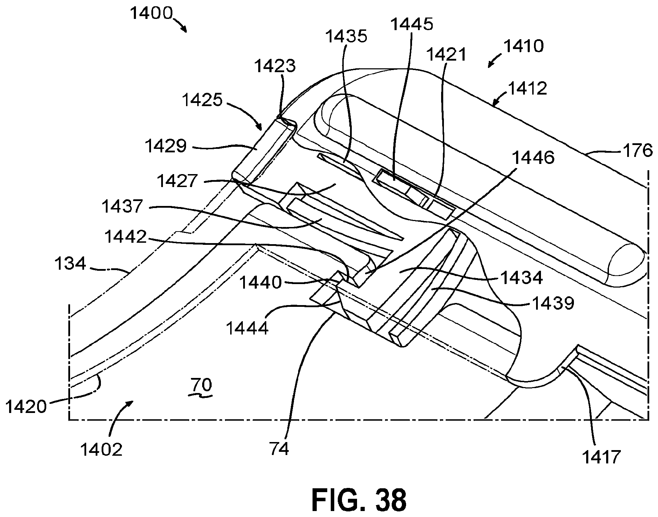

As shown in FIGS. 36-38, cartridge unit (1410) further includes a catch (1425) slidably positioned between first sidewall (1414) and first upper flange (1417) of cartridge housing (1412). As best shown in FIG. 38, catch (1425) of the present example includes a longitudinally extending body (1427), a distal release button (1429), and a proximal, downwardly extending beam (1434), such that catch (1425) may have a generally L-shaped profile. Catch (1425) further includes an upper spring arm (1435) extending proximally from release button (1429) above and generally parallel to body (1427), a lower spring arm (1437) extending downwardly and distally from an upper end of beam (1434) below and generally obliquely to body (1427), and a proximal spring arm (1439) extending downwardly and proximally from a proximal end of body (1427) proximal of and generally obliquely to beam (1434). Thus, beam (1434) and spring arms (1435, 1437, 1439) may be integrally formed with body (1427) and cantilevered relative thereto. It will be appreciated that beam (1434) and spring arms (1435, 1437, 1439) may be configured and/or arranged in any other suitable manner(s). In any event, a deflectable retention member in the form of a detent (1440) extends distally from a lower end of beam (1434) and includes an upper abutment surface (1442) and a lower tapered ramp surface (1444), the purposes of which are described below. Moreover, a fin (1445) extends laterally outwardly from a laterally outer surface of upper spring arm (1435), the purpose of which is also described below. In the present version, catch (1425) is configured to slide proximally relative to cartridge housing (1412), and is resiliently biased distally to its distal position, as shown in FIGS. 37-38. In this regard, a proximal surface of proximal spring arm (1439) may bear against a distal surface of tab (1419) for biasing catch (1425) distally. The integrally formed, cantilevered configuration of proximal spring arm (1439) relative to body (1427) may assist in maintaining catch (1425) in its distal position in the absence of external forces acting upon catch (1425).

In the present version, fin (1445) is configured to be slidably received within slot (1421). In this regard, upper spring arm (1435) is configured to flex laterally inwardly, and is resiliently biased laterally outwardly to its unflexed state for positioning fin (1445) within slot (1421), as shown in FIGS. 37-38. The integrally formed, cantilevered configuration of upper spring arm (1435) relative to body (1427) may assist in maintaining upper spring arm (1435) in its unflexed state in the absence of external forces acting upon upper spring arm (1435). In the present example, slot (1421) has a width substantially equal to or slightly greater than that of fin (1445) for inhibiting movement of catch (1425) relative to cartridge housing (1412) in the vertical direction, at least when upper spring arm (1435) is in its unflexed state with fin (1445) positioned within slot (1421). In any event, slot (1421) has a length greater than that of fin (1445) such that fin (1445) may be slidable longitudinally along slot (1421). The proximal and distal ends of slot (1421) may limit longitudinal sliding of fin (1445), and thus longitudinal sliding of catch (1425) relative to cartridge housing (1412) to a predetermined range of motion. For example, engagement of fin (1445) with the distal end of slot (1421) may define the distal position of catch (1425), and engagement of fin (1445) with the proximal end of slot (1421) may define a proximal position of catch (1425). Bore (1423) may accommodate button (1429), at least when catch (1425) is in the distal position, for permitting access to a distal surface of button (1429). In this regard, button (1429) may protrude distally at least partially through bore (1423) when catch (1425) is in the distal position, and may be recessed proximally when catch (1425) is in the proximal position.

Closure member (1402) of the present example includes first and second jaws (70, 72) including first and second upper notches (74, 76). A static (e.g., non-deflectable) retention member in the form of a detent (1446) extends proximally from an upper portion of a distal surface of first notch (74) of closure member (1402) for engaging detent (1440) when cartridge unit (1410) is installed in closure member (1402), as best shown in FIG. 38. It will be appreciated that detent (1446) may be configured and/or arranged in any other suitable manner(s). For example, in some versions, detent (1446) may be deflectable.

When jaws (70, 72) are initially received within the respective grooves of cartridge housing (1412), detent (1446) of first jaw (70) may cammingly engage ramp surface (1444) of detent (1440) to thereby urge detent (1440), together with catch (1425), proximally to permit advancement of jaws (70, 72) along the respective grooves. In this regard, ramp surface (1444) may be oriented relative to the lower surface of beam (1434) at a predetermined angle selected to provide a desired camming engagement with the upper edge of first jaw (70).

As shown in FIGS. 37 and 38, when jaws (70, 72) are fully docked in the respective grooves, detent (1440) may be positioned below detent (1446) of first jaw (70), such that detent (1446) disengages ramp surface (1444). Due to the biasing of catch (1425) toward its distal position, catch (1425) may return to its distal position to allow abutment surface (1442) to mate with or otherwise latch against detent (1446) for improved retention of cartridge unit (1410) within closure member (1402). Such latching of abutment surface (1442) against detent (1446) may coincide with a "hard stop" between anvil arm (196) of cartridge unit (1410) and medial arm (133) of support structure (128). In the present version, a lower surface of lower spring arm (1437) bears against the upper edge of first jaw (70) for biasing catch (1425) upwardly relative to closure member (1402). In some cases, such upward biasing of catch (1425) may assist with promoting latching of abutment surface (1442) against detent (1446).

In any event, abutment surface (1442) may securely latch against detent (1446) of first jaw (70) to thereby increase the threshold separation force for removing cartridge unit (1410) from closure member (1402). Moreover, the biasing of detent (1440) toward its distal position may increase the threshold insertion force for inserting cartridge unit (1410) into closure member (1402). Since detent (1440) is deflectable proximally by first jaw (70) (e.g., via sliding of catch (1425)), detent (1440) and detent (1446) may each be resistant to wear, even as one or more cartridge units (1410) are cyclically unloaded from and reloaded into closure member (1402), at least by comparison to a pair of interlocking static cartridge retention features. In some versions, the movement of detent (1440) into latching engagement with detent (1446) of first jaw (70) may generate an audible and/or tactile response (e.g., via a "clicking" sound) indicating that cartridge unit (1410) is fully installed within closure member (1402).

Thus, jaws (70, 72) may be advanced along the respective grooves of cartridge housing (1412) (e.g., for guiding first notch (74) toward detent (1440)) by applying a threshold insertion force to at least one of cartridge unit (1410) or closure member (1402) that is sufficient to overcome the biasing of detent (1440) and thereby urge detent (1440) proximally. Jaws (70, 72) may be retracted along the respective grooves of cartridge housing (1412) (e.g., for guiding first notch (74) away from detent (1440)) by applying a threshold unlatching force to detent (1440) that is sufficient to overcome the biasing of detent (1440) and thereby urge detent (1440) proximally while also applying a separation force to at least one of cartridge unit (1410) or closure member (1402). For example, the threshold unlatching force may be achieved via application of a threshold pushing force against the distal surface of button (1482). In some versions, detent (1446) of first jaw (70) may cammingly engage abutment surface (1442) of detent (1440) to thereby urge detent (1440), together with catch (1425), proximally to permit retraction of jaws (70, 72) along the respective grooves, such that the threshold unlatching force may be achieved via application of a threshold separation force to at least one of cartridge unit (1410) or closure member (1402). In this regard, abutment surface (1442) may be oriented relative to the distal surface of beam (1434) at a predetermined angle selected to provide a desired camming engagement with detent (1446). In some versions, the upward biasing of catch (1425) relative to closure member (1402) by lower spring arm (1437) may urge catch (1425) and thus cartridge unit (1410) away from closure member (1402), at least when catch (1425) is in its proximal position, to thereby assist with achieving the threshold separation force.

While catch (1425) of the present version is provided on the first side of cartridge unit (1410) and detent (1446) is provided on first jaw (70), catch (1425) may alternatively be provided on the second side of cartridge unit (1410) and detent (1446) may be provided on second jaw (72). In some versions, first and second catches (1425) may be provided on first and second sides of cartridge unit (1410), respectively, and first and second detents (1446) may be provided on first and second jaws (70, 72), respectively, for engagement therewith. It will also be appreciated that catch (1425) may alternatively be provided on one or more jaws (70, 72) and detent (1446) may be provided on one or more sides of cartridge unit (1410) in an inverse arrangement of end effector (1400).

### N. Exemplary Cartridge Unit with Pinch-to-Release Catch

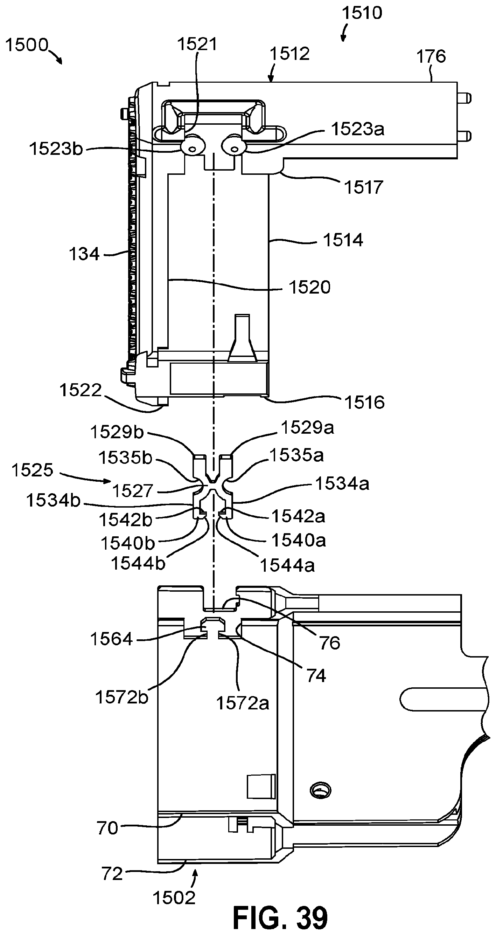

FIGS. 39-40 show an exemplary end effector (1500) including a closure member (1502) and a replaceable cartridge unit (1510) removably received by closure member (1502). As described in greater detail below, end effector (1500) has one or more cartridge retention features configured to provide improved mechanical engagement between cartridge unit (1510) and closure member (1502), at least relative to the mechanical engagement between cartridge unit (24) and closure member (54) of end effector (16). End effector (1500), closure member (1502), and cartridge unit (1510) are similar to end effector (16), closure member (54), and cartridge unit (24) described above, respectively, except as otherwise described below.

Cartridge unit (1510) of the present example includes an anvil (not shown), such as anvil (26), movably coupled to a cartridge housing (1512) similar to cartridge housing (28) described above except as otherwise described below. In this regard, cartridge housing (1512) defines staple deck (134) and houses various internal components including a plurality of staples (not shown), a retaining pin (not shown), such as retaining pin (30), a staple driver assembly (not shown), such as staple driver assembly (140), and a knife holder (not shown), such as knife holder (142), and includes upper arm (176). Cartridge housing (1512) includes a first rigid, C-shaped sidewall (1514) opposed from a second rigid, C-shaped sidewall (1516), each extending along respective lateral sides of cartridge unit (1510). More particularly, first sidewall (1514) extends along the concave first lateral side of cartridge unit (1510), and second sidewall (1516) extends along the convex second lateral side of cartridge unit (1510). Cartridge housing (1512) also includes a first upper flange (1517) positioned laterally outwardly of an upper portion of first sidewall (1514) and opposed from a second upper flange (not shown) positioned laterally outwardly of an upper portion of second sidewall (1516). Cartridge housing (1512) also includes a first C-shaped rail (1520) opposed from a second C-shaped rail (1522), each extending downwardly from respective flanges (1517) along respective lateral sides of cartridge unit (1510) and spaced apart from the respective sidewalls (1514, 1516) by grooves (not shown), similar to grooves (230, 232) described above, for slidably receiving respective jaws (70, 72) of closure member (1502), in a manner similar to that described above with respect to cartridge unit (210).

Cartridge housing (1512) of the present example also includes a vertically extending slot (1521) positioned along the first side of cartridge unit (1510) at a distal portion of upper arm (176) and bifurcating first flange (1517), and proximal and distal posts (1523a, 1523b) extending laterally outwardly from a laterally outer surface of slot (1521), the purposes of which are described below.

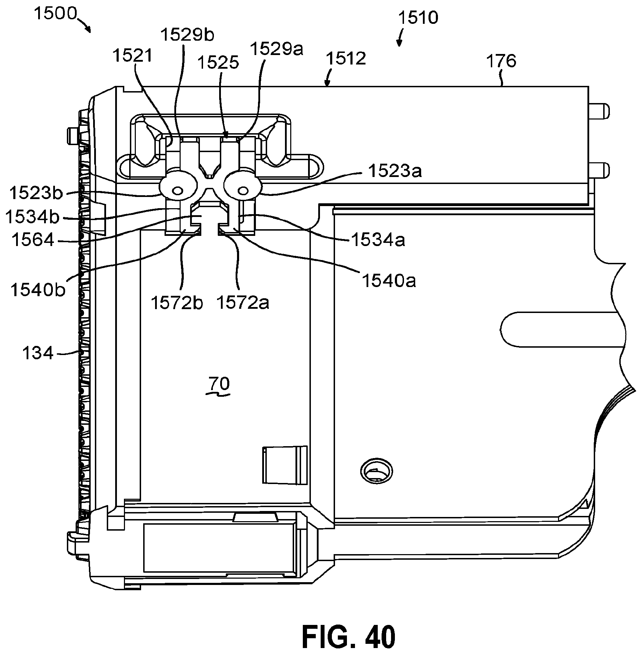

As shown in FIGS. 39-40, cartridge unit (1510) further includes a catch (1525) positioned within slot (1521) and removably coupled to posts (1523a, 1523b) of cartridge housing (1512). Catch (1525) of the present example includes a central body (1527), proximal and distal flexible upper release levers (1529a, 1529b), and proximal and distal flexible lower beams (1534a, 1534b), such that catch (1525) may have a generally X-shaped profile. In the present version, proximal and distal release levers (1529a, 1529b) are spaced apart from the corresponding beams (1534a, 1534b) by proximal and distal generally semi-circular recesses (1535a, 1535b), respectively. Thus, beams (1534a, 1534b) and levers (1529a, 1529b) may be integrally formed with body (1527) and cantilevered relative thereto. It will be appreciated that beams (1534a, 1534b) and levers (1529a, 1529b) may be configured and/or arranged in any other suitable manner(s). In any event, an opposed pair of deflectable retention members in the form of proximal and distal detents (1540a, 1540b) extend toward each other in the longitudinal direction from lower ends of respective beams (1534a, 1534b) and include respective upper abutment surfaces (1542a, 1542b) and respective lower tapered ramp surfaces (1544a, 1544b), the purposes of which are described below. In the present version, proximal beam (1534a) is configured to flex proximally, and is resiliently biased distally to its unflexed state, as shown in FIGS. 39 and 40. Likewise, distal beam (1534b) is configured to flex distally, and is resiliently biased proximally to its unflexed state, as shown in FIGS. 39 and 40. The integrally formed, cantilevered configurations of beams (1534a, 1534b) relative to body (1527) may assist in maintaining beams (1534a, 1534b) in their respective unflexed states in the absence of external forces acting upon beams (1534a, 1534b).

In the present version, proximal lever (1529a) is configured to flex distally, and is resiliently biased proximally to its unflexed state, as shown in FIGS. 39 and 40. Likewise, distal lever (1529b) is configured to flex proximally, and is resiliently biased distally to its unflexed state, as shown in FIGS. 39 and 40. The integrally formed, cantilevered configurations of levers (1529a, 1529b) relative to body (1527) may assist in maintaining levers (1529a, 1529b) in their respective unflexed states in the absence of external forces acting upon levers (1529a, 1529b). In any event, proximal lever (1529a) and proximal beam (1534a) may be configured to collectively pivot together about a fulcrum defined by body (1527), such that distal flexing of proximal lever (1529a) may cause proximal flexing of proximal beam (1534a). Likewise, distal lever (1529b) and distal beam (1534b) may be configured to collectively pivot together about the fulcrum defined by body (1527), such that proximal flexing of distal lever (1529b) may cause distal flexing of distal beam (1534b).

In the present version, proximal and distal recesses (1535a, 1535b) are configured to receive at least portions of proximal and distal posts (1523a, 1523b), respectively, for removably securing catch (1525) within slot (1521) of cartridge housing (1512). For example, catch (1525) may be captured between relatively narrow stem portions of posts (1523a, 1523b), and may further be captured between a relatively wide head portion of each post (152a, 1523b) and the laterally outer surface of slot (1521). In some versions, the narrow stem portions of posts (1523a, 1523b) may each have a generally circular cross-sectional shape defined by a radius substantially equal to that defining the semi-circular shape of recesses (1535a, 1535b) to provide bearing surfaces for facilitating pivoting of beams (1534a, 1534b) together with the corresponding levers (1529a, 1529b) about the fulcrum defined by body (1527). In any event, slot (1521) may have a length in the longitudinal direction sufficient to accommodate proximal flexing of proximal beam (1534a) and distal flexing of distal beam (1534b).

Closure member (1502) of the present example includes first and second jaws (70, 72) including first and second upper notches (74, 76). In the example shown, closure member (1502) further includes an upper beam (1564) extending upwardly from a lower edge of first notch (74). An opposed pair of static (e.g., non-deflectable) retention members in the form of proximal and distal recesses (1572a, 1572b) extend toward each other in the longitudinal direction from proximal and distal sides of a lower portion of beam (1564), respectively, the purposes of which are described below. It will be appreciated that recesses (1572a, 1572b) may be configured and/or arranged in any other suitable manner(s). For example, in some versions recesses (1572a, 1572b) may be deflectable.

When jaws (70, 72) are initially received within the respective grooves of cartridge housing (1512), beam (1564) of first jaw (70) may cammingly engage ramp surfaces (1544a, 1544b) of detents (1540a, 1540b) to thereby urge detents (1540a, 1540b), together with the respective beams (1534a, 1534b), away from each other in the longitudinal direction to permit advancement of jaws (70, 72) along the respective grooves. In this regard, ramp surfaces (1544a, 1544b) may be oriented relative to the lower surfaces of the respective beams (1534a, 1534b) at a predetermined angle selected to provide a desired camming engagement with beam (1564).

As shown in FIGS. 37 and 38, when jaws (70, 72) are fully docked in the respective grooves, detents (1540a, 1540b) may be aligned with the respective recesses (1572a, 1572b) of first jaw (70), such that beam (1564) disengages ramp surfaces (1544a, 1544b). Due to the biasing of beams (1534a, 1534b) toward their respective unflexed states, beams (1534a, 1534b) may return to their respective unflexed states to allow the respective abutment surfaces (1542a, 1542b) to mate with or otherwise latch against the upper surfaces of the corresponding recesses (1572a, 1572b) for improved retention of cartridge unit (1510) within closure member (1502). Such latching of abutment surfaces (1542a, 1542b) against the upper surfaces of the corresponding recesses (1572a, 1572b) may coincide with a "hard stop" between anvil arm (196) of cartridge unit (1510) and medial arm (133) of support structure (128).

In any event, abutment surfaces (1542a, 1542b) may securely latch against the upper surfaces of the corresponding recesses (1572a, 1572b) of first jaw (70) to thereby increase the threshold separation force for removing cartridge unit (1510) from closure member (1502). Moreover, the biasing of detents (1540a, 1540b) toward their respective unflexed states may increase the threshold insertion force for inserting cartridge unit (1510) into closure member (1502). Since detents (1540a, 1540b) are deflectable away from each other in the longitudinal direction by beam (1564) of first jaw (70) (e.g., via flexibility of the respective beams (1534a, 1534b)), detents (1540a, 1540b) and recesses (1572a, 1572b) may each be resistant to wear, even as one or more cartridge units (1510) are cyclically unloaded from and reloaded into closure member (1502), at least by comparison to a pair of interlocking static cartridge retention features. In some versions, the movement of detents (1540a, 1540b) into latching engagement with the upper surfaces of the corresponding recesses (1572a, 1572b) of first jaw (70) may generate an audible and/or tactile response (e.g., via a "clicking" sound) indicating that cartridge unit (1510) is fully installed within closure member (1502).

Thus, jaws (70, 72) may be advanced along the respective grooves of cartridge housing (1512) (e.g., for guiding first notch (74) toward detents (1540a, 1540b)) by applying a threshold insertion force to at least one of cartridge unit (1510) or closure member (1502) that is sufficient to overcome the biasing of detents (1540a, 1540b) and thereby urge detents (1540a, 1540b) away from each other in the longitudinal direction. Jaws (70, 72) may be retracted along the respective grooves of cartridge housing (1512) (e.g., for guiding first notch (74) away from detents (1540a, 1540b)) by applying a threshold unlatching force to detents (1540a, 1540b) that is sufficient to overcome the biasing of detents (1540a, 1540b) and thereby urge detents (1540a, 1540b) away from each other in the longitudinal direction while also applying a separation force to at least one of cartridge unit (1510) or closure member (1502). For example, the threshold unlatching force may be achieved via application of threshold pinching forces against the proximal surface of proximal lever (1529a) and the distal surface of distal lever (1529b).

While catch (1525) of the present version is provided on the first side of cartridge unit (1510) and beam (1564) is provided on first jaw (70), catch (1525) may alternatively be provided on the second side of cartridge unit (1510) and beam (1564) may be provided on second jaw (72). In some versions, first and second catches (1525) may be provided on first and second sides of cartridge unit (1510), respectively, and first and second beams (1564) may be provided on first and second jaws (70, 72), respectively, for engagement therewith. It will also be appreciated that catch (1525) may alternatively be provided on one or more jaws (70, 72) and beam (1564) may be provided on one or more sides of cartridge unit (1510) in an inverse arrangement of end effector (1500).

### III. Miscellaneous

Some versions of the devices described above may have application in conventional medical treatments and procedures conducted by a medical professional, as well as application in robotic-assisted medical treatments and procedures. By way of example only, various teachings herein may be readily incorporated into a robotic surgical system such as the DAVINCI^{™} system by Intuitive Surgical, Inc., of Sunnyvale, California. Similarly, those of ordinary skill in the art will recognize that various teachings herein may be readily combined with various teachings of any of the following: U.S. Pat. No. 8,479,969, entitled "Drive Interface for Operably Coupling a Manipulatable Surgical Tool to a Robot," issued July 9, 2013; U.S. Pat. Pub. No. 8,800,838, entitled "Robotically-Controlled Cable-Based Surgical End Effectors," issued Aug. 12, 2014; and/or U.S. Pat. No. 8,573,465, entitled "Robotically-Controlled Surgical End Effector System with Rotary Actuated Closure Systems," issued Nov. 5, 2013.

Some versions of the devices described above may be designed to be disposed of after a single use, or they can be designed to be used multiple times. They may, in either or both cases, be reconditioned for reuse after at least one use. Reconditioning may include any combination of the steps of disassembly of the device, followed by cleaning or replacement of particular pieces, and subsequent reassembly. In particular, some versions of the device may be disassembled, and any number of the particular pieces or parts of the device may be selectively replaced or removed in any combination. Upon cleaning and/or replacement of particular parts, some versions of the device may be reassembled for subsequent use either at a reconditioning facility, or by a user immediately prior to a procedure. Those skilled in the art will appreciate that reconditioning of a device may utilize a variety of techniques for disassembly, cleaning/replacement, and reassembly. Use of such techniques, and the resulting reconditioned device, are all within the scope of the present application.

By way of example only, some versions of the devices described herein may be sterilized before and/or after a procedure. In one sterilization technique, the device is placed in a closed and sealed container, such as a plastic or TYVEK bag. The container and device may then be placed in a field of radiation that can penetrate the container, such as gamma radiation, x-rays, or high-energy electrons. The radiation may kill bacteria on the device and in the container. The sterilized device may then be stored in the sterile container for later use. A device may also be sterilized using any other technique known in the art, including but not limited to beta or gamma radiation, ethylene oxide, or steam.

Having shown and described various embodiments of the present invention, further adaptations of the systems described herein may be accomplished by appropriate modifications by one of ordinary skill in the art without departing from the scope of the present invention. Several of such potential modifications have been mentioned, and others will be apparent to those skilled in the art. For instance, the examples, geometrics, materials, dimensions, ratios, steps, and the like discussed above are illustrative and are not required. Accordingly, the scope of the present invention should be considered in terms of the following claims and is understood not to be limited to the details of structure and operation shown and described in the specification and drawings.

## Claims

1. A surgical instrument (10) comprising:
(a) a body (12);
(b) a shaft (14) extending distally from the body (12); and
(c) an end effector (200, 300, 400, 500, 600, 700, 800, 900, 1000, 1100, 1200, 1300, 1400, 1500) at a distal end of the shaft (14), wherein the end effector includes:
(i) a staple cartridge unit (210, 310, 410, 510, 610, 710, 810, 910, 1010, 1110, 1210, 1310, 1410, 1510), wherein the staple cartridge unit includes:
(A) a housing (212, 312, 412, 512, 612, 712, 812, 912, 1212, 1112, 1212, 1312, 1412, 1512) that includes a plurality of staples, and
(B) an anvil (26) opposed from the housing, wherein the anvil (26) and the housing are configured to cooperate to clamp tissue, wherein the anvil (26) is configured to form staples ejected from the housing into the clamped tissue,
(ii) a cartridge retaining member (402, 502, 602, 702, 802, 1002, 1102, 1202, 1302, 1402, 1502), wherein the cartridge retaining member includes an opposed pair of jaws (70, 72) configured to cooperate to grip the housing of the staple cartridge unit, and
(iii) at least one deflectable retention member (224, 340, 540, 640, 772, 872, 1040, 1170, 1264a, 1264b, 1340, 1440, 1540a, 1540b) presented by one of the staple cartridge unit or the cartridge retaining member, wherein the at least one deflectable retention member is configured to be deflected relative to a portion of the one of the staple cartridge unit or the cartridge retaining member from an undeflected state to a deflected state
**characterized in that:**
(iv) the at least one deflectable retention member is configured to removably secure the housing of the staple cartridge unit to the cartridge retaining member, and configured to be deflected from the undeflected state to the deflected state by the other of the staple cartridge unit or the cartridge retaining member.

2. The surgical instrument (10) of claim 1, wherein the at least one deflectable retention member is presented by the staple cartridge unit, wherein the at least one deflectable retention member is configured to be deflected by the cartridge retaining member.

3. The surgical instrument (10) of claim 2, wherein the at least one deflectable retention member is presented by the housing of the staple cartridge unit.

4. The surgical instrument (10) of claim 2, wherein the staple cartridge unit further includes a catch (1425, 1525) removably secured to the housing, wherein the at least one deflectable retention member is presented by the catch.

5. The surgical instrument (10) of claim 1, wherein the at least one deflectable retention member is presented by the cartridge retaining member, wherein the at least one deflectable retention member is configured to be deflected by the staple cartridge unit.

6. The surgical instrument (10) of any one of claims 1 - 5, wherein the at least one deflectable retention member is configured to provide latching engagement between the staple cartridge unit and the cartridge retaining member when the at least one deflectable retention member is in the undeflected state.

7. The surgical instrument (10) of any one of claims 1 - 6, wherein the housing defines first and second lateral sides, wherein the at least one deflectable retention member is deflectable laterally outwardly or laterally inwardly.

8. The surgical instrument (10) of any one of claims 1 - 6, wherein the housing extends downwardly from the shaft (14), wherein the at least one deflectable retention member is deflectable upwardly or downwardly.

9. The surgical instrument (10) of any one of claims 1 - 8, wherein the at least one deflectable retention member includes a deflectable protrusion.

10. The surgical instrument of any one of claims 1 - 8, wherein the at least one deflectable retention member includes a deflectable recess (772, 872).

11. The surgical instrument (10) of any one of claims 1 - 10, further comprising a knife (32) movable relative to the housing and the anvil (26), wherein the housing is actuatable distally relative to the cartridge retaining member to clamp tissue against the anvil (26), wherein the knife (32) is configured to cut the clamped tissue.

12. A method of preparing a surgical instrument (10) including
(i) a body (12),
(ii) a shaft (14) extending distally from the body (12), and
(iii) an end effector (200, 300, 400, 500, 600, 700, 800, 900, 1000, 1100, 1200, 1300, 1400, 1500) at a distal end of the shaft (14), the method comprising:
(a) in response to a staple cartridge unit (210, 310, 410, 510, 610, 710, 810, 910, 1010, 1110, 1210, 1310, 1410, 1510) of the end effector being directed along a path relative to a cartridge retaining member (402, 502, 602, 702, 802, 1002, 1102, 1202, 1302, 1402, 1502) of the end effector from a removed state toward an installed state, deflecting at least one deflectable retention member (224, 340, 540, 640, 772, 872, 1040, 1170, 1264a, 1264b, 1340, 1440, 1540a, 1540b) presented by one of the staple cartridge unit or the cartridge retaining member relative to a portion of the one of the staple cartridge unit or the cartridge retaining member from an undeflected state to a deflected state by the other of the staple cartridge unit or the cartridge retaining member; and
(b) after the at least one deflectable retention member has assumed the deflected state, retaining the staple cartridge unit with the cartridge retaining member;
**characterized in that:**
the path is an arcuate path; and
the staple cartridge unit comprises:
(A) a housing (212, 312, 412, 512, 612, 712, 812, 912, 1212, 1112, 1212, 1312, 1412, 1512) that includes a plurality of staples, and
(B) an anvil (16) opposed from the housing, wherein the anvil (26) and the housing are configured to cooperate to clamp tissue, wherein the anvil (26) is configured to form staples ejected from the housing into the clamped tissue.

13. The method of claim 12, further comprising maintaining the at least one deflectable retention member in the deflected state when the staple cartridge unit is in the installed state.

14. The method of claim 12, further comprising returning the at least one deflectable retention member to the undeflected state when the staple cartridge unit is in the installed state.

15. The method of any one of claims 12 - 14, wherein the act of deflecting is performed via a camming engagement between the at least one deflectable retention member and the other of the staple cartridge unit or the support structure.

## Patentansprüche

1. Chirurgisches Instrument (10), umfassend:
(a) einen Körper (12);
(b) einen Schaft (14), der sich von dem Körper (12) distal erstreckt; und
(c) einen Endeffektor (200, 300, 400, 500, 600, 700, 800, 900, 1000, 1100, 1200, 1300, 1400, 1500) an einem distalen Ende des Schafts (14), wobei der Endeffektor einschließt:
(i) eine Klammermagazineinheit (210, 310, 410, 510, 610, 710, 810, 910, 1010, 1110, 1210, 1310, 1410, 1510), wobei die Klammermagazineinheit einschließt:
(A) ein Gehäuse (212, 312, 412, 512, 612, 712, 812, 912, 1212, 1112, 1212, 1312, 1412, 1512), das eine Vielzahl von Klammern einschließt, und
(B) einen Amboss (26), der dem Gehäuse gegenüberliegt, wobei der Amboss (26) und das Gehäuse konfiguriert sind, um zusammenwirken, um Gewebe zu klemmen, wobei der Amboss (26) konfiguriert ist, um Klammern auszubilden, die aus dem Gehäuse in das geklemmte Gewebe ausgestoßen werden,
(ii) ein Magazinzurückhalteelement (402, 502, 602, 702, 802, 1002, 1102, 1202, 1302, 1402, 1502), wobei das Magazinzurückhalteelement ein gegenüberliegendes Paar Backen (70, 72) einschließt, die konfiguriert sind, um zusammenzuwirken, um das Gehäuse der Klammermagazineinheit zu greifen, und
(iii) mindestens ein auslenkbares Zurückhaltungselement (224, 340, 540, 640, 772, 872, 1040, 1170, 1264a, 1264b, 1340, 1440, 1540a, 1540b), das durch eines von der Klammermagazineinheit oder dem Magazinzurückhalteelement dargestellt wird, wobei das mindestens eine auslenkbare Zurückhaltungselement konfiguriert ist, um relativ zu einem Abschnitt des einen von der Klammermagazineinheit oder dem Magazinzurückhalteelement von einem nicht ausgelenkten Zustand in einen ausgelenkten Zustand ausgelenkt zu werden;
**dadurch gekennzeichnet, dass:**
(iv) das mindestens eine auslenkbare Zurückhaltungselement konfiguriert ist, um das Gehäuse der Klammermagazineinheit an dem Magazinzurückhalteelement abnehmbar zu sichern, und konfiguriert ist, um von dem nicht ausgelenkten Zustand in den ausgelenkten Zustand durch die andere der Klammermagazineinheit oder des Magazinzurückhalteelements ausgelenkt zu werden.

2. Chirurgisches Instrument (10) nach Anspruch 1, wobei das mindestens eine auslenkbare Zurückhaltungselement durch die Klammermagazineinheit dargestellt wird, wobei das mindestens eine auslenkbare Zurückhaltungselement konfiguriert ist, um durch das Magazinzurückhalteelement abgelenkt zu werden.

3. Chirurgisches Instrument (10) nach Anspruch 2, wobei das mindestens eine auslenkbare Zurückhaltungselement durch das Gehäuse der Klammermagazineinheit dargestellt wird.

4. Chirurgisches Instrument (10) nach Anspruch 2, wobei die Klammermagazineinheit ferner eine Arretierung (1425, 1525) einschließt, die an dem Gehäuse abnehmbar gesichert ist, wobei das mindestens eine auslenkbare Zurückhaltungselement durch die Arretierung dargestellt wird.

5. Chirurgisches Instrument (10) nach Anspruch 1, wobei das mindestens eine auslenkbare Zurückhaltungselement durch das Magazinzurückhalteelement dargestellt wird, wobei das mindestens eine auslenkbare Zurückhaltungselement konfiguriert ist, um durch die Klammermagazineinheit ausgelenkt zu werden.

6. Chirurgisches Instrument (10) nach einem der Ansprüche 1 bis 5, wobei das mindestens eine auslenkbare Zurückhaltungselement konfiguriert ist, um einen Verriegelungseingriff zwischen der Klammermagazineinheit und dem Magazinzurückhalteelement bereitzustellen, wenn sich das mindestens eine auslenkbare Zurückhaltungselement in dem nicht ausgelenkten Zustand befindet.

7. Chirurgisches Instrument (10) nach einem der Ansprüche 1 bis 6, wobei das Gehäuse eine erste und eine zweite laterale Seite definiert, wobei das mindestens eine auslenkbare Zurückhaltungselement seitlich nach außen oder seitlich nach innen auslenkbar ist.

8. Chirurgisches Instrument (10) nach einem der Ansprüche 1 bis 6, wobei sich das Gehäuse von dem Schaft (14) nach unten erstreckt, wobei das mindestens eine auslenkbare Zurückhaltungselement nach oben oder unten auslenkbar ist.

9. Chirurgisches Instrument (10) nach einem der Ansprüche 1 bis 8, wobei das mindestens eine auslenkbare Zurückhaltungselement einen auslenkbaren Vorsprung einschließt.

10. Chirurgisches Instrument nach einem der Ansprüche 1 bis 8, wobei das mindestens eine auslenkbare Zurückhaltungselement eine auslenkbare Aussparung (772, 872) einschließt.

11. Chirurgisches Instrument (10) nach einem der Ansprüche 1 bis 10, ferner umfassend ein Messer (32), das relativ zu dem Gehäuse und zu dem Amboss (26) bewegbar ist, wobei das Gehäuse distal relativ zu dem Magazinzurückhalteelement betätigbar ist, um Gewebe gegen den Amboss (26) zu klemmen, wobei das Messer (32) konfiguriert ist, um das geklemmte Gewebe zu schneiden.

12. Verfahren zum Vorbereiten eines chirurgischen Instruments (10), das einschließt
(i) einen Körper (12),
(ii) einen Schaft (14), der sich von dem Körper (12) distal erstreckt und
(iii) einen Endeffektor (200, 300, 400, 500, 600, 700, 800, 900, 1000, 1100, 1200, 1300, 1400, 1500) an einem distalen Ende des Schafts (14), das Verfahren umfassend:
(a) als Reaktion darauf, dass eine Klammermagazineinheit (210, 310, 410, 510, 610, 710, 810, 910, 1010, 1110, 1210, 1310, 1410, 1510) des Endeffektors entlang eines Pfads relativ zu einem Magazinzurückhalteelement (402, 502, 602, 702, 802, 1002, 1102, 1202, 1302, 1402, 1502) des Endeffektors von einem entfernten Zustand zu einen installierten Zustand hin gelenkt wird, Auslenken mindestens eines auslenkbaren Zurückhaltungselements (224, 340, 540, 640, 772, 872, 1040, 1170, 1264a, 1264b, 1340, 1440, 1540a, 1540b), das durch eines von der Klammermagazineinheit oder dem Magazinzurückhalteelement relativ zu einem Abschnitt des einen von einer Klammermagazineinheit oder dem Magazinzurückhalteelement dargestellt wird, von einem nicht ausgelenkten Zustand in einen ausgelenkten Zustand durch die andere der Klammermagazineinheit oder des Magazinzurückhalteelements; und
(b) nachdem das mindestens eine auslenkbare Zurückhaltungselement den ausgelenkten Zustand eingenommen hat, Zurückhalten der Klammermagazineinheit mit dem Magazinzurückhalteelement;
**dadurch gekennzeichnet, dass:**
der Pfad ein bogenförmiger Pfad ist; und
die Klammermagazineinheit umfasst:
(A) ein Gehäuse (212, 312, 412, 512, 612, 712, 812, 912, 1212, 1112, 1212, 1312, 1412, 1512), das eine Vielzahl von Klammern einschließt, und
(B) einen Amboss (16), der dem Gehäuse gegenüberliegt, wobei der Amboss (26) und das Gehäuse konfiguriert sind, um zusammenwirken, um Gewebe zu klemmen, wobei der Amboss (26) konfiguriert ist, um Klammern auszubilden, die aus dem Gehäuse in das geklemmte Gewebe ausgestoßen werden.

13. Verfahren nach Anspruch 12, ferner umfassend das Halten des mindestens einen auslenkbaren Zurückhaltungselements in dem ausgelenkten Zustand, wenn sich die Klammermagazineinheit in dem installierten Zustand befindet.

14. Verfahren nach Anspruch 12, ferner umfassend ein Zurückführen des mindestens einen auslenkbaren Zurückhaltungselements in den nicht ausgelenkten Zustand, wenn sich die Klammermagazineinheit in dem installierten Zustand befindet.

15. Verfahren nach einem der Ansprüche 12 bis 14, wobei der Vorgang des Auslenkens über einen Nockeneingriff zwischen dem mindestens einen auslenkbaren Zurückhaltungselement und dem anderen des Klammermagazins oder der Trägerstruktur durchgeführt wird.

## Revendications

1. Instrument chirurgical (10), comprenant :
(a) un corps (12) ;
(b) une tige (14) s'étendant distalement à partir du corps (12) ; et
(c) un effecteur terminal (200, 300, 400, 500, 600, 700, 800, 900, 1000, 1100, 1200, 1300, 1400, 1500) au niveau d'une extrémité distale de la tige (14), dans lequel l'effecteur terminal comporte :
(i) une unité de cartouche d'agrafes (210, 310, 410, 510, 610, 710, 810, 910, 1010, 1110, 1210, 1310, 1410, 1510), dans lequel l'unité de cartouche d'agrafes comporte :
(A) un logement (212, 312, 412, 512, 612, 712, 812, 912, 1212, 1112, 1212, 1312, 1412, 1512) qui comporte une pluralité d'agrafes, et
(B) une enclume (26) opposée au logement, dans lequel l'enclume (26) et le logement sont conçus pour coopérer pour serrer un tissu, dans lequel l'enclume (26) est conçue pour former des agrafes éjectées du logement dans le tissu serré,
(ii) un élément de retenue de cartouche (402, 502, 602, 702, 802, 1002, 1102, 1202, 1302, 1402, 1502), dans lequel l'élément de retenue de cartouche comporte une paire opposée de mâchoires (70, 72) conçues pour coopérer pour saisir le logement de l'unité de cartouche d'agrafes, et
(iii) au moins un élément de rétention déviable (224, 340, 540, 640, 772, 872, 1040, 1170, 1264a, 1264b, 1340, 1440, 1540a, 1540b) présenté par l'un parmi l'unité de cartouche d'agrafes ou l'élément de retenue de cartouche, dans lequel l'au moins un élément de rétention déviable est conçu pour être dévié par rapport à une partie de l'un parmi l'unité de cartouche d'agrafes ou l'élément de retenue de cartouche d'un état non dévié à un état dévié ;
**caractérisé en ce que** :
(iv) l'au moins un élément de rétention déviable est conçu pour fixer de manière amovible le logement de l'unité de cartouche d'agrafes à l'élément de retenue de cartouche, et conçu pour être dévié de l'état non dévié à l'état dévié par l'autre parmi l'unité de cartouche d'agrafes ou l'élément de retenue de cartouche.

2. Instrument chirurgical (10) selon la revendication 1, dans lequel l'au moins un élément de rétention déviable est présenté par l'unité de cartouche d'agrafes, dans lequel l'au moins un élément de rétention déviable est conçu pour être dévié par l'élément de retenue de cartouche.

3. Instrument chirurgical (10) selon la revendication 2, dans lequel l'au moins un élément de rétention déviable est présenté par le logement de l'unité de cartouche d'agrafes.

4. Instrument chirurgical (10) selon la revendication 2, dans lequel l'unité de cartouche d'agrafes comporte en outre un loquet (1425, 1525) fixé de façon amovible au logement, dans lequel l'au moins un élément de rétention déviable est présenté par le loquet.

5. Instrument chirurgical (10) selon la revendication 1, dans lequel l'au moins un élément de rétention déviable est présenté par l'élément de retenue de cartouche, dans lequel l'au moins un élément de rétention déviable est conçu pour être dévié par l'unité de cartouche d'agrafes.

6. Instrument chirurgical (10) selon l'une quelconque des revendications 1 à 5, dans lequel l'au moins un élément de rétention déviable est conçu pour fournir une mise en prise d'accrochage entre l'unité de cartouche d'agrafes et l'élément de retenue de cartouche lorsque l'au moins un élément de rétention déviable est dans l'état non dévié.

7. Instrument chirurgical (10) selon l'une quelconque des revendications 1 à 6, dans lequel le logement définit des premier et second côtés latéraux, dans lequel l'au moins un élément de rétention déviable peut être dévié latéralement vers l'extérieur ou latéralement vers l'intérieur.

8. Instrument chirurgical (10) selon l'une quelconque des revendications 1 à 6, dans lequel le logement s'étend vers le bas à partir de la tige (14), dans lequel l'au moins un élément de rétention déviable peut être dévié vers le haut ou vers le bas.

9. Instrument chirurgical (10) selon l'une quelconque des revendications 1 à 8, dans lequel l'au moins un élément de rétention déviable comporte une partie saillante déviable.

10. Instrument chirurgical selon l'une quelconque des revendications 1 à 8, dans lequel l'au moins un élément de rétention déviable comporte un évidement déviable (772, 872).

11. Instrument chirurgical (10) selon l'une quelconque des revendications 1 à 10, comprenant en outre un couteau (32) mobile par rapport au logement et à l'enclume (26), dans lequel le logement est actionnable distalement par rapport à l'élément de retenue de cartouche pour serrer un tissu contre l'enclume (26), dans lequel le couteau (32) est conçu pour couper le tissu serré.

12. Procédé de préparation d'un instrument chirurgical (10) comportant
(i) un corps (12),
(ii) une tige (14) s'étendant distalement à partir du corps (12), et
(iii) un effecteur terminal (200, 300, 400, 500, 600, 700, 800, 900, 1000, 1100, 1200, 1300, 1400, 1500) au niveau d'une extrémité distale de la tige (14), le procédé comprenant :
(a) en réponse à une unité de cartouche d'agrafes (210, 310, 410, 510, 610, 710, 810, 910, 1010, 1110, 1210, 1310, 1410, 1510) de l'effecteur terminal étant dirigée le long d'un trajet par rapport à un élément de retenue de cartouche (402, 502, 602, 702, 802, 1002, 1102, 1202, 1302, 1402, 1502) de l'effecteur terminal d'un état retiré vers un état installé, la déviation d'au moins un élément de rétention déviable (224, 340, 540, 640, 772, 872, 1040, 1170, 1264a, 1264b, 1340, 1440, 1540a, 1540b) présenté par l'un parmi l'unité de cartouche d'agrafes ou l'élément de retenue de cartouche par rapport à une partie de l'un parmi l'unité de cartouche d'agrafes ou l'élément de retenue de cartouche, d'un état non dévié à un état dévié par l'autre parmi l'unité de cartouche d'agrafes ou l'élément de retenue de cartouche ; et
(b) après que l'au moins un élément de rétention déviable a adopté l'état dévié, la rétention de l'unité de cartouche d'agrafes avec l'élément de retenue de cartouche ;
**caractérisé en ce que :**
le trajet est un trajet arqué ; et
l'unité de cartouche d'agrafes comprend :
(A) un logement (212, 312, 412, 512, 612, 712, 812, 912, 1212, 1112, 1212, 1312, 1412, 1512) qui comporte une pluralité d'agrafes, et
(B) une enclume (16) opposée au logement, dans lequel l'enclume (26) et le logement sont conçus pour coopérer pour serrer un tissu, dans lequel l'enclume (26) est conçue pour former des agrafes éjectées du logement dans le tissu serré.

13. Procédé selon la revendication 12, comprenant en outre le maintien de l'au moins un élément de rétention déviable dans l'état dévié lorsque l'unité de cartouche d'agrafes est dans l'état installé.

14. Procédé selon la revendication 12, comprenant en outre le retour de l'au moins un élément de rétention déviable à l'état non dévié lorsque l'unité de cartouche d'agrafes est dans l'état installé.

15. Procédé selon l'une quelconque des revendications 12 à 14, dans lequel le fait de dévier est mis en œuvre par l'intermédiaire d'une mise en prise de came entre l'au moins un élément de rétention déviable et l'autre parmi l'unité de cartouche d'agrafes ou la structure de support.
